# FASCICULE DE BREVET EUROPEEN

(11) **EP 1 792 606 B1**
(45) Date de publication et mention de la délivrance du brevet: **18.05.2011**
(21) Numéro de dépôt: 06123702.0
(22) Date de dépôt: 08.11.2006
(51) Int. Cl.: A61K 8/49, A61Q 5/10, C07D 471/04

(54) **Composition pour la teinture des fibres keratiniques comprenant au moins un dérive 3-amino pyrazolopyridine**
Mischung zum Färben von Keratinfasern enthaltend mindestens ein 3-Amino Pyrazolpyridinderivat
Composition for dyeing keratinic fibers comprising at least one derivative of 3-amino pyrazolopyridine

(30) Priorité: 09.11.2005 FR 0553403
(43) Date de publication de la demande: 06.06.2007
(73) Titulaire: L'Oréal, 75008 Paris (FR)
(72) Inventeur: Fadli, Aziz, 77500, CHELLES (FR); Vidal, Laurent, 75013, PARIS (FR); Sabelle, Stéphane, 75005, PARIS (FR)
(74) Mandataire: Fevrier, Murielle Françoise E.

(56) Documents cités:
- EP-A- 0 433 854
- EP-A- 1 389 618
- WO-A-01/35917
- WO-A-02/076416
- WO-A-02/076417
- FR-A- 2 822 691
- FR-A- 2 822 692

## Description

L'invention a pour objet une composition pour la teinture des fibres kératiniques, et en particulier des fibres kératiniques humaines telles que les cheveux, comprenant au moins un dérivé 3-amino pyrazolo-[1,5-a]-pyridine particulier ou un de ses sels d'addition et un procédé mettant en oeuvre ce dérivé. Elle a de même pour objet les dérivés 3-amino pyrazolo-[1,5-a]-pyridines ou un de ses sels d'addition.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4-triazoles, des dérivés de pyrazolo[3,2-c]-1,2,4-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

L'utilisation de base d'oxydation telle que les dérivés de para-phénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, de variété de nuances, d'uniformité de la couleur et de la ténacité aux agents extérieurs.

L'utilisation de ces bases à pH neutre est de plus inefficace pour atteindre une gamme de nuances variées, en particuliers pour les nuances chaudes.

Il a été déjà proposé dans la demande de brevet EP 1 233 743 des compositions de teintures contenant en tant que base d'oxydation des 3-amino pyrazolo-[1,5-a]-pyridines. Les compositions de teinture décrites dans ce document ne permettent pas d'atteindre de bonnes propriétés de chromaticité, de résistance aux agents extérieurs telle que le lavage et la lumière. De plus, l'étendue de la gamme des nuances est limitée.

Le but de la présente invention est de fournir de nouvelles bases d'oxydation pour la teinture des fibres kératiniques qui ne présentent pas les inconvénients des bases d'oxydation existantes. En particulier, le but de l'invention est de fournir de nouvelles bases d'oxydation qui permettent d'obtenir une coloration aux nuances variées, puissante, chromatique, esthétique, peu sélective et résistant bien aux diverses agressions que peuvent subir les cheveux tels que les shampooings, la lumière, la sueur et les déformations permanentes.

Ce but est atteint avec la présente invention qui a pour objet une composition de teinture des fibres kératiniques comprenant, dans un milieu approprié à la teinture, à titre de base de coloration d'oxydation, un dérivé 3-amino pyrazolo-[1,5-a]-pyridine de formule (I) suivante ainsi que ses sels et solvates : 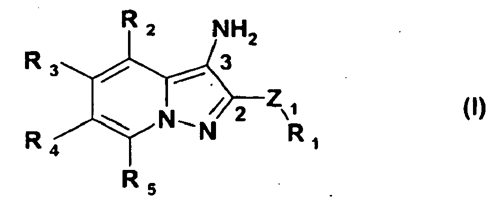 dans laquelle
- Z₁ représente un atome d'oxygène, un groupement NR₆, lorsque Z₁ représente NR₆ alors R₁ et R₆ peuvent former ensemble avec l'atome d'azote auquel ils sont rattachés un hétérocycle saturé, insaturé ou aromatique de 5 à 8 chaînons, éventuellement substitué,
   Z₁ peut aussi représenter un radical divalent S, SO, SO₂ lorsque R₁ est un radical méthyle,
- R₁ est choisi parmi un radical alkyle, un radical alkyle substitué par un hydroxy, un radical alkyle substitué par un amino ou (di)alkylamino, un radical alkyle substitué par un hétérocycle azoté par exemple pipérazinyle, imidazolyle, pyrrolidinyle, pyridinyle, morpholinyle, pipéridinyle. Selon une variante, le radical alkyle comprend de 1 à 6 atomes de carbone.
- R₆ est choisi parmi un atome d'hydrogène, un radical alkyle ou un radical alkyle substitué par un radical hydroxy, un radical amino, un radical alkylamino, un radical dialkyle amino, un radical alkyle substitué par un hétérocycle azoté par exemple pipérazinyle, imidazolyle, pyrrolidinyle, pyridinyle, morpholinyle, pipéridinyle. Selon une variante, R₆ comprend de 1 à 6 atomes de carbone.
- R₂, R₃, R₄, R₅, indépendamment, représentent :
   ■ un atome d'hydrogène,
   ■ un radical alkyle en C₁-C₄ éventuellement substitué,
- R₂, R₃, R₄, R₅, peuvent former deux à deux avec des radicaux adjacents un (hétéro)cycle saturé ou insaturé, éventuellement substitué,
à l'exception du 2-méthoxy-pyrazolo[l.5-a]pyridine-3-ylamine et du 2-morpholino-pyrazolo[1.5-a]pyridine-3-ylamine respectivement de formules suivantes :

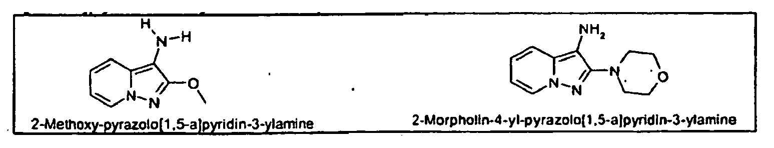

La présente invention permet en particulier d'obtenir une coloration des fibres kératiniques tenace, résistante à la lumière et au lavage.

Un autre objet de l'invention est un procédé de teinture des fibres kératiniques mettant en oeuvre la composition de la présente invention, ainsi que l'utilisation de cette composition pour la teinture des fibres kératiniques.

L'invention a aussi pour objet de nouveaux dérivés pyrazolopyridines de formule (I) à l'exception des composés 2-méthylsulfanyl-pyrazolo[1,5-a]pyridin-3-ylamine et 2,3-diamino-pyrazolo[1,5-a]pyridine.ainsi que les dérivés nitro ou nitroso correspondants.

Dans le cadre de l'invention, on entend par radical alkyle, les radicaux alkyles linéaires ou ramifiés, pouvant être substitués ou non substitués. Ils peuvent être substitués par n'importe quel substituant classique dans le domaine de la coloration et qui ne changent pas les propriétés de base d'oxydation des composés de formule (I).

De même lorsque les radicaux (hétéro)cycliques définis pour la formule (I) sont substitués, ils peuvent être substitués par n'importe quel radical classique dans le domaine de la coloration qui ne change pas les propriétés de base d'oxydation des composés de formule (I). A titre d'exemples de substituants des cycles ou hétérocycles, on peut citer les radicaux alkyles, les radicaux alkyles substitués, les radicaux hydroxy, alcoxy, amino, alkylamino, dialkylamino, thio, alkylthio, carboxy, alkylcarbonyle, sulfonyle, amido,etc.

L'hétérocycle azoté formé par R₁ et R₆ peut contenir un ou plusieurs autres hétéroatomes notamment un hétéroatome choisi parmi N, O, S, SO, SO₂, -CO- et leurs combinaisons. Il peut de plus être substitué ou non, notamment comme décrit ci dessus.

Les composés de formule (I) peuvent être éventuellement salifiés par des acides minéraux forts tels que par exemple HCl, HBr, HI, H₂SO₄, H₃PO₄, ou des acides organiques tels que, par exemple, l'acide acétique, lactique, tartrique, citrique ou succinique, benzènesulfonique, para-toluènesulfonique, formique, méthanesulfonique.

Ils peuvent aussi être sous forme de solvates par exemple un hydrate ou un solvate d'alcool linéaire ou ramifié tel que l'éthanol ou l'isopropanol.

Dans le cadre de l'invention, on entend par dérivé de formule (I) toutes formes mésomères ou isomères.

Dans la formule (I) ci-dessus, lorsque R₁ et/ou R₆ représentent un radical alkyle substitué, alors les substituants sont notamment choisis parmi les halogènes, les radicaux -OH, -OR₉, -NH₂, -NHR₁₀ -NR₁₁R₁₂, -COR₁₃, -O-CO-R₁₃, -CO-OR₁₄, -NR₁₅-CO-R₁₆, -CO-NR₁₅R₁₆, -SO₃H, les radicaux cycliques saturés ou insaturés contenant éventuellement un hétéroatome choisi parmi N, S, O, le cycle pouvant être lui-même substitué, dans lesquelles R₉, R₁₀ ,R₁₁ et R₁₂ identiques ou différents sont tels que définis précédemment; R₁₃, R₁₄ R₁₅ et R₁₆, identiques ou différents, représentent un hydrogène ou un radical alkyle en C₁-C₆. Dans la formule (I) ci-dessus, lorsque R₁ et/ou R₆ représentent un radical alkyle substitué, alors les substituants sont choisis également parmi les radicaux -OSO2R, R représentant une radical alkyle linéaire ou ramifié en C1-C4 ou un radical aromatique éventuellement substitué. A titre d'exemple, on peut citer les radicaux -OH, -OR₉, -NH₂, -NHR₁₀, -NR₁₁R₁₂, -COR₁₃, les radicaux cycliques type imidazole, pipérazine, pyrrolidine, pyridine, pipéridine, morpholine, pyrimidine.

A titre d'exemples de dérivés de formule (I), on peut citer les composés suivants ou leurs sels d'addition ou solvates :

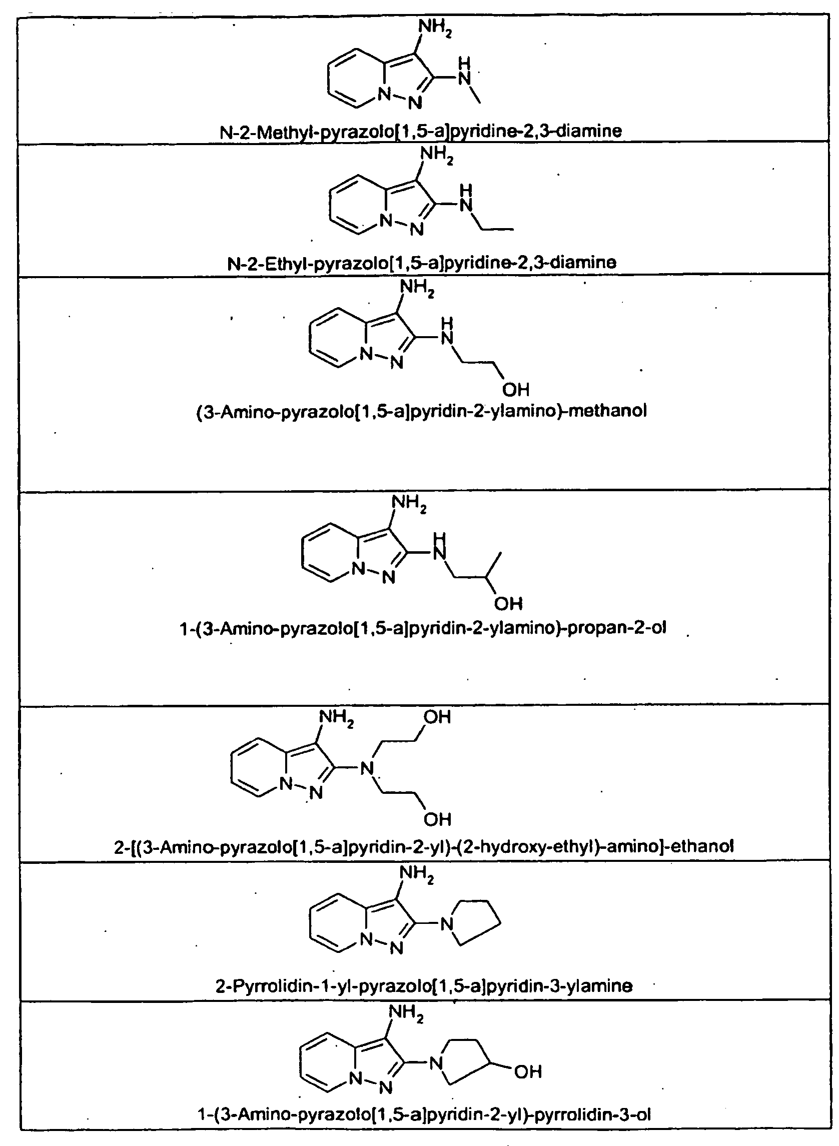

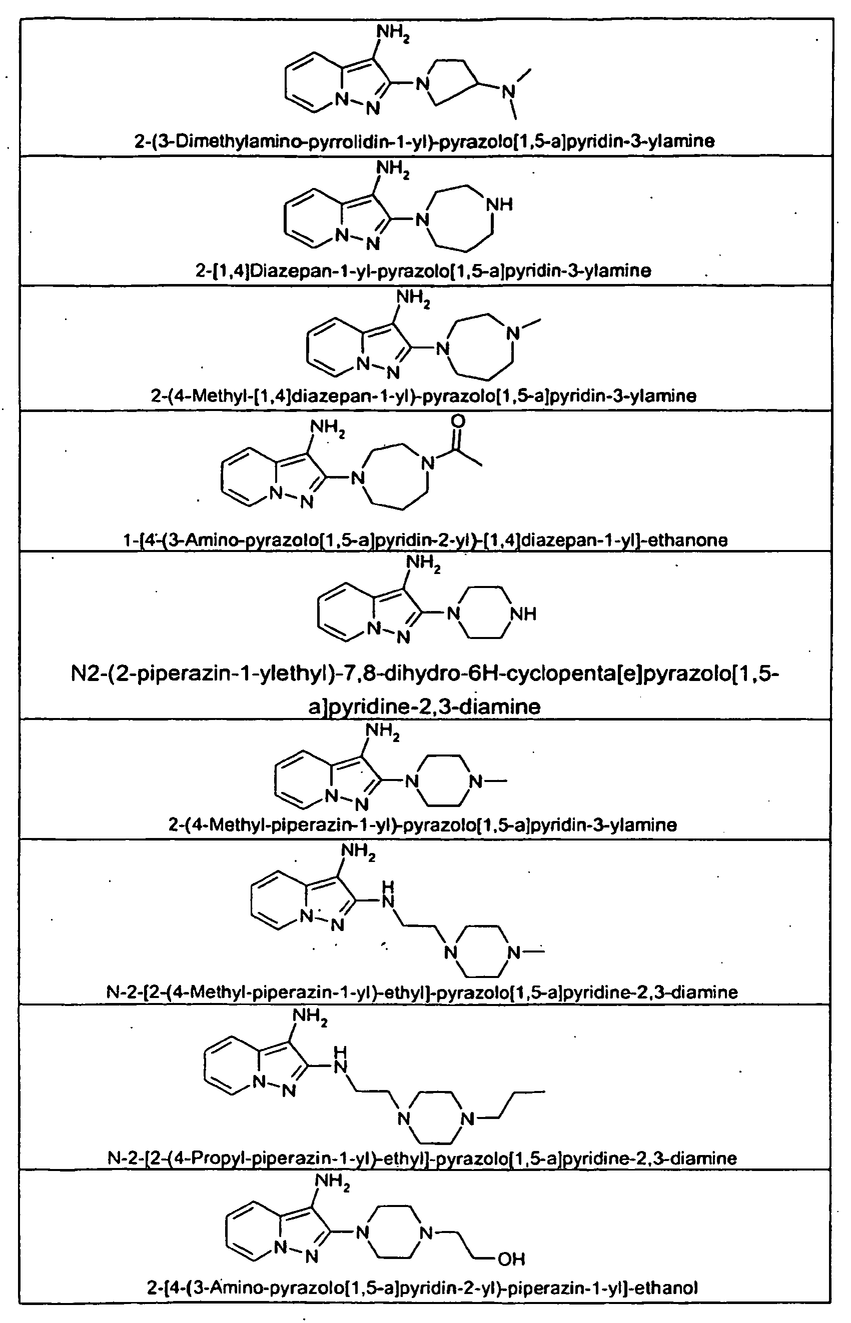

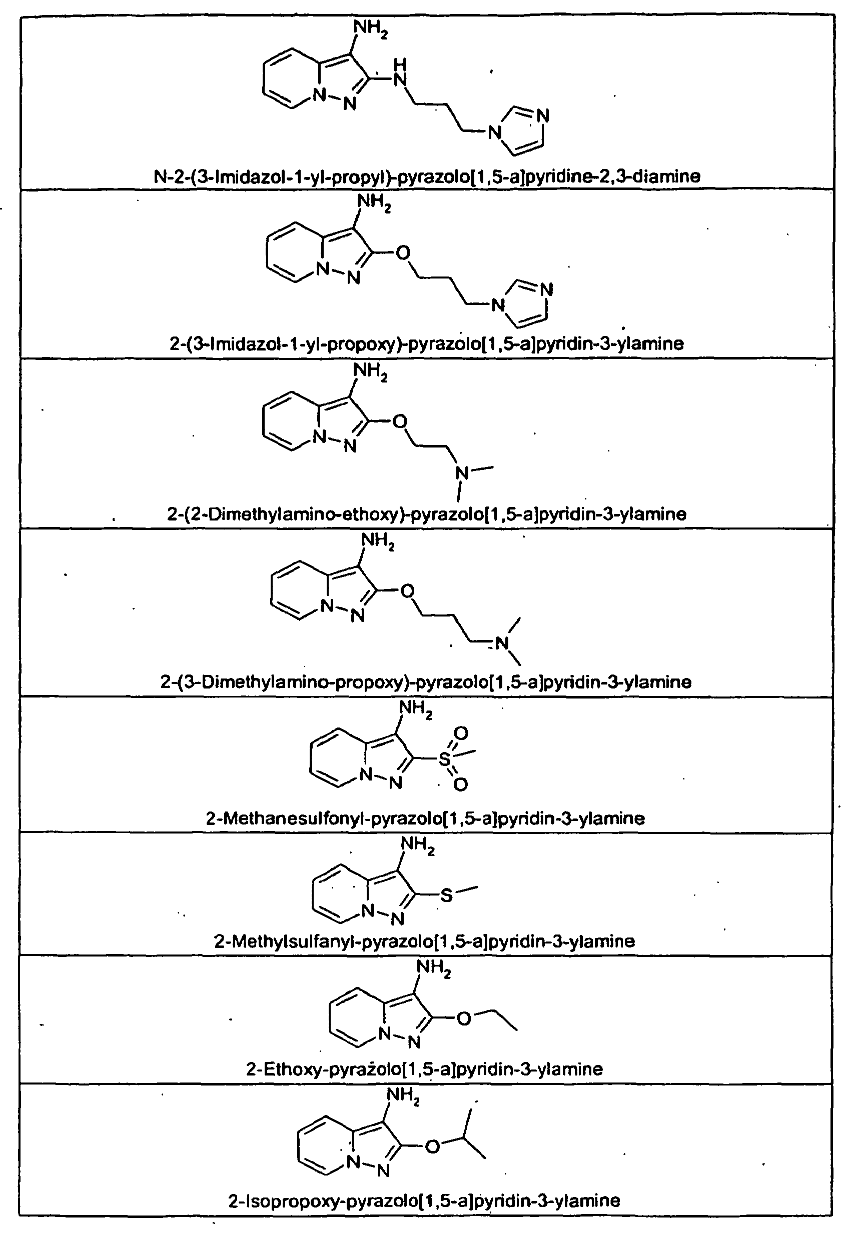

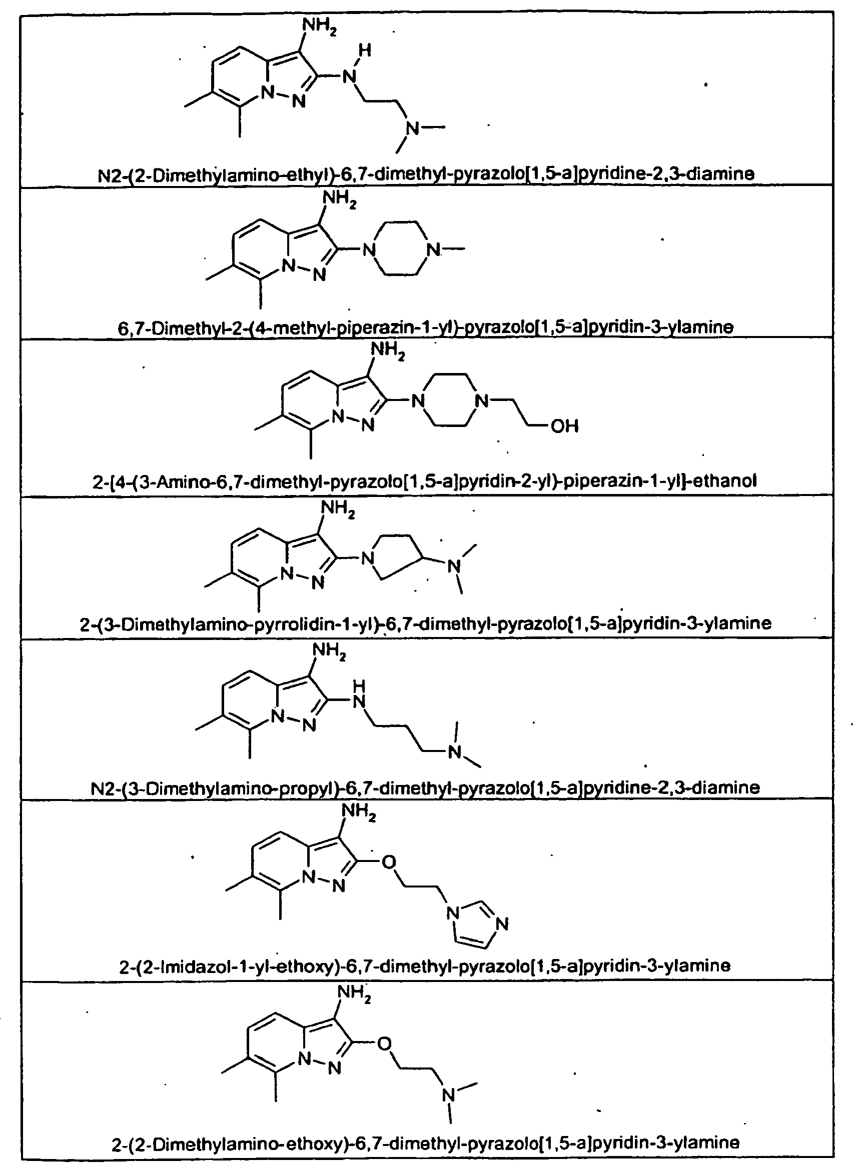

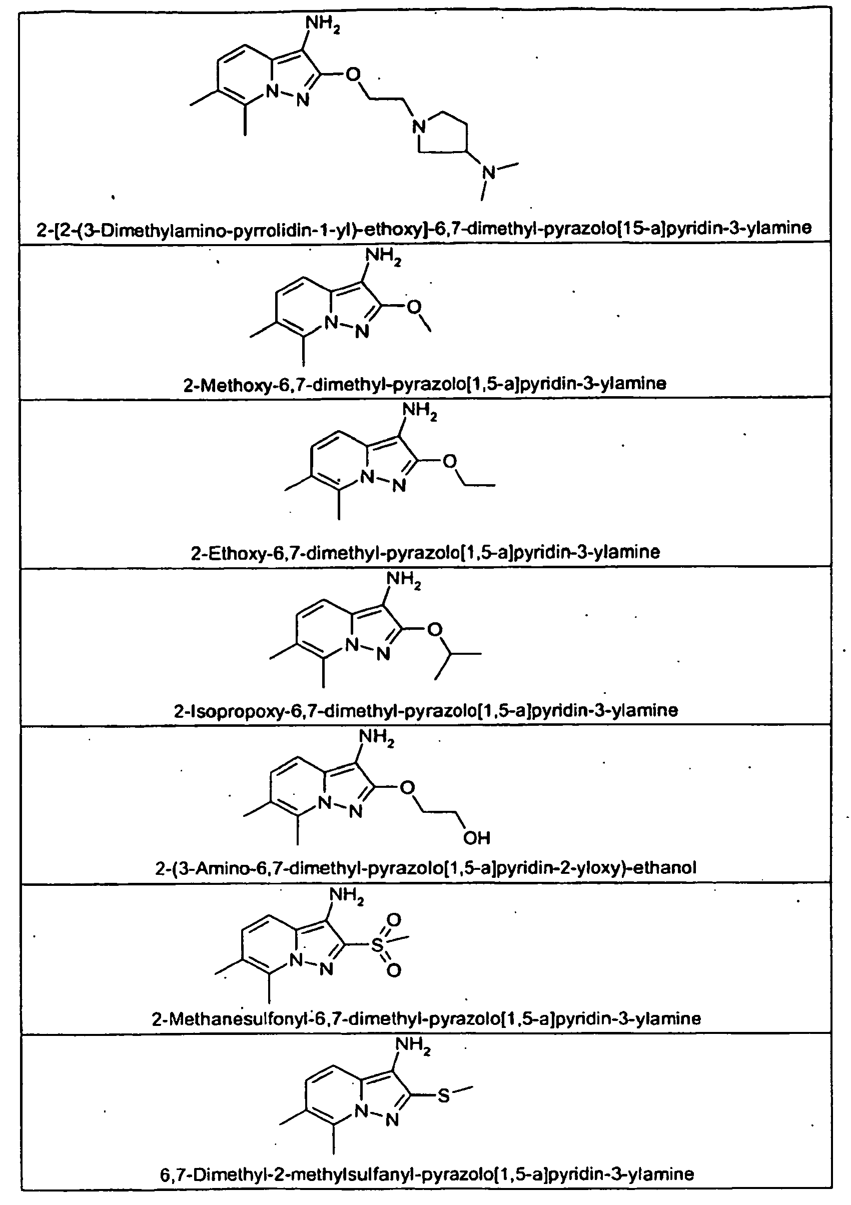

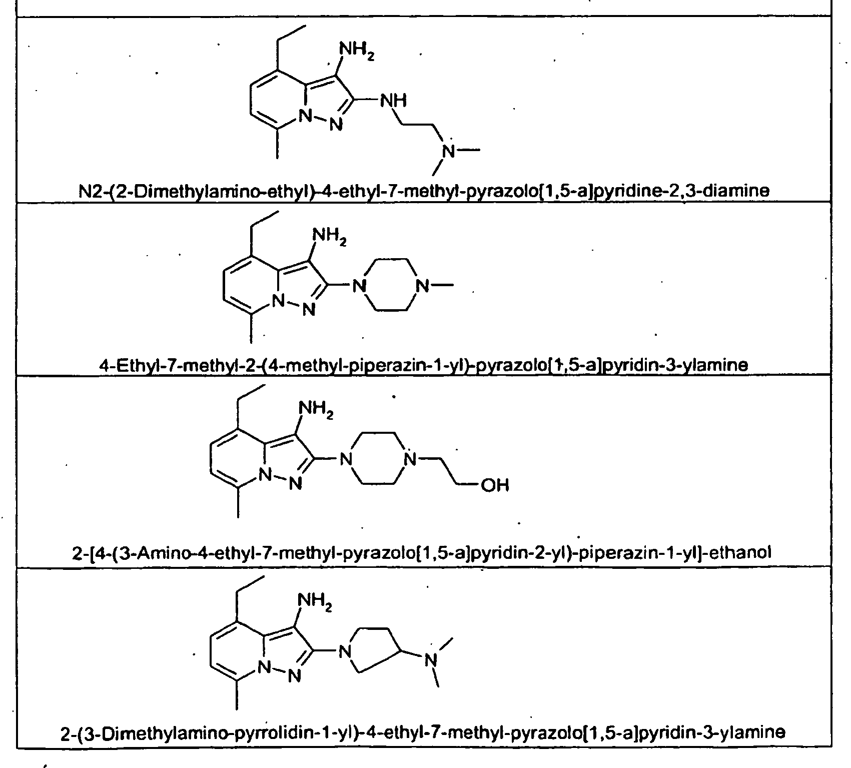

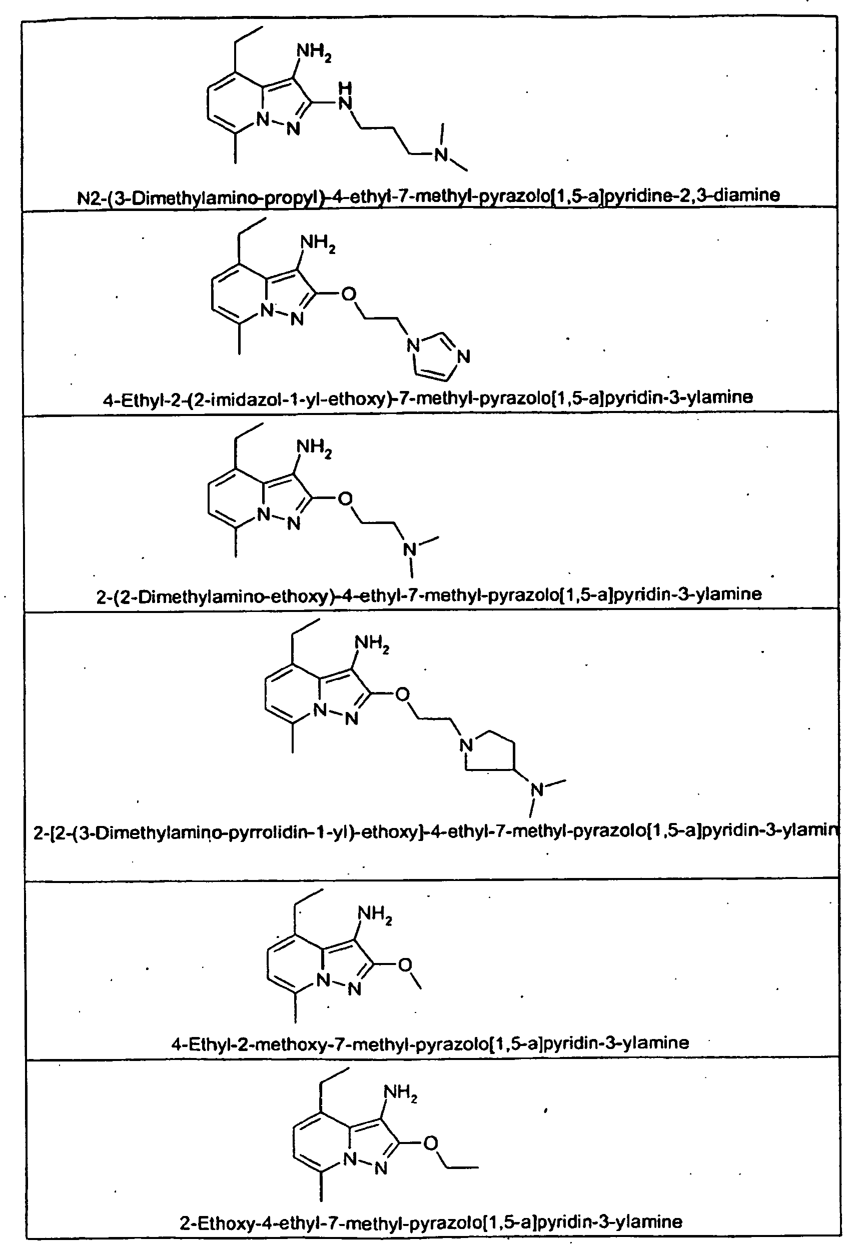

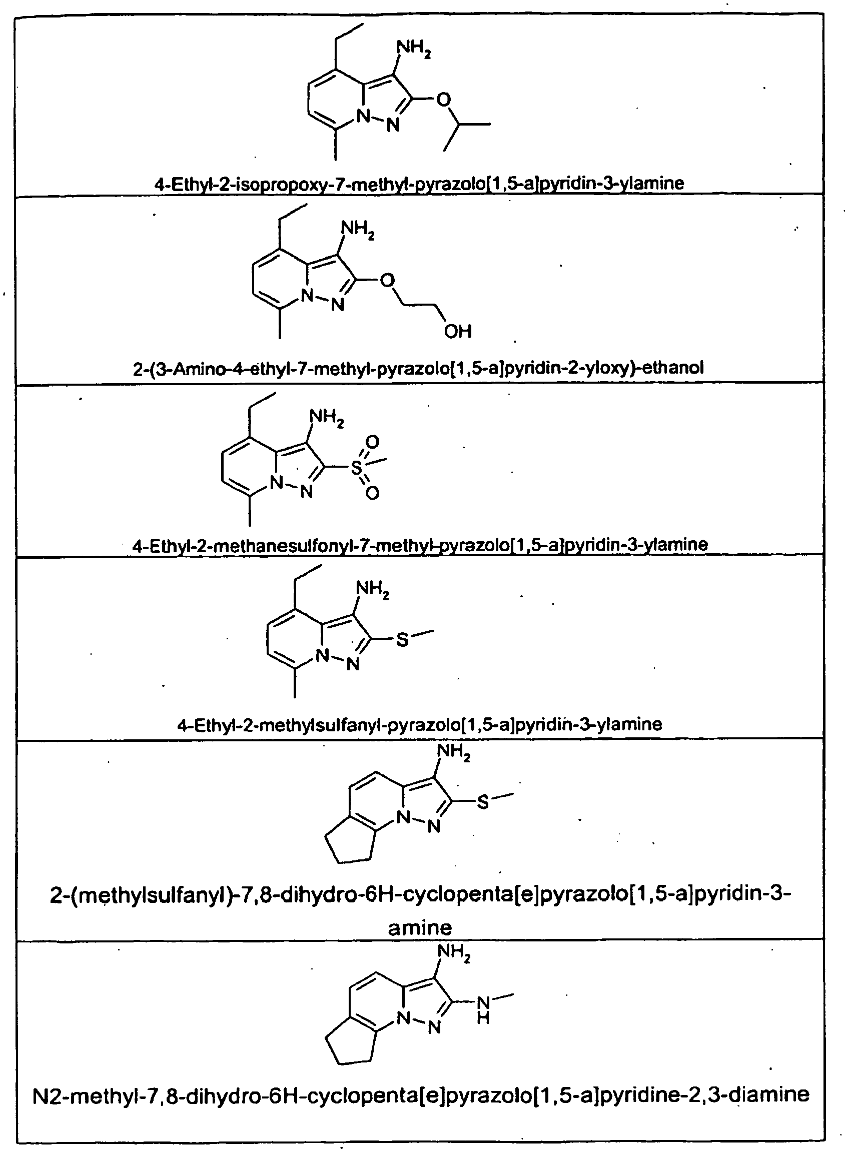

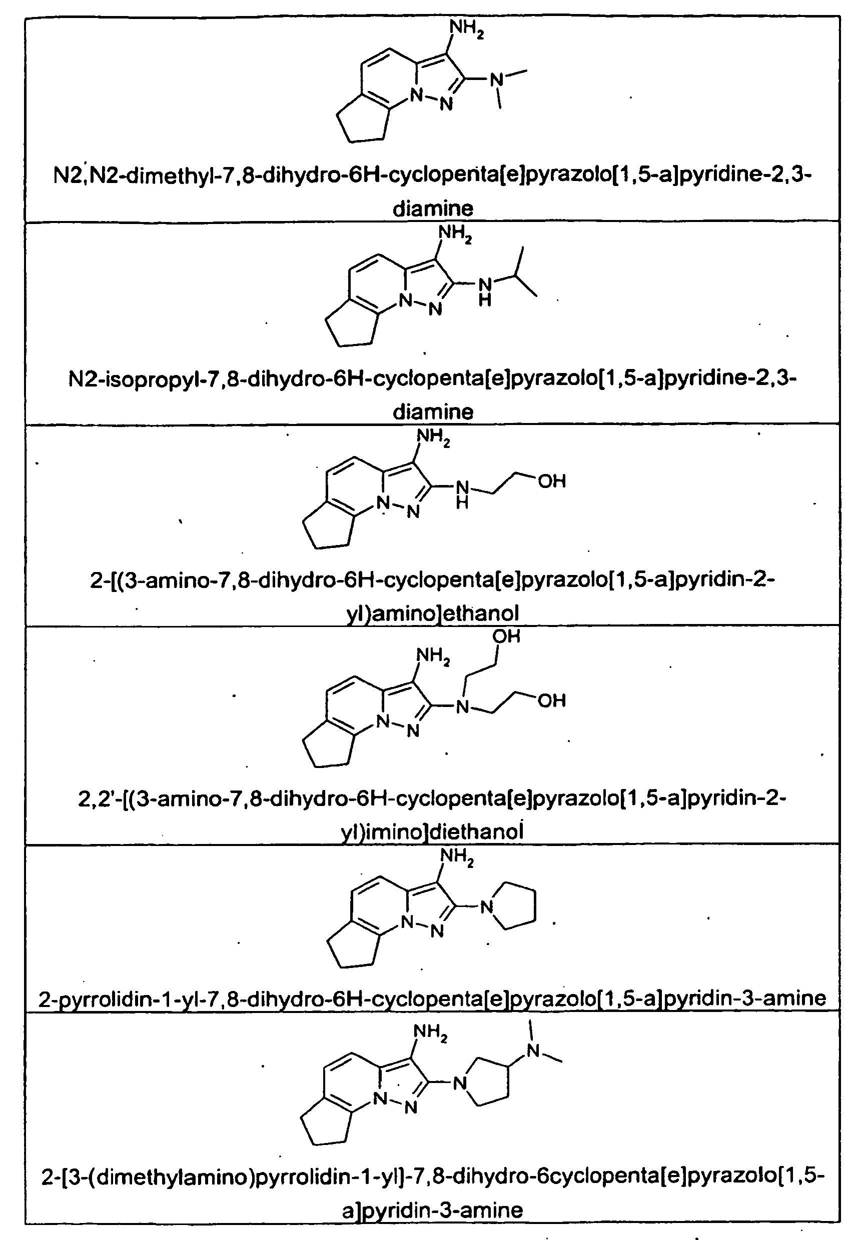

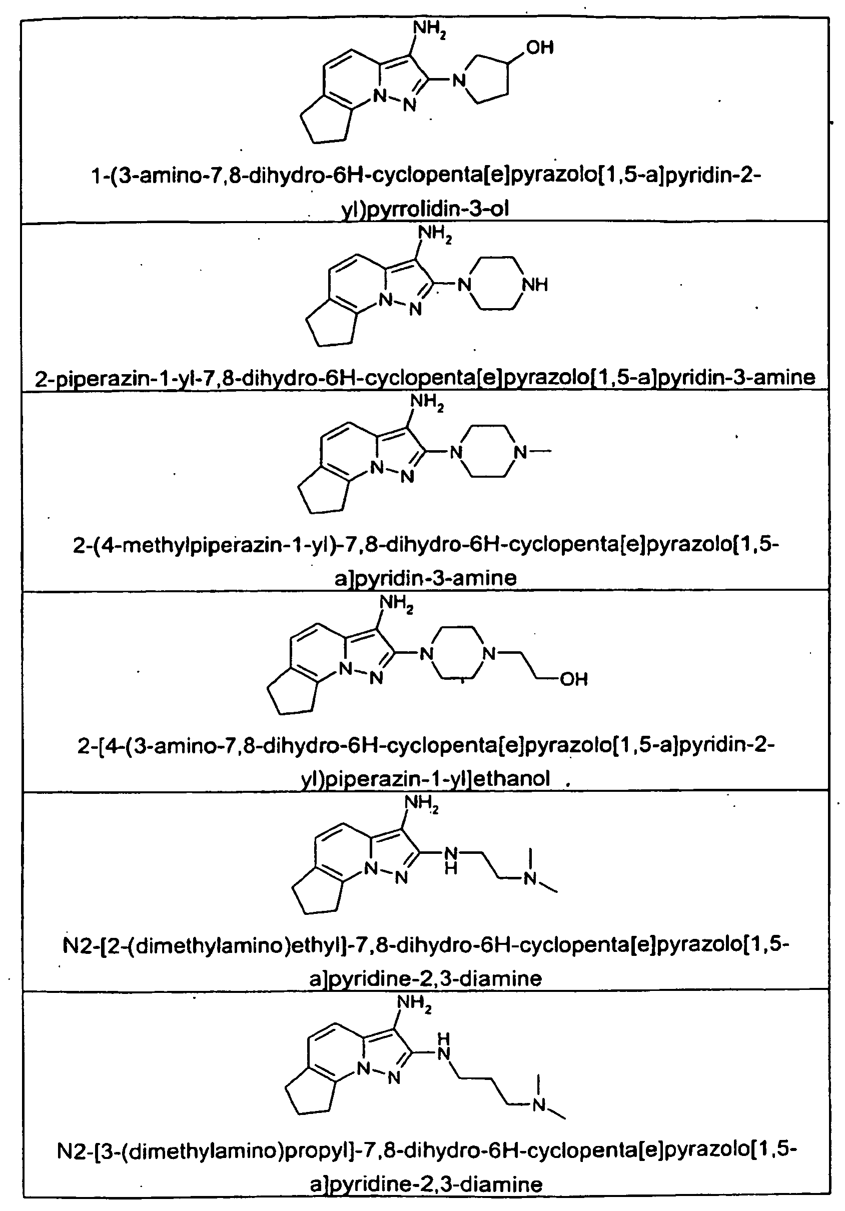

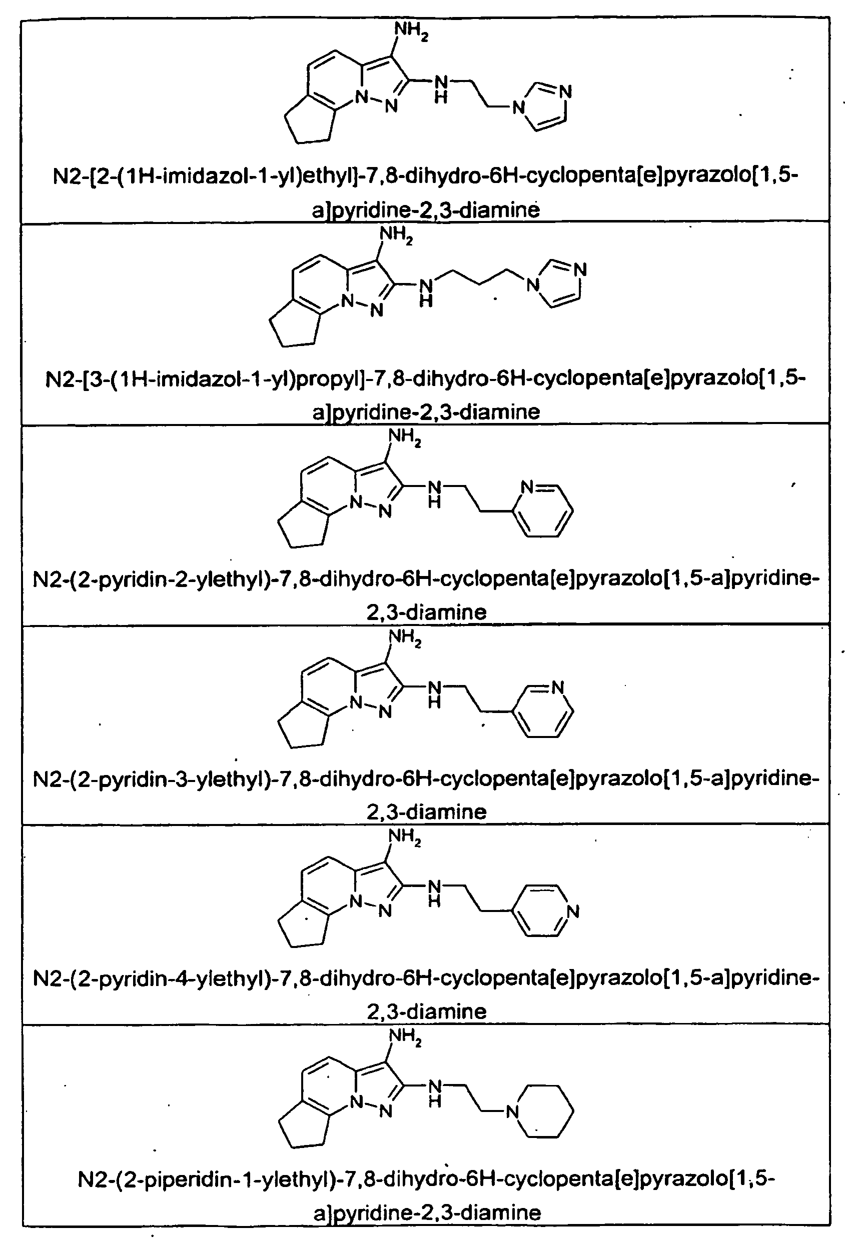

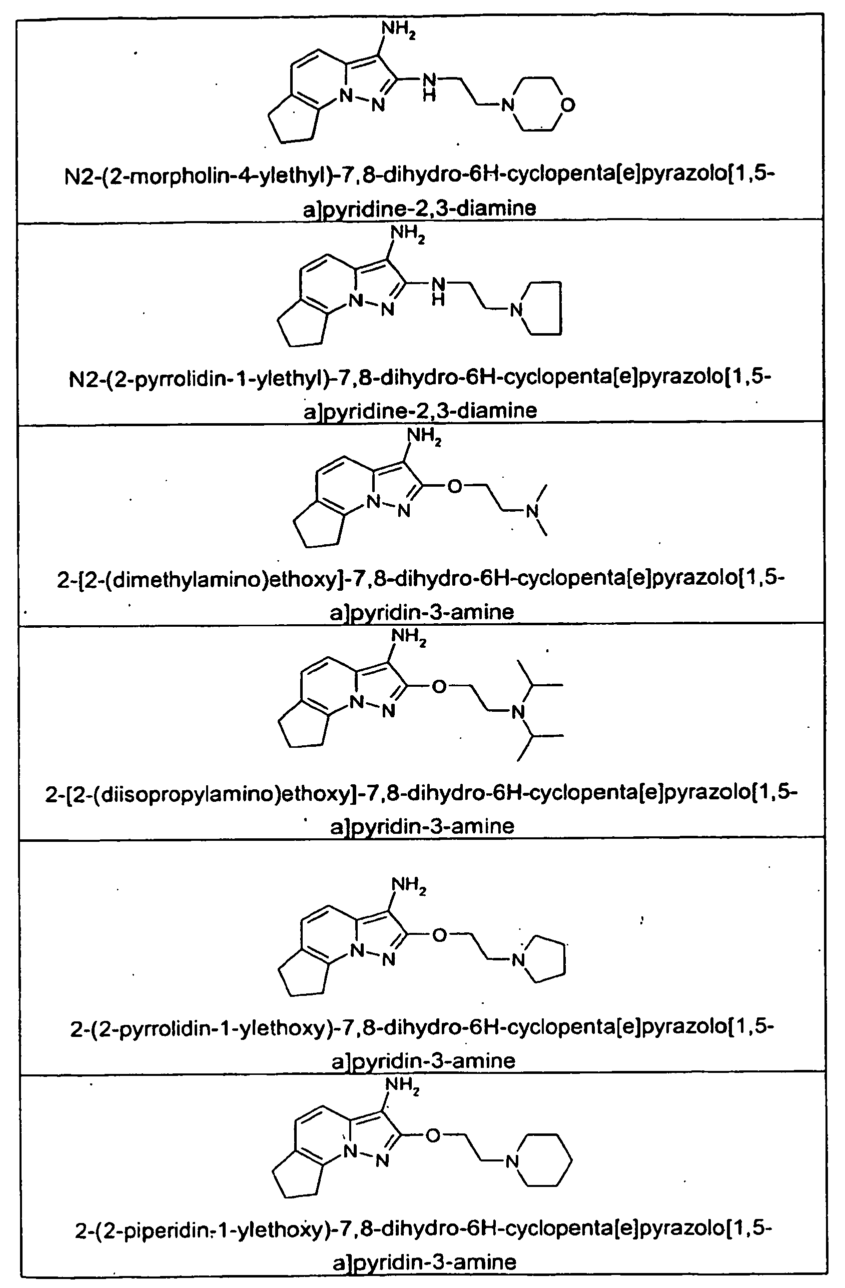

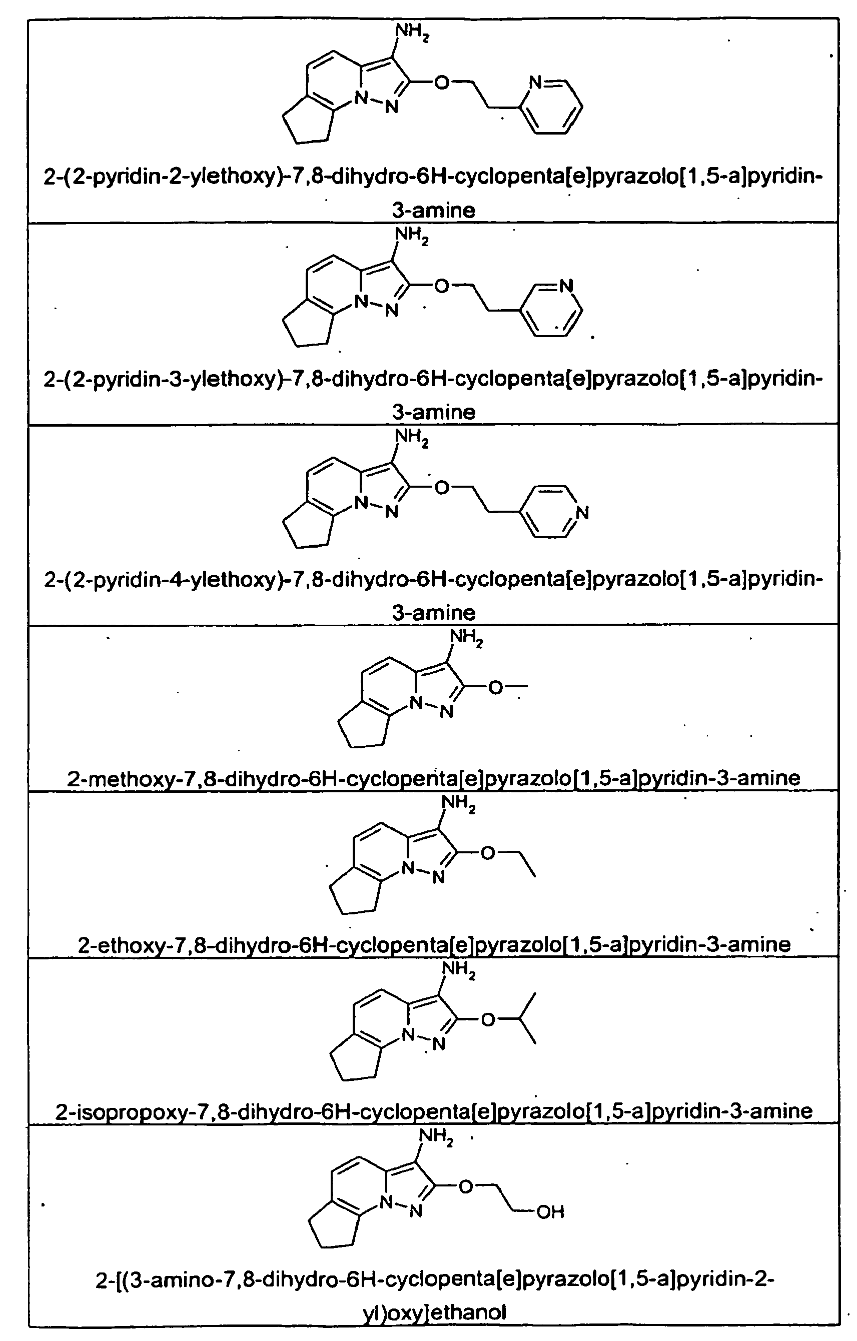

Selon un mode de réalisation particulier de l'invention, la base d'oxydation de formule (I) ci-dessus est telle que Z₁ représente un atome d'oxygène, , un radical NR₆ ou un radical NR₆ formant avec R₁ un hétérocycle.

Selon le mode de réalisation particulier dans lequel R₁ et R₆ forment ensemble un hétérocycle avec l'atome d'azote auquel ils sont rattachés, alors l'hétérocycle peut être choisi parmi les imidazoles, les pipérazines, les pyrrolidines, les diazépanes, ces hétérocycles pouvant être substitués ou non..

Lorsque R₂, R₃, R₄ et R₅ représentent un radical alkyle substitué, alors ce radical alkyle peut être notamment substitué par un groupement choisi parmi OH, OR₉, NH₂, NHR₁₀, NR₁₁R₁₂ ou SR₉ dans lesquelles R₉, R₁₀,R₁₁ et R₁₂ identiques ou différents sont tels que définis précédemment.

A titre d'exemple, on peut citer les radicaux méthyle, éthyle, hydroxyéthyle, aminoéthyle, propyle, butyle. Selon une variante, R₂, R₃, R₄ et R₅ représentent indépendamment un atome d'hydrogène ou un méthyle, éthyle, propyle, isopropyle, butyle, tertiobutyle, ou R₄ et R₅ forment ensemble un (hétéro)cycle de 5 à 8 chaînons. Selon un mode de réalisation particulier, R₄ et R₅ forment ensembles un cycle saturé ou insaturé à 5 ou 8 chaînons, notamment de 5 à 6 chaînons, par exemple un cyclopentane ou cyclohexane, éventuellement substitué.

A titre de composés préférés, on peut citer les composés ci-dessous :
le dichlorhydrate de N2-éthylpyrazolo[1,5-a]pyridine-2,3-diamine,
le dichlorydrate de 2-(3-Amino-pyrazolo[1,5-a]pyridin-2-ylamino)-éthanol,
le dichlorhydrate de 1-(3-Amino-pyrazolo[1,5-a]pyridin-2-ylamino)-propan-2-ol,
le dichlorhydrate de N-2-(3-Imidazol-1-yl-propyl)-pyrazolo[1,5-a]pyridine-2,3-diamine,
le dichlorhydrate de 2-Pyrrolidin-1-yl-pyrazolo[1,5-a]pyridin-3-ylamine,
le dichlorhydrate de 2-(3-Diméthylamino-pyrrolidin-1-yl)-pyrazolo[1,5-a]pyridin-3-ylamine,
le dichlorhydrate de 2-Imidazol-1-yl-pyrazolo[1,5-a]pyridin-3-ylamine,
le dichlorhydrate de N2-(2-pyrrolidin-1-yléthyl)pyrazolo[1,5-a]pyridine-2,3-diamine,
le dichlorhydrate de 2-(3-Amino-pyrazolo[1,5-a]pyridin-2-yloxy)-éthanol,
le dichlorhydrate de 2-éthoxy--pyrazolo[1,5-a]pyridin-3-ylamine,
le chlorhydrate de 2-Méthoxy-7-méthyl-pyrazolo[1,5-a]pyridin-3-ylamine,
le dichlorhydrate de 4-éthyl-2-méthoxy-7-methyl-pyrazolo[1,5-a]pyridin-3-ylamine,
le dichlorhydrate de 4-éthyl-7-methyl-2-pyrrolidin-1-yl-pyrazolo[1,5-a]pyridin-3-ylamine,
le dichlorhydrate de N2-(2-pipéridin-1-ylethyl)pyrazolo[1,5-a]pyridine-2,3-diamine,
le dichlorhydrate de N2-[2-(diisopropylamino)éthyl]pyrazolo[1,5-a]pyridine-2,3-diamine,
le dichlorhydrate de N2-[2-(diéthylamino)ethyl]pyrazolo[1,5-a]pyridine-2,3-diamine,
le dichlorhydrate de N2-(2-pyridin-3-yléthyl)pyrazolo[1,5-a]pyridine-2,3-diamine,
le dichlorhydrate de N2-(2-pyridin-4-yléthyl)pyrazolo[1,5-a]pyridine-2,3-diamine,
le dichlorhydrate de N2-(2-pyridin-2-yléthyl)pyrazolo[1,5-a]pyridine-2,3-diamine diamine,
le N2-(2-pipérazin-1-ylethyl)pyrazolo[1,5-a]pyridine-2,3-diamine,
le dichlorhydrate de N2-[2-(4-méthylpiperazin-1-yl)éthyl]pyrazolo[1,5-a]pyridine-2,3-diamine,
le dichlorhydrate de N2-(2-morpholin-4-yléthyl)pyrazolo[1,5-a]pyridine-2,3-diamine,
le dichlorhydrate de N2-[2-(diméthylamino)éthyl]-7,8-dihydro-6H-cyclopenta[e]pyrazolo[1,5-a]pyridine-2,3-diamine,
le chlorhydrate de 2-[(3-amino-7,8-dihydro-6H-cyclopenta[e]pyrazolo[1,5-a]pyridin-2-yl)amino]éthanol,
le chlorhydrate de 2-méthoxy-7,8-dihydro-6H-cyclopenta[e]pyrazolo[1,5-a]pyridin-3-amine,
le chlorhydrate de 2-isopropoxy-7,8-dihydro-6H-cyclopenta[e]pyrazolo[1,5-a]pyridin-3-amine,
le dichlorhydrate de 2-[2-(diméthylamino)ethoxy]pyrazolo[1,5-a]pyridin-3-amine,
le dichlorhydrate de 2-(2-pyrrolidin-1-yléthoxy)pyrazolo[1,5-a]pyridin-3-amine,
le dichlorhydrate de 2-(2-piperidin-1-yléthoxy)pyrazolo[1,5-a]pyridin-3-amine,
le dichlorhydrate de 2-(2-morpholin-4-yléthoxy)pyrazolo[1,5-a]pyridin-3-amine,
le dichlohrydrate de 2-[2-(diisopropylamino)éthoxy]pyrazolo[1,5-a]pyridin-3-amine,
le dichlorhydrate de 2-(2-pyridin-2-yléthoxy)pyrazolo[1,5-a]pyridin-3-amine,
le dichlorhydrate de 2-(2-pyridin-3-yléthoxy)pyrazolo[1,5-a]pyridin-3-amine,
le dichlorhydrate de 2-(2-pyridin-4-yléthoxy)pyrazolo[1,5-a]pyridin-3-amine.

La ou les bases d'oxydation de l'invention sont en général présentes chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les para-phénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis précédemment et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique; la 2-méthoxy-pyrazolo[1,5-a]pyridine-3-ylamino; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol; le 2-[(3-amino-pyrazolo[1,5-a]pyridin-7-yl)-(2-hydroxyéthylpamino]-éthanol; le 3-amino-pyrazolo[1,5-a]pyridine-5-ol; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation additionnelles autres que celles de formule (I) présentes dans la composition de l'invention sont en général présentes chacune en quantité comprise entre 0,001 à 10 % en poids environ du poids total de la composition tinctoriale, de préférence entre 0,005 et 6 %.

D'une manière générale, les sels d'addition de ces bases d'oxydation additionnelles et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 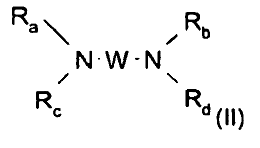 dans laquelle W est un reste propylène éventuellement substitué par un groupement hydroxyle ou un radical alkyle en C₁-C₄; Rₐ, R_{b}, R_{c}, et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

La présente invention a également pour objet l'utilisation pour la teinture d'oxydation des fibres kératiniques, et en particulier des fibres kératiniques humaines telles que les cheveux, d'un dérivé pyrazolopyridine de formule (I) ou d'un de ses sels d'addition tel que défini précédemment.

Les composés de formule (I) utiles dans la présente invention peuvent être obtenus à partir d'intermédiaires et de voies de synthèse décrites dans la littérature et notamment dans les références suivantes : J. Het. Chem., 2001, 38(3), 613-616 , Helvetica Chimica Acta, 1950, 33, 1183-1194, J. Org. Chem., 23, 2029 (1958), J. Am. Chem. Soc., 73, 3240 (1951), J. Am. Chem. Soc., 84, 590 (1962), Justus Liebig Ann. Chem., 686, 134 (1965), Tetrahedron. Lett., 31, 2859-2862 (1973), les brevets US4128425 et US 2841584 et les références citées.

Selon un mode de réalisation particulier, la synthèse des composés de formule (I) peut être réalisée selon le schéma suivant : 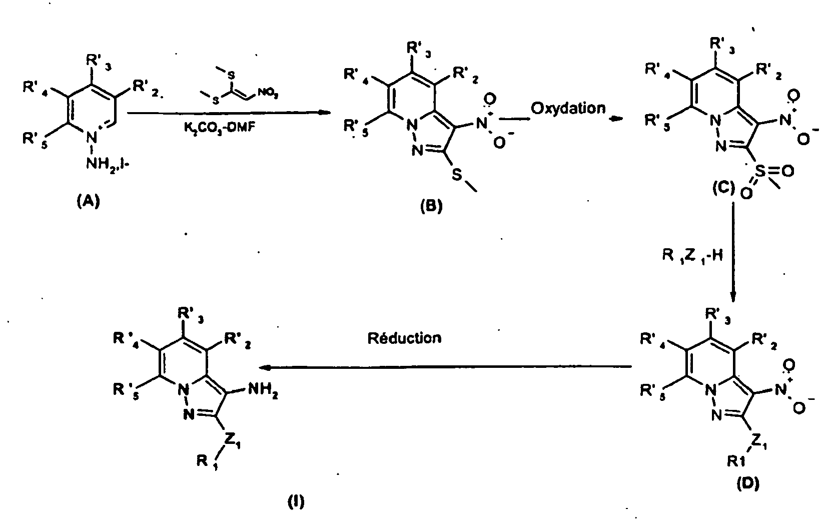 dans lequel R'₂, R'₃, R'₄ et R'₅ ont les mêmes significations que R₂, R₃, R₄ et R₅ ou en sont des précurseurs.

A titre d'exemple, lorsque R₁ représente un radical alkyle (C₁-C₁₀)-NR₁₁R₁₂ ou un radical alkyle (C₁-C₁₀)-OR₁₁, alors le procédé de synthèse utilisé peut être le suivant :

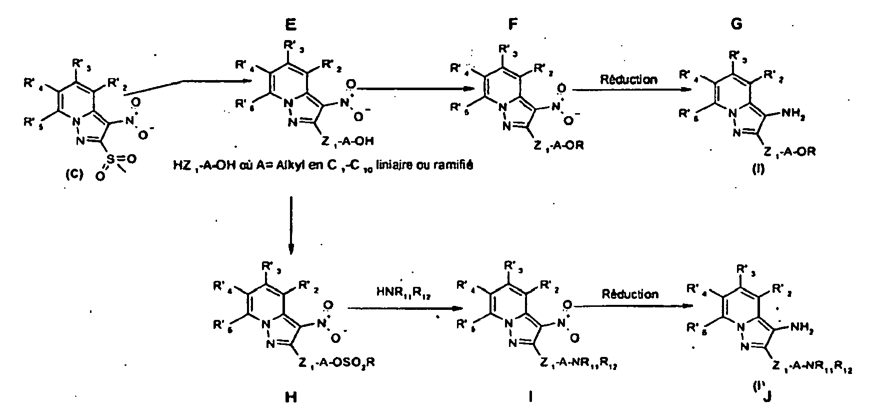

Des précisions sur ces modes de synthèse sont données dans les exemples décrits ci-après.

L'invention a aussi pour objet les composés intermédiaires (A) et (B) tels que définis ci-dessus dans lesquels au moins un des radicaux R'₂, R'₃, R'₄ ou R'₅ est différent d'un atome d'hydrogène à l'exclusion du composé pour lequel R'₂ = Me lorsque tous les autres substituants sont l'hydrogène.

L'invention a de plus pour objet les composés intermédiaires de formule (C), (D), (F), (H), (I) tels que définis ci-dessus

### EXEMPLES

### Exemple 1 : dichlorhydrate de N-2-éthylpyrazolo[1,5-a]pyridine-2,3-diamine

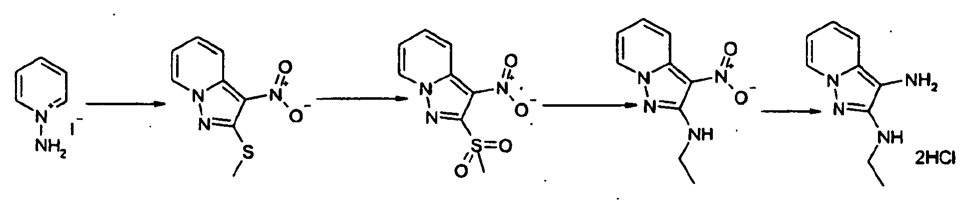

### Synthèse du 2-méthylsulfanyl-3-nitro-pyrazolo[1,5-a]pyridine

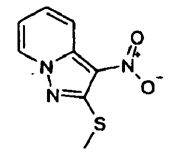

Dans un tricol de deux litres, équipé d'une agitation mécanique et d'une sonde de température interne et placé sous balayage d'azote, on prépare une solution de 111 g d'iodure de 1-N-aminopyridinium (0,5 mol) dans le DMF (500ml).

Le carbonate de potassium (207,3, 3éq.) est alors ajouté en une fois, suivi du 1,1-bis(méthylthio)-2-nitroéthylène (165,2g, 2 éq.) également en une seule fois. Le milieu réactionnel prend en masse. 500 ml de DMF sont ajoutés afin de rendre le milieu réactionnel plus fluide.

Après 48 heures d'agitation à la température ambiante, le milieu réactionnel est versé sur 4 litres d'eau glacée. Le précipité qui se forme est filtré et lavé abondamment à l'eau (5 litres) puis séché à 80°C sous vide.

Le solide ainsi obtenu est débarrassé du 1,1-bis(méthylthio)-2-nitroéthylène en excès (30% molaire déterminé par 1H-RMN) par un ré-empatage dans l'acétate d'éthyle. Après essorage et séchage, on obtient 72g d'un solide jaune beige correspondant au produit attendu.

RMN ¹H(300 MHz, DMSO-d6) :
8,98 (1H, d) ; 8,20 (1H, d), 7,89 (1H, m), 7,36 (1H, m), 2,63 (3H, s).

### Synthèse du 2-méthanesulfonyl-3-nitro-pyrazolo[1,5-a]pyridine

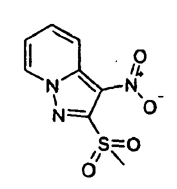

Dans un tricol de 4 litres, équipé d'une agitation mécanique et d'une sonde de température interne, sont chargés successivement 880g d'oxone (5 éq.), 2 litres d'eau et 60g de 2-méthyleulfonyl-3-nitro-pyrazolo[1,5-a]pyridine (0,287 éq.) obtenu précédemment. Le tout est agité à la température ambiante.

Pour compléter la réaction, de l'oxone (120g, 0,7 éq.) est additionnée et après 4 heures sous agitation à la température ambiante, la réaction est terminée.

Le solide formé est essoré et lavé abondamment à l'eau jusqu'à l'obtention d'un filtrat ne contenant plus de peroxyde. Il est ensuite placé sous vide à 40°C sur P₂O₅.

59g de produit attendu sont obtenus sous forme d'une poudre jaune-beige.

RMN ¹H (300 MHz, DMSO-d₆):
9.15 (1H, d) ; 8,35 (1H, d), 8,03 (1H, m), 7,56 (1 H, m), 3,59 (3H, s).

### Synthèse du éthyl-(3-nitro-pyrazolo[1,5-a]pyridin-2-yl)-amine.

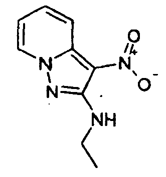

Dans un tricol de 10 ml, surmonté d'un réfrigérant à boules, d'un thermomètre et d'une agitation magnétique, on introduit, 3ml de N-méthypyrrolidinone, 2.41g (0,01 moles) 2-méthanesulfonyl-3-nitro-pyrazol[1,5-a]pyridine et 4.015g d'éthylamine. Sous agitation, on porte à 70°C à l'aide d'un bain d'huile, pendant 5 heures.

Le composé jaune, isolé en versant le milieu réactionnel sur de l'eau, est essoré sur fritté puis lavé à l'eau plusieurs fois. Après séchage sous vide, en présence de P₂O₅, on récupère 1.68g de solide jaune correspondant au composé attendu.

Les analyses RMN (¹H 400 MHz et ¹³C 100,61 MHz DMSO d₆) sont conformes à la structure attendue. La masse du composé attendu C₉H₁₀N₄O₂ est détectée en spectrométrie de masse. Les ions quasi-moléculaires [M+H]+, [M+Na]+ et [M-H]- de la molécule attendue, C₉H₁₀N₄O₂, sont principalement détectés.

### Synthèse du dichlorhydrate de N-2-ethylpyrazolo[1,5-a]pyridine-2,3-diamine

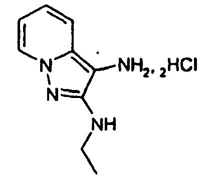

Dans un tricol de 500 ml, surmonté d'un réfrigérant à boules, d'un thermomètre et d'une agitation magnétique, on introduit 400 ml d'éthanol et 11 g de poudre de zinc et on porte au reflux.

On ajoute alors 2.25g de chlorure d'ammonium solubilisé dans 0.5ml d'eau et 3g de éthyl-(3-nitro-pyrazolo[1,5-a]pyridin-2-yl)-amine sont ajoutés par portion en 15 minutes et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi, le zinc est lavé avec de l'éthanol et les filtrats réunis sont évaporés à sec.

Après séchage sous vide, en présence de P₂O₅ et de pastilles de soude, on obtient 2.1g d'une poudre bleue correspondant au composé attendu.

Les analyses RMN (¹H 400 MHz et ¹³C 100,61 MHz DMSO d₆) sont conformes à la structure attendue. Les ions quasi-moléculaires [M+H]+ et [M+Na]+ de la molécule attendue sont détectés : C₉H₁₂N₄

### Exemple 2 : dichlorydrate de 2-(3-Amino-pyrazolo[1,5-a]pyridin-2-ylamino)-éthanol,

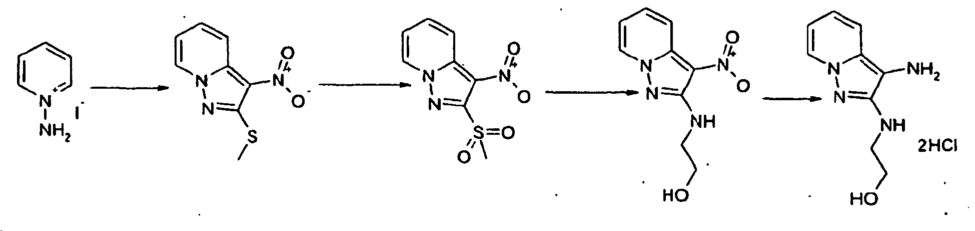

### Synthèse du 2-[(3-nitropyrazolo[1,5-a]pyridin-2-yl)amino]éthanol

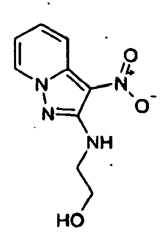

Dans un tricol de 50 ml, surmonté d'un réfrigérant à boules, d'un thermomètre et d'une agitation magnétique, on introduit, 10ml de N-méthypyrrolidinone, 7.25g (0,03 moles) 2-méthanesulfonyl-3-nitro-pyrazol[1,5-a]pyridine et 6ml d'éthanolamine. Sous agitation, on porte à 70°C à l'aide d'un bain d'huile, pendant 5 heures.

Le composé jaune, isolé en versant le milieu réactionnel sur de l'eau, est essoré sur fritté puis lavé à l'eau plusieurs fois. Après séchage sous vide, en présence de P₂O₅, on récupère 6.45g de solide jaune correspondant au composé attendu.

Les analyses RMN (¹H 400 MHz et ¹³C 100,61 MHz DMSO d₆) sont conformes à la structure attendue.

Les ions quasi-moléculaires [M+H]+, [M+Na]+ et [M-H]- de la molécule attendue, C₉H₁₀N₄O₃, sont principalement détectés

### Synthèse du dichlorhydrate de 2-(3-Amino-pyrazolo[1,5-a]pyridin-2-ylamino)-éthanol.

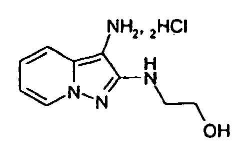

Dans un tricol de 500 ml, surmonté d'un réfrigérant à boules, d'un thermomètre et d'une agitation magnétique, on introduit 350 ml d'éthanol et 5g de poudre de zinc et on porte au reflux.

On ajoute alors 1g de chlorure d'ammonium solubilisé dans 0.5ml d'eau et 2g de 2-[(3-nitropyrazolo[1,5-a]pyridin-2-yl)amino]ethanol sont ajoutés par portion en 15 minutes et le reflux est maintenu 4 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi, le zinc est lavé avec de l'éthanol et les filtrats réunis sont évaporés à sec.

Après séchage sous vide, en présence de P₂O₅ et de pastilles de soude, on obtient 1.23 g d'une poudre bleutée correspondant au composé attendu.

Les analyses RMN (¹H 400 MHz et ¹³C 100,61 MHz DMSO d₆) sont conformes à la structure attendue.

Les ions quasi-moléculaires [M+H]+, [M+Na]+ et [M-H]- de la molécule attendue, C₉H₁₂N₄O, sont principalement détectés

### Exemple 3:1-(3-Amino-pyrazolo[1,5-a]pyridin-2-ylamino)-propan-2-ol

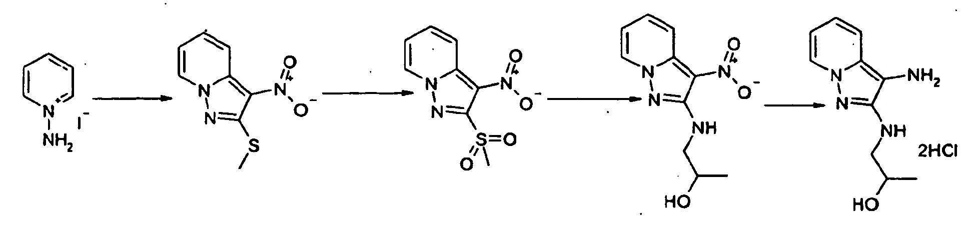

### Synthèse du 1-[(3-nitropyrazolo[1,5-a]pyridin-2-yl)amino]propan-2-ol

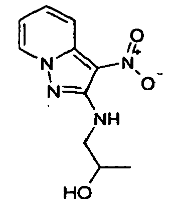

Dans un tricol de 50 ml, surmonté d'un réfrigérant à boules, d'un thermomètre et d'une agitation magnétique, on introduit, 10ml de N-méthypyrrolidinone, 7.23g (0,03 moles) 2-méthanesulfonyl-3-nitro-pyrazol[1,5-a]pyridine et 7ml de 1-amino-2-propanol. Sous agitation, on porte à 70°C à l'aide d'un bain d'huile, pendant 5 heures.

Le composé jaune, isolé en versant le milieu réactionnel sur de l'eau, est essoré sur fritté puis lavé à l'eau plusieurs fois. Après séchage sous vide, en présence de P₂O₅, on récupère 6.02g de solide jaune correspondant au composé attendu.

Les analyses RMN (¹H 400 MHz et ¹³C 100,61 MHz DMSO d₆) sont conformes à la structure attendue.

### Synthèse du dichlorhydrate de 1-(3-Amino-pyrazolo[1,5-a]pyridin-2-ylamino)-propan-2-ol

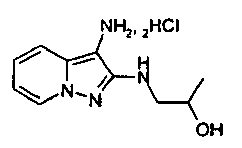

Dans un tricol de 500 ml, surmonté d'un réfrigérant à boules, d'un thermomètre et d'une agitation magnétique, on introduit 350 ml d'éthanol et 5g de poudre de zinc et on porte au reflux.

On ajoute alors 1g de chlorure d'ammonium solubilisé dans 0.5ml d'eau et 2g de1-[(3-nitropyrazolo[1,5-a]pyridin-2-yl)amino]propan-2-ol sont ajoutés par portion en 15 minutes et le reflux est maintenu 4 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi, le zinc est lavé avec de l'éthanol et les filtrats réunis sont évaporés à sec.

Après séchage sous vide, en présence de P₂O₅ et de pastilles de soude, on obtient 1.5g d'une poudre bleutée correspondant au composé attendu.

Les analyses RMN (¹H 400 MHz et ¹³C 100,61 MHz DMSO d₆) sont conformes à la structure attendue. Les ions quasi moléculaires [M+H]+, [M+Na]+ de la molécule attendue C₁₀H₁₄N₄O sont principalement détectés.

### Exemple 4 : dichlorhydrate de N-2-(3-Imidazol-1-yl-propyl)-pyrazolo[1,5-a]pyridine-2,3-diamine

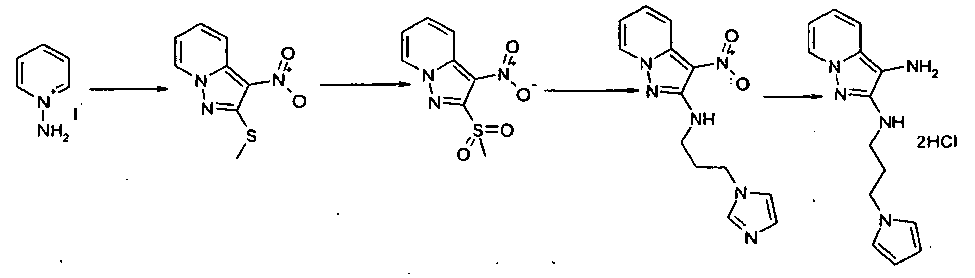

### Synthèse du N-[3-(1H-imidazol-1-yl)propyl]-3-nitropyrazolo[1,5-a]pyridin-2-amine

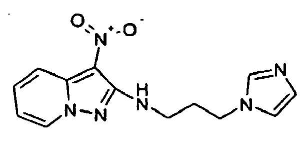

Dans un tricol de 100 ml, surmonté d'un réfrigérant à boules, d'un thermomètre et d'une agitation magnétique, on introduit 50 ml de N-méthypyrrolidinone, 5g (0,021 mole) de 2-méthanesulfanyl-3-nitro-pyrazol[1,5-a]pyridine et 26g de 3-(1H-imidazol-1-yl)propan-1-amine et on maintient l'agitation pendant 6 heures en suivant par CCM (éluant : 95:5 Dichlorométhane / Méthanol).

Le milieu est versé sur un mélange de 200g de glace et d'eau. Le composé jaune qui a cristallisé est essoré sur fritté n°3, lavé à l'eau 2x100ml puis avec 3x100ml d'éther isopropylique. Après séchage à 35°C sous vide, en présence de P₂O₅, pendant 12 heures, on récupère 5g de solide vert correspondant au composé attendu.

Les analyses RMN (¹H 400MHz et ¹³C 100,61 MHz DMSO d₆) et spectrophotométrie de masse sont conformes à la structure attendue. Les ions quasi moléculaires [M+H]+, [2M+H]+ de la molécule attendue C₁₃H₁₄N₆O₂ sont principalement détectés.

### Synthèse du dichlorhydrate de N-2-(3-Imidazol-1-yl-propyl)-pyrazolo[1,5-a]pyridine-2,3-diamine

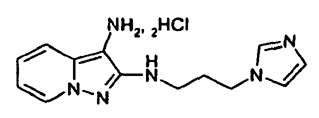

Dans un tricol de 500 ml, surmonté d'un réfrigérant à boules, d'un thermomètre et d'une agitation magnétique, on introduit 350 ml d'éthanol et 5g de poudre de zinc et on porte au reflux.

On ajoute alors 1g de chlorure d'ammonium solubilisé dans 0.5ml d'eau et 1g de N-[3-(1H-imidazol-1-yl)propyl]-3-nitropyrazolo[1,5-a]pyridin-2-amine sont ajoutés par portion en 15 minutes et le reflux est maintenu 4 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi, le zinc est lavé avec de l'éthanol et les filtrats réunis sont évaporés à sec.

Après séchage sous vide, en présence de P₂O₅ et de pastilles de soude, on obtient 0.5 g d'une poudre bleutée correspondant au composé attendu.

Les ions quasi moléculaires [M+H]+, [M+Na]+ de la molécule attendue C₁₃H₁₆N₆ sont principalement détectés

### Exemple 5 : dichlorhydrate de 2-Pyrrolidin-1-yl-pyrazolo[1,5-a]pyridin-3-ylamine

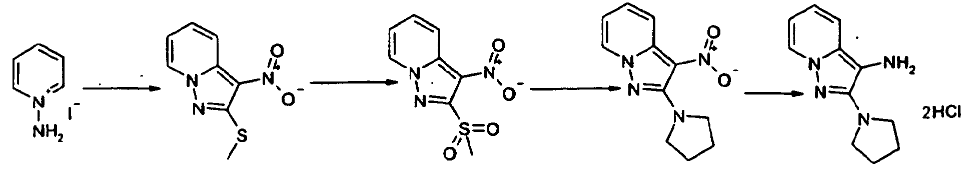

### Synthèse du 3-nitro-2-pyrrolidin-1-ylpyrazolo[1,5-a]-pyridine

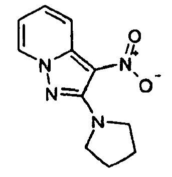

Dans un tricol de 50 ml, surmonté d'un réfrigérant à boules, d'un thermomètre et d'une agitation magnétique, on introduit, 10ml de N-méthypyrrolidinone, 7.23g (0,03 moles) 2-méthanesulfonyl-3-nitro-pyrazol[1,5-a]pyridine et 8ml de pyrrolidine. Sous agitation, on porte à 70°C à l'aide d'un bain d'huile, pendant 1 heure en suivant par CCM (éluant : 98:2 Dichlorométhane / Méthanol).

Le composé jaune, isolé en versant le milieu réactionnel sur de l'eau, est essoré sur fritté puis lavé à l'eau plusieurs fois. Après séchage sous vide, en présence de P₂O₅, on récupère 6.84g de solide jaune correspondant au composé attendu.

Les analyses RMN (¹H 400 MHz et ¹³C 100,61 MHz DMSO d₆) sont conformes à la structure attendue.

### Synthèse du dichlorhydrate de 2-Pyrrolidin-1-yl-pyrazolo[1,5-a]pyridin-3-ylamine

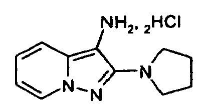

Dans un tricol de 500 ml, surmonté d'un réfrigérant à boules, d'un thermomètre et d'une agitation magnétique, on introduit 400ml éthanol et 5g de poudre de zinc et on porte au reflux.

On ajoute alors 1g de chlorure d'ammonium solubilisé dans 0.5ml d'eau et 1g de 3-nitro-2-pyrrolidin-1-ylpyrazolo[1,5-a]pyridine sont ajoutés par portion en 15 minutes et le reflux est maintenu 4 heures.

Lorsque la réduction est terminée, le zinc est éliminé par filtration sous argon sur un lit de célite et le filtrat recueilli dans une fiole contenant 50ml d'isopropanol chlorhydrique 6N préalablement refroidi, le zinc est lavé avec de l'éthanol et les filtrats réunis sont évaporés à sec.

Après séchage sous vide, en présence de P₂O₅ et de pastilles de soude, on obtient 1.2g d'une poudre bleutée correspondant au composé attendu.

Les analyses RMN (¹H 400 MHz et ¹³C 100,61 MHz DMSO d₆) sont conformes à la structure attendue.

¹H NMR (DMSO-d6): 1.91 (m, 4H), 3:46(m, 1H), 6.70 (m, 1H), 7.21 (m, 1H), 7.73 (m, 1H), 7.79(se, 2H), 8,42(m, 2H), 10.24(se, 3H).

Les ions quasi moléculaires [M+H]+, [M+Na]+ de la molécule attendue C₁₁H₁₄N₄ sont principalement détectés

### Exemple 6 : dichlorhydrate de 2-(3-Diméthylamino-pyrrolidin-1-yl)-pyrazolo[1,5-a]pyridin-3-ylamine

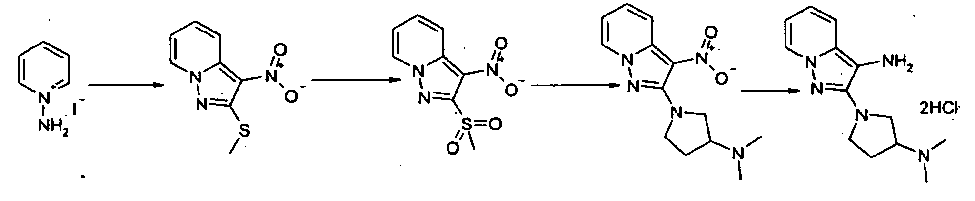

### Synthèse du N,N-dimethyl-1-(3-nitropyrazolo[1,5-a]pyridin-2-yl)pyrrolidin-3-amine

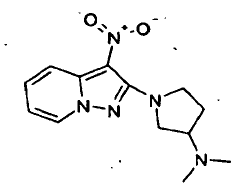

Dans un tricol de 100 ml, surmonté d'un réfrigérant à boules, d'un thermomètre et d'une agitation magnétique, on introduit 5 ml de N-méthypyrrolidinone, 3g de 2-(méthylsulfonyl)-3-nitropyrazolo[1,5-a]pyridine et 3,77 ml de 3(diméthylamino)pyrrolidine et on porte à 80°C pendant une heure.

Le milieu est versé sur un mélange de 200g de glace et d'eau. Le composé jaune qui a cristallisé est essoré sur fritté, lavé à l'eau 2x100ml puis avec 3x100ml d'éther isopropylique. Après séchage à 35°C sous vide, en présence de P₂O₅, pendant 12 heures, on récupère 3,26g de solide jaune correspondant au composé attendu.

Les analyses RMN (¹H 400 MHz et ¹³C 100,61 MHz DMSO d₆) et spectrophotométrie de masse sont conformes à la structure attendue.

### Synthèse du dichlorhydrate de 2-(3-Diméthylamino-pyrrolidin-1-yl)-pyrazolo[1,5-a]pyridin-3-ylamine

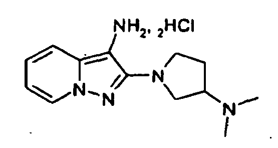

Dans un tricol de 500 ml, surmonté d'un réfrigérant à boules, d'un thermomètre et d'une agitation magnétique, on introduit 400ml éthanol et 5g de poudre de zinc et on porte au reflux.

On ajoute alors 1g de chlorure d'ammonium solubilisé dans 0.5ml d'eau et 1g de N,N-diméthyl-1-(3-nitropyrazolo[1,5-a]pyridin-2-yl)pyrrolidin-3-amine sont ajoutés par portion en 15 minutes et le reflux est maintenu 4 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi, le zinc est lavé avec de l'éthanol et les filtrats réunis sont évaporés à sec.

Après séchage sous vide, en présence de P₂O₅ et de pastilles de soude , on obtient 0.9 g d'une poudre bleutée correspondant au composé attendu.

Les analyses RMN (¹H 400 MHz et ¹³C 100,61 MHz DMSO d₆) sont conformes à la structure attendue. Les ions quasi moléculaires [M+H]+, [M+Na]+ de la molécule attendue C₁₃H₁₉N₅ sont principalement détectés

### Exemple 7 : dichlorhydrate de 2-Imidazol-1-yl-pyrazolo[1,5-a]pyridin-3-ylamine

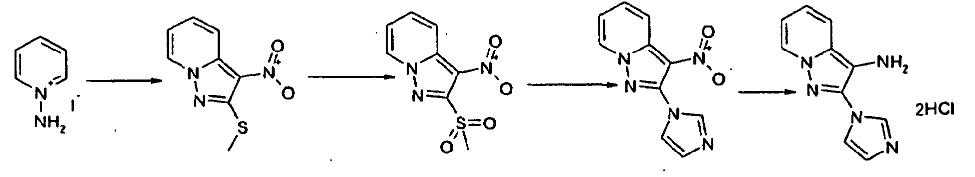

### Synthèse du 2-imidazol-1-yl-3-nitro-pyrazolo[1,5-a]pyridine.

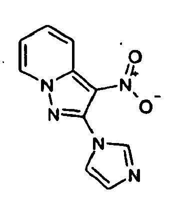

Dans un tricol de 100 ml, équipé d'une agitation magnétique, d'un thermomètre et d'un réfrigérant, sont chargés successivement 5g de 2-méthanesulfanyl-3-nitro-pyrazolo[1,5-a]pyridine, 20ml de N-méthylpyrrolidinone et 7g d'imidazole. Ce milieu est chauffé à 100°C pendant 30 minutes puis après refroidissement, on précipite le 2-imidazol-1-yl-3-nitro-pyrazolo[1,5-a]pyridine dans 5 volumes d'eau. Après essorage et séchage sous vide en présence de penta-oxyde de phosphore, on récupère une masse de 4,23g de solide jaune.

Les analyses RMN (¹H 400 MHz et ¹³C 100,61 MHz DMSO d₆) sont conformes à la structure attendue. La masse du composé attendu C₁₀H₇N₅O₂ est détectée en spectrométrie de masse.

### Synthèse du dichlorhydrate de 2-Imidazol-1-yl-pyrazolo[1,5-a]pyridin-3-ylamine

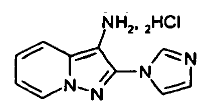

Dans un tricol de 500 ml, surmonté d'un réfrigérant à boules, d'un thermomètre et d'une agitation magnétique, on introduit 200ml éthanol et 6g de poudre de zinc et on porte au reflux.

On ajoute alors 1g de chlorure d'ammonium solubilisé dans 0.5ml d'eau et 2g de 2-imidazol-1-yl-3-nitro-pyrazolo[1,5-a]pyridine sont ajoutés par portion en 15 minutes et le reflux est maintenu 4 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi, le zinc est lavé avec de l'éthanol et les jus réunis sont concentrés au 1/3 et le produit formé est filtré sur fritté.

Après séchage sous vide, en présence de P₂O₅ et de pastilles de soude, on obtient 2.5g d'une poudre rose clair correspondant au composé attendu.

Les analyses RMN (¹H 400 MHz et ¹³C 100,61 MHz DMSO d₆) sont conformes à la structure attendue.

¹H NMR (DMSO-d6): 7.07 (m, 1H), 7.37(dd, 1H), 7.97 (m, 2H), 8.23 (dd, 1H), 8.67 (d, 1H), 9.78(se, 1H)

Les ions quasi moléculaires [M+H]+, [M+Na]+ de la molécule attendue C₁₀H₉N₅ sont principalement détectés

### Exemple 8 : dichlorhydrate de N2-(2-pyrrolidin-1-ylethyl)pyrazolo[1,5-a]pyridine-2,3-diamine

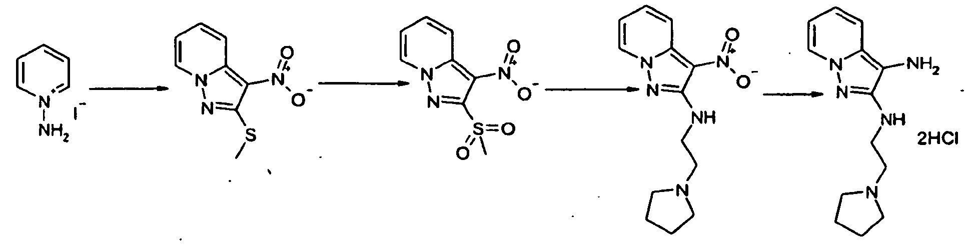

### Synthèse du 3-nitro-N-(2-pyrrolidin-1-yléthyl)pyrazolo[1,5-a]pyridin-2-amine.

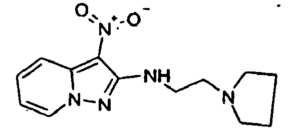

Dans un tricol de 100 ml, surmonté d'un réfrigérant à boules, d'un thermomètre et d'une agitation magnétique, on introduit 20 ml de N-méthypyrrolidinone, 15g (0,062 mole) 2-méthanesulfanyl-3-nitro-pyrazol[1,5-a]pyridine et 16ml de 2,3-aminoéthylpyrrolidine. Sous agitation, on porte à 80°C à l'aide d'un bain d'huile, pendant 1 heure en suivant par CCM (éluant : 90:5 acétate d'éthyle / Méthanol).

Le milieu réactionnel est refroidi à la température ambiante puis versé sur un mélange de 400g de glace et d'eau. Le précipité jaune beige formé est essoré sur fritté puis lavé à l'eau plusieurs fois. Après séchage à 35°C sous vide, en présence de P₂O₅, on récupère 15,2g de solide jaune correspondant au composé attendu.

Les analyses RMN (¹H 400 MHz et ¹³C 100,61 MHz DMSO d₆) et spectrométrie de masse sont conformes à la structure attendue.

### Synthèse du dichlorhydrate de N2-(2-pyrrolidin-1-ylethyl)pyrazolo[1,5-a]pyridine 2,3-diamine

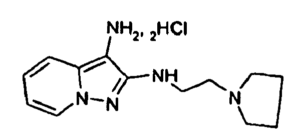

Dans un tricol de 250 ml, surmonté d'un réfrigérant à boules, d'un thermomètre et d'une agitation magnétique, on introduit 200 ml d'éthanol et 5g de poudre de zinc et on porte au reflux.

On ajoute alors au goutte à goutte 2 ml d'acide acétique puis par portion 5g de 3-nitro-N-(2-pyrrolidin-1-yléthyl)pyrazolo[1,5-a]pyridin-2-amine.

En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide gris bleu que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 5g d'une poudre bleutée correspondant au composé attendu.

Les analyses RMN (¹H 400 MHz et ¹³C 100,61 MHz DMSO d₆) sont conformes à la structure attendue.

¹H NMR (DMSO-d6): 1.83 (m, 2H), 1.99(m, 2H), 3.07 (m, 2), 3.38 (m, 2H), 3.63 (m, 4H), 6.71 (m, 1H), 7.24(m, 1H), 7.50(d, 1H), 8.42(d, 1H), 10.13(se, 1H), 10.25(se, 3H).

Les ions quasi moléculaires [M+H]+, [M+Na]+, [M+Na+CH₃OH]+, [2M+H]+, [2M+Na]+ de la molécule attendue C₁₃H₁₉N₅ sont principalement détectés.

### Exemple 9 : dichlorhydrate de 2-(3-Amino-pyrazolo[1,5-a]pyridin-2-yloxy)-éthanol

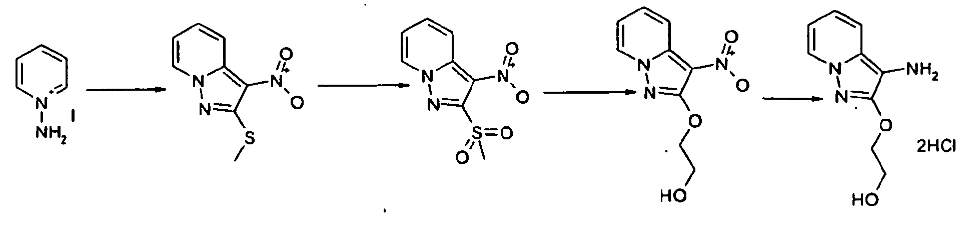

### Synthèse du 2-[(3-nitropyrazolo[1,5-a]pyridin-2-yl)amino]éthanol

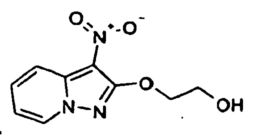

Dans un tricol de 100 ml, surmonté d'un réfrigérant à boules, d'un thermomètre et d'une agitation magnétique, on introduit 20 ml d'éthylène glycol, 4.2g de pastille de soude solubilisées dans 10 ml d'eau et une solution de 2.5 g de -méthanesulfanyl-3-nitro-pyrazol[1,5-a]pyridine et 20 ml de N-méthypyrrolidinone. Sous agitation, on porte à 30°C à l'aide d'un bain d'huile, pendant 1 heure en suivant par CCM (éluant : 90:5 acétate d'éthyle / Méthanol).

Le milieu réactionnel est refroidi à la température ambiante puis versé sur un mélange de 450g de glace et d'eau. Après neutralisation à l'acide chlorhydrique, le précipité jaune beige formé est essoré sur fritté puis lavé à l'eau plusieurs fois. Après séchage à 35°C sous vide, en présence de P₂O₅, on récupère 20.2g de solide jaune correspondant au composé attendu.

Les analyses RMN (¹H 400 MHz et ¹³C 100,61 MHz DMSO d₆) et spectrométrie de masse sont conformes à la structure attendue.

### Synthèse du dichlorhydrate de 2-(3-Amino-pyrazolo[1,5-a]pyridin-2-yloxy)-éthanol

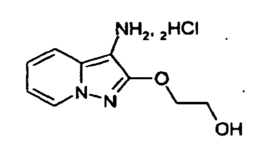

Dans un tricol de 100 ml, surmonté d'un réfrigérant à boules, d'un thermomètre et d'une agitation magnétique, on introduit 50 ml d'éthanol et 5 ml d'acide chlorhydrique et au reflux, on ajoute par portion le mélange 1.2g de zinc et 500mg de méthanesulfanyl-3-nitro-pyrazol[1,5-a]pyridiné.

Le reflux est maintenu 2 heures après la fin de l'addition, puis le zinc est éliminé par filtration sous argon sur un lit de célite et le filtrat est recueilli dans une fiole contenant 50ml d'isopropanol chlorhydrique 6N préalablement refroidi.

Les filtrats sont évaporés à sec et repris trois fois avec une solution d'éther chlorhydrique suivi d'une évaporation pour l'obtention d'un liquide épais bleu.

Ce liquide est versé progressivement sous agitation et amorçage sur de l'éther isopropylique, on observe alors la précipitation d'un solide bleu que l'on essore sur fritté n°3 et que l'on lave avec 2x50 ml d'éther isopropylique. Après séchage sous vide, en présence de P₂O₅ et de pastilles de soude, nous récupérons une poudre bleutée correspondant au composé attendu.

Analyse Masse :
Les ions quasi-moléculaires [M+H]+ , [M+Na]+ et [M-H]- de la molécule attendue, C₉H₁₁N₃O₂, sont principalement détectés

### Exemple 10 : Trichlorhydrate de la 2-Ethoxy-pyrazolo[1,5-a]pyridin-3-ylamine

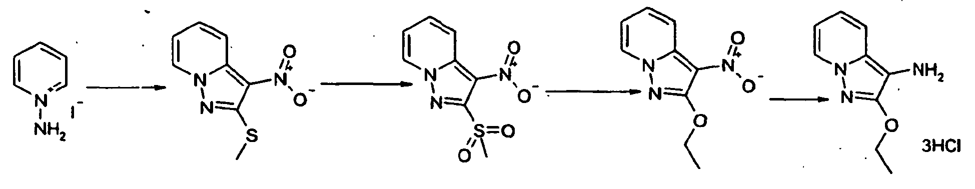

### Synthèse du 2-éthoxy-3-nitropyrazolo[1,5-a]pyridine

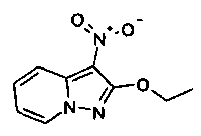

Dans un tricol de 100 ml, surmonté d'un réfrigérant à boules, d'un thermomètre et d'une agitation magnétique, on introduit 50ml d'éthylate de sodium, 4.9g de méthanesulfanyl-3-nitro-pyrazol[1,5-a]pyridine et on porte au reflux sous agitation pendant 0.5 heure.

Le milieu est versé sur 500 ml d'eau, le composé qui a cristallisé est essoré sur fritté, lavé à l'eau puis avec 3x50ml d'éther isopropylique. Après séchage à 35°C sous vide, en présence de P₂O₅, pendant 12 heures, on récupère 4 g de solide beige correspondant au composé attendu.

Les analyses RMN (¹H 400 MHz et ¹³C 100,61 MHz DMSO d₆) et spectrométrie de masse sont conformes à la structure attendue.

### Synthèse du trichlorhydrate de la 2-Ethoxy-pyrazolo[1,5-a]pyridin-3-ylamine

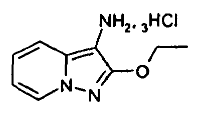

Dans un tricol de 500 ml, surmonté d'un réfrigérant à boules, d'un thermomètre et d'une agitation magnétique, on introduit 350 ml d'éthanol et 12g de poudre de zinc et on porte au reflux.

On ajoute alors 500mg de chlorure d'ammonium puis par portion 3.88g de 2-ethoxy-3-nitropyrazolo[1,5-a]pyridine.

En fin d'addition, on maintient le reflux pendant 2 heures , la réduction est terminée, le zinc est éliminé par filtration sous argon sur un lit de célite et le filtrat recueilli dans une fiole contenant 50ml d'isopropanol chlorhydrique 6N préalablement refroidi.

La précipitation d'un solide est favorisée par ajout d'éther isopropylique au filtrat, le solide crème obtenu est essoré sur fritté lavé avec 2x15ml d'éthanol et 2x50 ml d'éther isopropylique. Après séchage sous vide, en présence de P₂O₅ et de pastilles de soude, nous récupérons une poudre beige crème correspondant au composé attendu.

Les analyses RMN (¹H 400 MHz et ¹³C 100,61 MHz D₂O) sont conformes à la structure attendue.

¹H NMR (D₂O): 1.45 (t, 3H), 4.43(q, 2H), 6.91 (m, 1H), 7.41 (m, 1H), 7.51 (m, 1H), 8.3(m, 1H).

Analyse Masse :
Les ions quasi-moléculaires [M+H]+ , [M+Na]+ et [M-H]- de la molécule attendue, C₉H₁₁N₃O, sont principalement détectés

### Exemple 11 : dichlorhydrate de N-2-(2-pipéridin-1-yléthyl)pyrazolo[1,5-a]pyridine-2,3-diamine

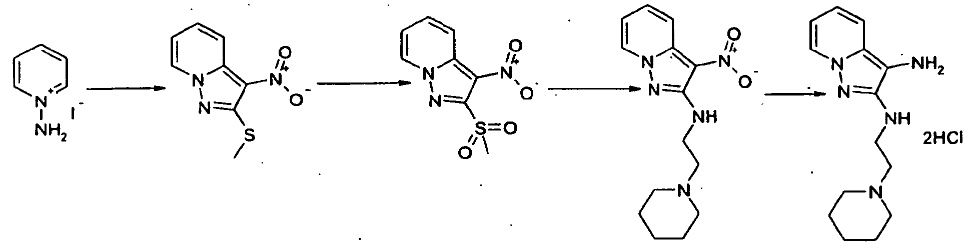

### Synthèse du 3-nitro-N-(2-pipéridin-1-yléthyl)pyrazolo[1,5-a]pyridin-2-amine.

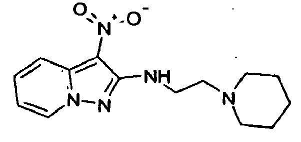

Dans un tricol de 250 ml, surmonté d'un réfrigérant à boules, d'un thermomètre et d'une agitation magnétique, on introduit 100 ml de N-méthypyrrolidinone, 20g (0,0711 mole) 2-méthanesulfanyl-3-nitro-pyrazol[1,5-a]pyridine et 20,3 ml(0,142 mole) de 2-pipéridin-1-yléthanamine. Sous agitation, on porte à 70°C à l'aide d'un bain d'huile, pendant 4 heures en suivant par CCM (éluant : 95:5 Dichlorométhane / Méthanol).

On refroidit le milieu à la température ambiante et le milieu est versé sur un mélange de 400g de glace et d'eau. Le composé beige qui a cristallisé est essoré sur fritté n°4 puis lavé à l'eau plusieurs fois. Après séchage à 35°C sous vide, en présence de P₂O₅, on récupère 20,43g de solide jaune correspondant au composé attendu.

Les analyses RMN (¹H 400 MHz et ¹³C 100,61 MHz DMSO d₆) sont conformes à la structure attendue. La masse du composé attendu C14H19N5O2 est détectée en spectrométrie de masse.

Les ions quasi moléculaires [M+H]+, [M+Na]+, [M+Na+CH₃OH]+ de la molécule attendue C₁₄H₁₉N₅O₂ sont principalement détectés.

### Synthèse du dichlorhydrate de N-2-(2-pipéridin-1-yléthyl)pyrazolo[1,5-a]pyridine-2,3-diamine

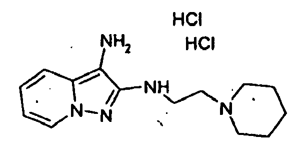

Dans un tricol de 250 ml, surmonté d'un réfrigérant à boules, d'un thermomètre et d'une agitation magnétique, on introduit 150 ml d'éthanol et 2g de poudre de zinc et on porte au reflux.

On ajoute alors au goutte à goutte 2 ml d'acide acétique puis par portion 2g de 3-nitro-N-(2-pipéridin-1-yléthyl)pyrazolo[1,5-a]pyridin-2-amine

En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide gris bleu que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 2g d'une poudre gris bleu correspondant au composé attendu.

Les analyses RMN (¹H 400 MHz et ¹³C 100,61 MHz DMSO d₆) sont conformes à la structure attendue.

### Exemple 12 : dichlorhydrate de N-2-[2-(diisopropylamino)éthyl]pyrazolo[1,5-a]pyridine-2,3-diamine

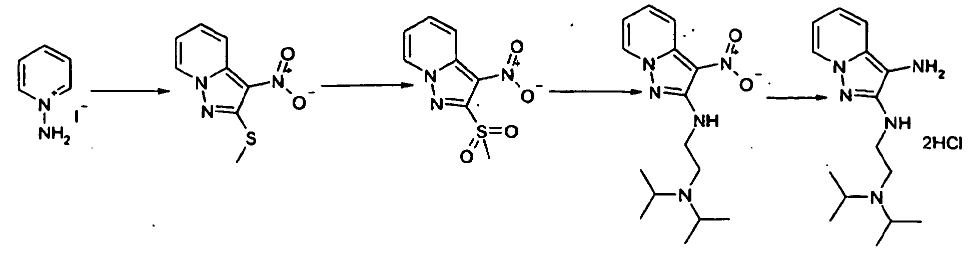

### Synthèse du N,N-diisopropyl-N'-(3-nitropyrazolo[1,5-a]pyridin-2-yl)éthane-1,2-diamine.

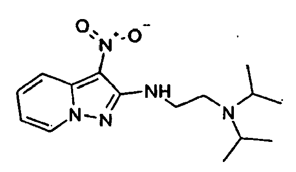

Dans un tricol de 100 ml, surmonté d'un réfrigérant à boules, d'un thermomètre et d'une agitation magnétique, on introduit 20ml de N-méthypyrrolidinone, 10g (0,0414 mole) 2-méthanesulfanyl-3-nitro-pyrazol[1,5-a]pyridine et 14,4ml (0,082mole de N,N-diisopropyléthane-1,2-diamine. Sous agitation, on porte à 70°C à l'aide d'un bain d'huile, pendant 1 heure en suivant par CCM (éluant : 95:5 Dichlorométhane/Méthanol).

Le milieu réactionnel est refroidi à la température ambiante versé sur un mélange de 100g de glace et d'eau. Le précipité jaune formé est essoré sur fritté n°3 puis lavé à l'eau puis avec 3x100ml d'éther de pétrole. Après séchage à 35°C sous vide, en présence de P₂O₅, on récupère 11,88g de solide jaune correspondant au composé attendu.

Les analyses RMN (¹H 400 MHz et ¹³C 100,61 MHz DMSO d₆) sont conformes à la structure attendue. Les ions quasi moléculaires [M+H]+, [M+Na]+, [M+Na+CH₃OH]+ de la molécule attendue C₁₅H₂₃N₅O₂ sont principalement détectés.

### Synthèse du dichlorhydrate de N-2-[2-(diisopropylamino)éthyl]pyrazolo[1,5-a]pyridine-2,3-diamine

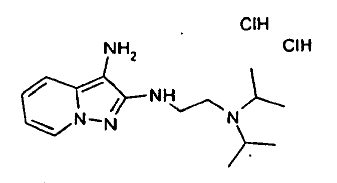

Dans un tricol de 250 ml, surmonté d'un réfrigérant à boules, d'un thermomètre et d'une agitation magnétique, on introduit 150 ml d'éthanol et 2g de poudre de zinc et on porte au reflux.

On ajoute alors au goutte à goutte 1.75 ml d'acide acétique puis par portion de 1.75g de N,N-diisopropyl-N'-(3-nitropyrazolo[1,5-a]pyridin-2-yl)éthane-1,2-diamine En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide gris bleu que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons une poudre gris bleu correspondant au composé attendu.

Les analyses RMN (¹H 400 MHz et ¹³C 100,61 MHz DMSO d₆) sont conformes à la structure attendue.

La masse du composé attendu C₁₅H₂₅N₅ est détectée en spectrométrie de masse. Les ions quasi moléculaires [M+H]+, [M+Na]+, [M+Na+CH₃OH]+ de la molécule C₁₅H₂₅N₅ attendue sont principalement détectés.

### Exemple 13: dichlorhydrate de N2-[2-(diéthylamino)éthyl]pyrazolo[1,5-a]pyridine-2,3-diamine

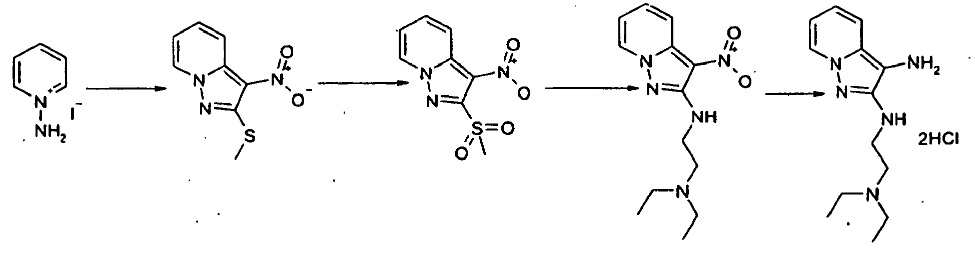

### Synthèse du N,N-diéthyl-N'-(3-nitropyrazolo[1,5-a]pyridin-2-yl)éthane-1,2-diamine.

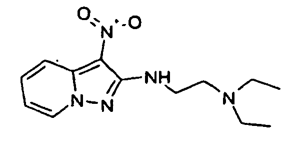

Dans un tricol de 100 ml, surmonté d'un réfrigérant à boules, d'un thermomètre et d'une agitation magnétique, on introduit 100 ml de N-méthypyrrolidinone, 10g (0,0414 mole) 2-méthanesulfanyl-3-nitro-pyrazol[1,5-a]pyridine et 11,65 ml (0,0829mol) de N,N-diéthylènediamine. Sous agitation, on porte à 70°C à l'aide d'un bain d'huile, pendant 4 heures.

On refroidit le milieu à la température ambiante et le milieu est versé sur un mélange de 100g de glace et d'eau. Le composé jaune qui a cristallisé est essoré sur fritté n°3 puis lavé 3×100ml d'éther de pétrole. Après séchage à 35°C sous vide, en présence de P₂O₅, on récupère 9,80g de solide jaune correspondant au composé attendu.

Les analyses RMN (¹H 400 MHz et ¹³C 100,61 MHz DMSO d₆) sont conformes à la structure attendue. Les ions quasi moléculaires [M+H]+, [M+Na]+, [M+Na+CH3OH]+ de la molécule attendue C₁₃H₁₉N₅O₂ sont principalement détectés.

### Synthèse du dichlorhydrate de N2-[2-(diéthylamino)éthyl]pyrazolo[1,5-a]pyridine-2,3-diamine

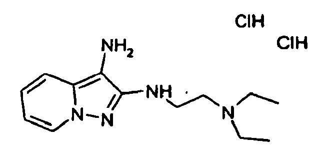

Dans un tricol de 250 ml, surmonté d'un réfrigérant à boules, d'un thermomètre et d'une agitation magnétique, on introduit 150 ml d'éthanol et 2g de poudre de zinc et on porte au reflux.

On ajoute alors au goutte à goutte 2 ml d'acide acétique puis par portion 2g de N,N-diisopropyl-N'-(3-nitropyrazolo[1,5-a]pyridin-2-yl)éthane-1,2-diamine.

En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide gris bleu que l'on essore sur fritté et que l'on lave avec 2x15ml d'éthanol et 2x50 ml d'éther isopropylique. Après séchage sous vide, en présence de P₂O₅ et de pastilles de soude, nous récupérons 2g d'une poudre gris vert correspondant au composé attendu.

Les analyses RMN (¹H 400 MHz et ¹³C 100,61 MHz DMSO d₆) sont conformes à la structure attendue.

La masse du composé attendu C13H21 N5 est détectée en spectrométrie de masse. Les ions quasi moléculaires [M+H]+, [M+Na]+, [M+Na+CH₃OH]+ de la molécule C₁₃H₂₁N₅ attendue sont principalement détectés.

### Exemple 14: dichlorhydrate de N2-(2-pyridin-3-yléthyl)pyrazolo[1,5-a]pyridine-2,3-diamine

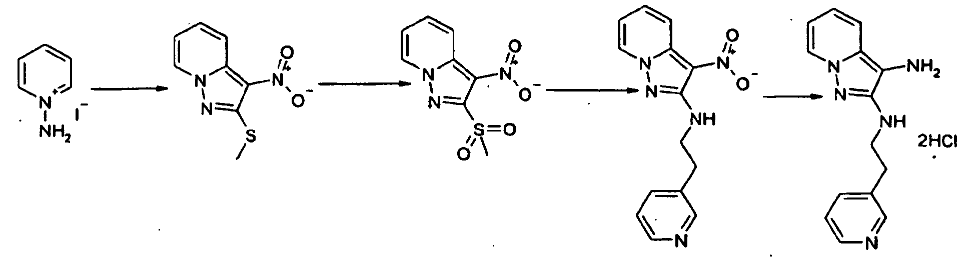

### Synthèse du 3-nitro-N-(2-pyridin-3-yléthyl)pyrazolo[1,5-a]pyridin-2-amine

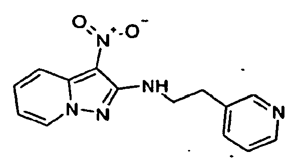

Dans un tricol de 100 ml, surmonté d'un réfrigérant à boules, d'un thermomètre et d'une agitation magnétique, on introduit 10 ml de N-méthypyrrolidinone, 4,93g (0,02046 mole) 2-méthanesulfanyl-3-nitro-pyrazol[1,5-a]pyridine et 5g ml (0,04092 mole) de 3-(2-aminoéthyl)-pyridine. Sous agitation, on porte à 70°C à l'aide d'un bain d'huile, pendant 1 heure.

Le milieu réactionnel est refroidi jusqu'à la température ambiante puis versé sur un mélange de 100g de glace et d'eau. Le composé jaune qui a cristallisé est essoré sur fritté, lavé à l'eau puis avec 3x100ml d'éther de pétrole. Après séchage à 35°C sous vide, en présence de P₂O₅, on récupère 5,61g de solide jaune correspondant au composé attendu.

Les analyses RMN (¹H 400 MHz et ¹³C 100,61 MHz DMSO d₆) sont conformes à la structure attendue.

Les ions quasi moléculaires [M+H]+, [M+Na]+, [M+Na+CH₃OH]+, [2M+H]+, [2M+Na]+ de la molécule attendue C₁₄H₁₃N₅O₂ sont principalement détectés.

### Synthèse du dichlorhydrate de N2-(2-pyridin-3-yléthyl)pyrazolo[1,5-a]pyridine-2,3-diamine

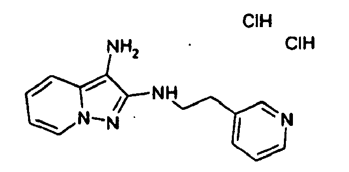

Dans un tricol de 250 ml, surmonté d'un réfrigérant à boules, d'un thermomètre et d'une agitation magnétique, on introduit 150 ml d'éthanol et 2g de poudre de zinc et on porte au reflux.

On ajoute alors au goutte à goutte 2 ml d'acide acétique puis par portion 1.6g de 3-nitro-N-(2-pyridin-3-yléthyl)pyrazolo[1,5-a]pyridin-2-amine.

En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide gris bleu que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 1.46g d'une poudre gris vert correspondant au composé attendu.

Les analyses RMN (¹H 400 MHz et ¹³C 100,61 MHz DMSO d₆) sont conformes à la structure attendue.

Les ions quasi moléculaires [M+H]+, [M+Na]+, [M+Na+CH₃OH]+, [2M+H]+, [2M+Na]+ de la molécule attendue C₁₄H₁₅N₅ sont principalement détectés.

### Exemple 15 : dichlorhydrate de N-2-(2-pyridin-4-yléthyl)pyrazolo[1,5-a]pyridine-2,3-diamine

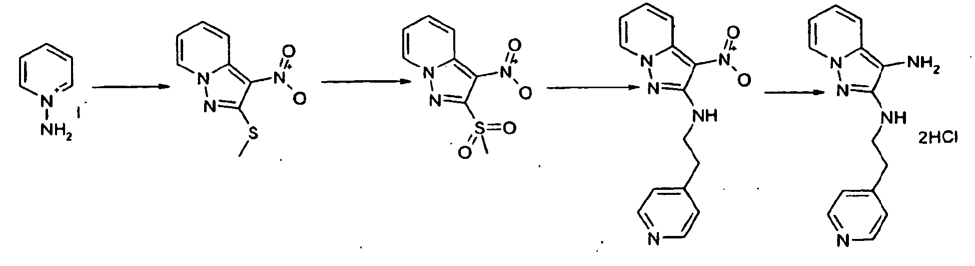

### Synthèse du 3-nitro-N-(2-pyridin-4-yléthyl)pyrazolo[1,5-a]pyridin-2-amine

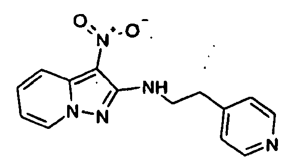

Dans un tricol de 100 ml, surmonté d'un réfrigérant à boules, d'un thermomètre et d'une agitation magnétique, on introduit 50 ml de N-méthypyrrolidinone, 10g (0,03555 mole) 2-méthanesulfanyl-3-nitro-pyrazol[1,5-a]pyridine et 8,68g (0,07110mole) de 4-(2-aminoéthyl). Sous agitation, on porte à 70°C à l'aide d'un bain d'huile, pendant 4 heures en suivant par CCM (éluant : 95:5 Dichlorométhane / Méthanol).

On refroidit le milieu à la température ambiante et le milieu est versé sur un mélange de 200g de glace et d'eau. Le composé jaune qui a cristallisé est essoré sur fritté n°3, lavé à l'eau puis avec 3x100ml d'éther de pétrole. Après séchage à 35°C sous vide, en présence de P₂O₅, pendant 12 heures, on récupère 10,81g (produit non totalement sec) de solide jaune correspondant au composé attendu.

Les analyses RMN (¹H 400 MHz et ¹³C 100,61 MHz DMSO d₆) sont conformes à la structure attendue.

### Synthèse du dichlorhydrate de N-2-(2-pyridin-4-yléthyl)pyrazolo[1,5-a]pyridine-2,3-diamine

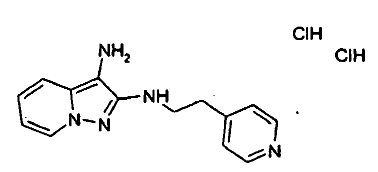

Dans un tricol de 250 ml, surmonté d'un réfrigérant à boules, d'un thermomètre et d'une agitation magnétique, on introduit 150 ml d'éthanol et 2g de poudre de zinc et on porte au reflux.

On ajoute alors au goutte à goutte 2 ml d'acide acétique puis par portion 2g de 3-nitro-N-(2-pyridin-4-yléthyl)pyrazolo[1,5-a]pyridin-2-amine.

En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide gris bleu que l'on essore sur fritté et que l'on lave avec 2x15ml d'éthanol et 2x50 ml d'éther isopropylique. Après séchage sous vide, en présence de P₂O₅ et de pastilles de soude, nous récupérons 1.8g d'une poudre gris vert correspondant au composé attendu.

Les analyses RMN (¹H 400 MHz et ¹³C 100,61 MHz DMSO d₆) sont conformes à la structure attendue.

¹H NMR (DMSO-d6): 3.21 (t, 2H), 3.64(t, 2H), 6.66 (m, 1), 7.19 (m, 1H), 7.50 (m, 1H), 8.04 (d, 2H), 8.39(m, 1H), 8.82(d, 2H).

Les ions quasi moléculaires [M+H]+, [M+Na]+, [M+Na+CH₃OH]+, [2M+H]+, [2M+Na]+ de la molécule attendue C₁₄H₁₅N₅ sont principalement détectés.

### Exemple 16 : dichlorhydrate de N2-[2-(4-méthylpipérazin-1-yl)éthyl]pyrazolo[1,5-a]pyridine-2,3-diamine

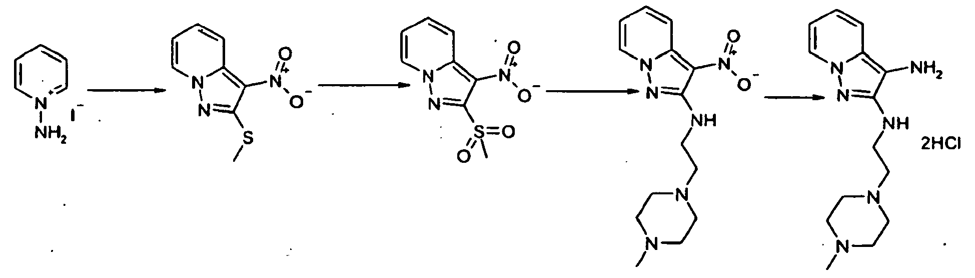

### Synthèse du N-[2-(4-methylpiperazin-1-yl)ethyl]-3-nitropyrazolo[1,5-a]pyridin-2-amine.

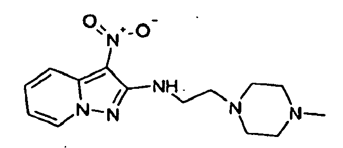

Dans un tricol de 50 ml, surmonté d'un réfrigérant à boules, d'un thermomètre et d'une agitation magnétique, on introduit 10 ml de N-méthypyrrolidinone, 7.23g (0,03 mole) 2-méthanesulfanyl-3-nitro-pyrazol[1,5-a]pyridine et 10 ml (0,1 mole) de N-méthylpiperidine. Sous agitation, on porte à 70°C à l'aide d'un bain d'huile, pendant 4 heures.

On refroidit le milieu à la température ambiante et le milieu est versé sur un mélange de 400g de glace et d'eau. Le composé beige qui a cristallisé est essoré sur fritté puis lavé à l'eau plusieurs fois. Après séchage à 35°C sous vide, en présence de P₂O₅, on récupère 7.99g de solide jaune correspondant au composé attendu.

Les analyses RMN (¹H 400 MHz et ¹³C 100,61 MHz DMSO d₆) sont conformes à la structure attendue.

La masse du composé attendu C₁₂H₁₅N5₆O₂ est détectée en spectrométrie de masse. Les ions quasi moléculaires [M+H]+, [M+Na]+, [M+Na+CH₃OH]+ de la molécule attendue C₁₂H₁₅N₅O₂ sont principalement détectés.

### Synthèse du dichlorhydrate de N2-[2-(4-méthylpipérazin-1-yl)éthyl]pyrazolo[1,5-a]pyridine-2,3-diamine

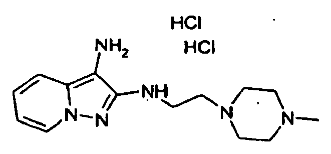

Dans un tricol de 500 ml, surmonté d'un réfrigérant à boules, d'un thermomètre et d'une agitation magnétique, on introduit 200ml éthanol et 6g de poudre de zinc et on porte au reflux.

On ajoute alors 1g de chlorure d'ammonium solubilisé dans 0.5ml d'eau et 2g de N-[2-(4-méthylpipérazin-1-yl)éthyl]-3-nitropyrazolo[1,5-a]pyridin-2-amine sont ajoutés par portion en 15 minutes et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi, lavé avec de l'éthanol et filtrats réunis sont dilués avec de l'éther isopropylique et le solide formé est essoré sur fritté.

Après séchage sous vide, en présence de P₂O₅ et de pastilles de soude, on obtient 0.3 g d'une poudre bleu vert clair correspondant au composé attendu.

Les analyses RMN (¹H 400 MHz et ¹³C 100,61 MHz DMSO d₆) sont conformes à la structure attendue.

### Exemple 17: chlorhydrate de 2-Methoxy-7-methyl-pyrazolo[1,5-a]pyridin-3-ylamine

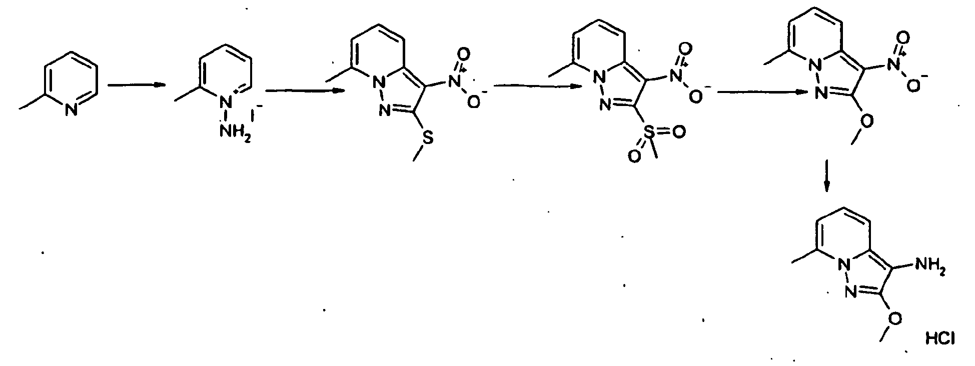

### Synthèse du 1-amino-2-methylpyridinium iodide

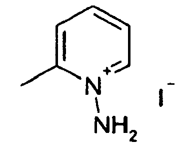

Dans un tricol de 2 litres, surmonté d'un réfrigérant à boules, d'un thermomètre et d'une agitation mécanique et d'une ampoule de coulée on introduit 400ml d'eau, 100g de picoline et à 30°C la solution de 40.1g de d'acide hydroxylamino sulfonique dans 600 ml d'eau est ajoutée rapidement et le milieu est porté à 85°C 6 heures puis l'agitation est maintenue à température ambiante pendant la nuit.

Apres avoir ajouter 69.3 g de carbonate de potassium, on extrait avec 4x500ml d'acétate d'éthyle et la phase aqueuse est évaporée, le solide formé est repris avec 1.5 litre d'éthanol.

Apres élimination des sels, les jus sont acidifiés à -60°C avec 47.3 ml d'acide iodhydrique

L'agitation est maintenue pendant une heure est le solide est précipité avec 400ml d'éther isopropylique.

Le solide formé est repris dans de l'éther de pétrole essoré sur fritté puis lavé avec de l'éther de pétrole.

Après séchage sous vide, en présence de P₂O₅, nous récupérons 37.6g de solide marron correspondant au composé attendu.

Les analyses RMN (¹H 400 MHz et ¹³C 100,61 MHz DMSO d₆) et spectrophotométrie de masse sont conformes à la structure attendue.

### Synthèse du 7-methyl-2-(methylsulfanyl)-3-nitropyrazolo[1,5-a]pyridine

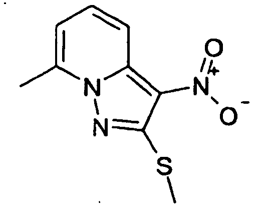

Dans un tricol de 500ml, surmonté d'un réfrigérant à boules, d'un thermomètre et d'une agitation magnétique et d'une ampoule de coulée on introduit 200ml de DMF, 400ml d'éthanol, 34g d'iodure de 1 1-amino-2-methylpyridinium, 32 ml de triéthylamine sous agitation en 30 minutes.

La solution de 25.9g de 1,1-bis(methylthio)-2-nitro éthylène dans 200ml de DMF est ajoutée et le milieu est porté au reflux pendant 24 heures

Après refroidissement à température ambiante, le milieu réactionnel est versé sur 4 litres de mélange glace- eau.

L'insoluble marron formé est essoré sur fritté, lavé à l'eau et à l'acétone et après séchage sous vide, en présence de P₂O₅, nous récupérons 15.9g de solide marron ocre correspondant au composé attendu.

Les analyses RMN (¹H 400 MHz et ¹³C 100,61 MHz DMSO d₆) et spectrométrie de masse sont conformes à la structure attendue.

### Synthèse du 7-methyl-2-(methylsulfonyl)-3-nitropyrazolo[1,5-a]pyridine

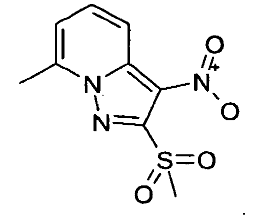

Dans un tricol de 1 L, surmonté d'un réfrigérant à boules, d'un thermomètre et d'une agitation magnétique et d'une ampoule de coulée on introduit 400ml de dichlorométhane, 3.2g de 7-méthyl-2-(méthylsulfanyl)-3-nitropyrazolo[1,5-a]pyridine puis à 10°C, une solution de 13.2g d'acide méta chloroperbenzoïque dans 250ml de dichlorométhane est ajoutée au goutte à goutte en 2 heures.

L'agitation est maintenue pendant plusieurs heures et le milieu réactionnel récupéré est lavé avec 2x 500ml de solution saturée d'hydrogénocarbonate de sodium puis 500ml d'eau.

La phase organique est lavée plusieurs fois à l'eau et après séchage sur sulfate de sodium la phase organique est évaporée a sec pour conduire après séchage à 35°C sous vide, en présence de P₂O₅, pendant 12 heures, à l'obtention de 2.33g de solide jaune terne correspondant au composé attendu.

Les analyses RMN (¹H 400 MHz et ¹³C 100,61 MHz DMSO d₆) et spectrométrie de masse sont conformes à la structure attendue.

### Synthèse du 2-methyl-7-methyl-3-nitropyrazolo[1,5-a]pyridine

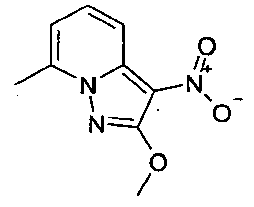

Dans un tricol de 100 ml, surmonté d'un réfrigérant à boules, d'un thermomètre et d'une agitation magnétique, on introduit 15ml de méthylate de sodium à 30% et 2g de 7-methyl-2-(méthylsulfonyl)-3-nitropyrazolo[1,5-a]pyridine.

L'agitation est maintenue 2 heures et le milieu réactionnel est versé sur 500 ml d'eau, le pH est ajusté à 7 par ajout d'acide chlorhydrique, le composé qui a cristallisé est essoré sur fritté, lavé à l'eau puis avec 3x50ml d'éther isopropylique. Après séchage à 35°C sous vide, en présence de P₂O₅, pendant 12 heures, on récupère 1.62 g de solide beige correspondant au composé attendu.

Les analyses RMN (¹H 400 MHz et ¹³C 100,61 MHz DMSO d₆) et spectrométrie de masse sont conformes à la structure attendue.

### Synthèse du chlorhydrate de 2-Methoxy-7-methyl-pyrazolo[1,5-a]pyridin-3-ylamine

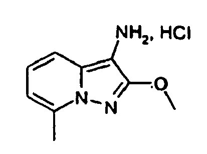

Dans un tricol de 100 ml, surmonté d'un réfrigérant à boules, d'un thermomètre et d'une agitation magnétique, on introduit 40 ml d'éthanol et 4g de poudre de zinc et on porte au reflux.

On ajoute goutte à goutte 10 ml d'acide chlrohydrique à 35% puis par portion 1.8g de 2-methoxy-7-methyl-3-nitropyrazolo[1,5-a]pyridine.

En fin de d'addition, on ajoute quelquesgouttes d'ethanol chlorhydrique et le reflux est maintenu 1 heure.

Lorsque la réduction est terminée, le zinc est éliminé par filtration sous argon sur un lit de célite et le filtrat est recueilli dans une fiole contenant 20ml d'éthanol chlorhydrique 6N préalablement refroidi et après concentration au 1/3, le résidu est additionné d'acétate d'éthyle suivi d'une évaporation au maximum.

Le résidu est repris avec de l'éthanol chlorhydrique et le solide formé est essoré sur fritté puis lavé avec 2x50 ml d'éther isopropylique. Après séchage sous vide, en présence de P₂O₅ et de pastilles de soude, nous récupérons 1.2g d'une poudre correspondant au composé attendu.

Les analyses RMN (¹H 400 MHz et ¹³C 100,61 MHz D₂O) sont conformes à la structure attendue.

Analyse Masse :
Les ions quasi-moléculaires [M+H]+ , [M+Na]+ et [M-H]- de la molécule attendue, C₉H₁₁N₃O, sont principalement détectés

### Exemple 18 : dichlorhydrate de 4-éthyl-2-méthoxy-7-méthyl-pyrazolo[1,5-a]pyridin-3-ylamine

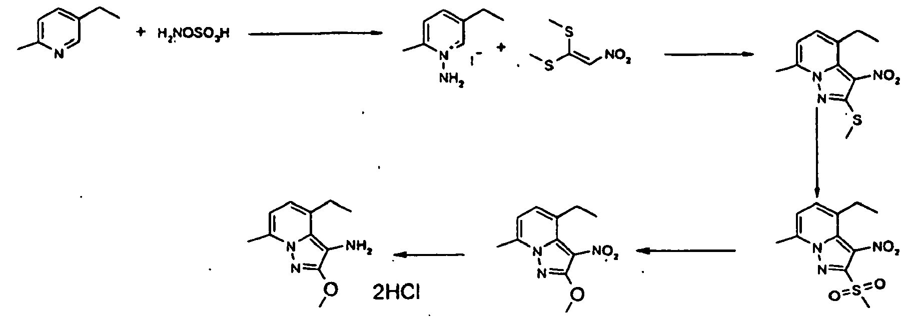

### Synthèse du 1-amino-5-éthyl-2-méthylpyridinium iodure

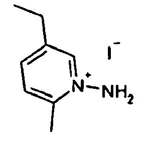

Dans un tricol de 500ml, surmonté d'un réfrigérant à boules, d'un thermomètre et d'une agitation magnétique et d'une ampoule de coulée on introduit 100ml d'eau, 50 ml de 5-ethyl-2-methylpyridine et à 30°C la solution de 20g d'acide hydroxylamino sulfonique dans 250 ml d'eau est ajoutée rapidement et le milieu est porté à 85°C pendant 6 heures puis l'agitation est maintenue à température ambiante pendant la nuit.

On extrait avec 5x500 ml l'acétate d'éthyle et la phase aqueuse est évaporée à sec pour conduire à un solide qui est lavé par 500ml d'éthanol

Les jus éthanoliques sont chargés dans un tricol de 1 litre, refroidis à -40°C et 29 ml d'acide iodhydrique sont ajoutés au goutte à goutte rapidement sous bonne agitation.

L'agitation est maintenue pendant une heure est le solide obtenu est précipité avec 3 litres d'éther isopropylique.

Le solide rose-saumon formé est essoré sur fritté puis lavé avec de l'éther isopropylique.

Après séchage sous vide, en présence de P₂O₅, nous récupérons 15.2g de solide marron ocre correspondant au composé attendu.

Les analyses RMN (¹H 400 MHz et ¹³C 100,61 MHz DMSO d₆) et spectrophotométrie de masse sont conformes à la structure attendue.

### Synthèse du 4-ethyl-7-methyl-2-(methylsulfanyl)-3-nitropyrazolo[1,5-a]pyridine

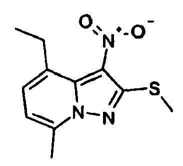

Dans un tricol de 500ml, surmonté d'un réfrigérant à boules, d'un thermomètre et d'une agitation magnétique et d'une ampoule de coulée on introduit 160ml de DMF, 24.8g d'iodure de 1-amino-5-ethyl-2-methylpyridinium, 39g de carbonate de potassium et 15.5 g de 1,1-bis(methylthio)-2-nitro éthylène.

L'agitation est maintenue pendant 96 heures à la température ambiante est le milieu réactionnel est versé sur 2,5 litres de mélange glace - eau.

L'insoluble marron ocre formé est essoré sur fritté n°3 puis lavé abondamment à l'eau, par 3x 50ml d'isopropanol puis avec 3x500 ml d' éther isopropylique.

Après séchage sous vide, en présence de P₂O₅, nous récupérons 10.59g de solide marron ocre correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires [M+H]+, [M+Na]+, [M+Na+CH3OH]+, [2M+Na]+ sont principalement détectés

### Synthèse du 4-éthyl-7-methyl-2-(méthylsulfonyl)-3-nitropyrazolo[1,5-a]pyridine

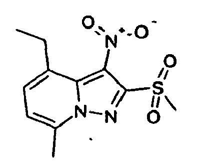

Dans un tricol de 1 L, surmonté d'un réfrigérant à boules, d'un thermomètre et d'une agitation magnétique et d'une ampoule de coulée on introduit 100ml de dichlorométhane, 10g de 4-éthyl-7-méthyl-2-(méthylsulfanyl)-3-nitropyrazolo[1,5-a]pyridine puis à 10°C, une solution de 39.2g d'acide méta chloroperbenzoïque dans 600ml de dichlorométhane est ajoutée au goutte à goutte en 2 heures.

L'agitation est maintenue pendant une heure supplémentaire et le milieu réactionnel est lavé avec 4x 500ml de solution saturée d'hydrogénocarbonate de sodium puis 500ml d'eau.

Après séchage, la phase organique est évaporée a sec, le composé jaune formé est repris avec de d'éther isopropylique.

Le solide est essoré sur fritté, lavé à l'eau puis avec 3x50ml d'éther isopropylique. Après séchage à 35°C sous vide, en présence de P₂O₅, pendant 12 heures, on récupère un solide jaune correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires [M+H]+, [M+Na]+, [M+Na+CH₃OH]+, [2M+Na]+ sont principalement détectés

### Synthèse du 4-éthyl-2-méthoxy-7-méthyl-3-nitropyrazolo[1,5-a]pyridine

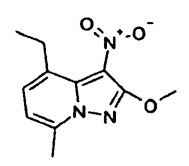

Dans un tricol de 100 ml, surmonté d'un réfrigérant à boules, d'un thermomètre et d'une agitation magnétique, on introduit 30ml de méthylate de sodium à 30% 4g de 4-éthyl-7-methyl-2-(méthylsulfonyl)-3-nitropyrazolo[1,5-a]pyridine dans 30 ml de méthanol et on agite pendant 2 heures.

Le milieu réactionnel est versé sur 500 ml d'eau, le composé qui a cristallisé est essoré sur fritté, lavé à l'eau puis avec 3x50ml d'éther isopropylique. Après séchage à 35°C sous vide, en présence de P₂O₅, pendant 12 heures, on récupère1.2 g de solide beige correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires [M+H]+, [M+Na]+, [M+Na+CH₃OH]+, [2M+Na]+ sont principalement détectés

### Synthèse du dichlorhydrate de 4-éthyl-2-méthoxy-7-méthyl-pyrazolo[1,5-a]pyridin-3-ylamine

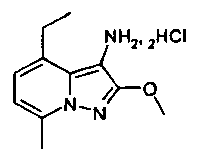

Dans un tricol de 100ml, surmonté d'un réfrigérant à boules, d'un thermomètre et d'une agitation magnétique, on introduit 50 ml d'éthanol et 4.5g de poudre de zinc et on porte au reflux.

On ajoute alors 175 mg de chlorure d'ammonium puis par portion 620mg de 4-éthyl-2-méthoxy-7-méthyl-3-nitropyrazolo[1,5-a]pyridine.

En fin d'addition, on ajoute au goutte à goutte 10 ml d'éthanol chlorhydrique au goutte à goutte et le reflux est maintenu 1 heure.

Lorsque la réduction est terminée, le zinc est éliminé par filtration sous argon sur un lit de célite et le filtrat est recueilli dans une fiole contenant 20ml d'éthanol chlorhydrique 6N préalablement refroidi et après concentration au 1/3, ces jus sont dilués avec 400ml d'éther isopropylique.

On observe alors la précipitation d'un solide qui gomme et que l'on reprend avec de éther isopropylique. Une fois cristallisé, le solide est essoré sur fritté puis lavé avec 2x50 ml d'éther isopropylique. Après séchage sous vide, en présence de P₂O₅ et de pastilles de soude, nous récupérons 1.5g d'une poudre correspondant au composé attendu.

Les analyses RMN (¹H 400 MHz et ¹³C 100,61 MHz DMSO d₆) sont conformes à la structure attendue.

Les ions quasi-moléculaires [M+H]+, [M+Na]+ et [M-H]- de la molécule attendue, C₁₁H₁₅N₃O, sont principalement détectés

### Exemple 19 : dichlorhydrate de 4-éthyl-7-méthyl-2-pyrrolidin-1-yl-pyrazolo[1,5-a]pyridin-3-ylamine

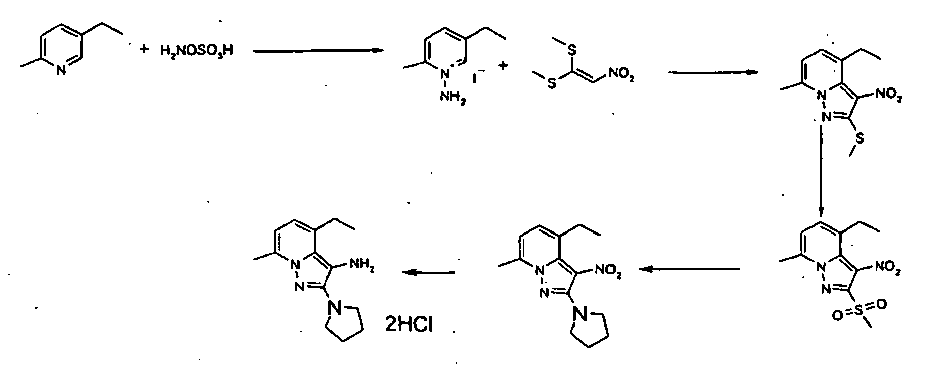

### Synthèse du 4-éthyl-7-méthyl-3-nitro-2-pyrrolidin-1-ylpyrazolo[1,5-a]pyridine

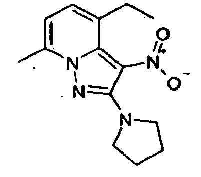

Dans un tricol de 50 ml, surmonté d'un réfrigérant à boules, d'un thermomètre et d'une agitation magnétique, on introduit, 25ml de pyrrolidine, et 1.5g de 4-éthyl-7-méthyl-2-(méthylsulfanyl)-3-nitropyrazolo[1,5-a]pyridine ; ce milieu est agité pendant une heure.

Le composé jaune, isolé en versant le milieu réactionnel sur de l'eau glacée, est essoré sur fritté puis lavé à l'eau plusieurs fois et l'éther de pétrole. Après séchage sous vide, en présence de P₂O₅, on récupère 0.651 g de solide jaune correspondant au composé attendu.

Les analyses RMN (¹H 400 MHz et ¹³C 100,61 MHz DMSO d₆) sont conformes à la structure attendue.

### Synthèse du dichlorhydrate de 4-éthyl-7-méthyl-2-pyrrolidin-1-yl-pyrazolo[1,5-a]pyridin-3-ylamine

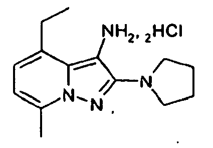

Dans un tricol de 50 ml, surmonté d'un réfrigérant à boules, d'un thermomètre et d'une agitation magnétique, on introduit 30 ml d'éthanol et 1,6g de poudre de zinc et on porte au reflux.

On ajoute alors 60 mg de chlorure d'ammonium puis par portion, 620mg de 4-éthyl-7-méthyl-3-nitro-2-pyrrolidin-1-ylpyrazolo[1,5-a]pyridine.

En fin d'addition, on ajoute au goutte à goutte 5 ml d'éthanol chlorhydrique et le reflux est maintenu 2 heures.

Lorsque la réduction est terminée, le zinc est éliminé par filtration sous argon sur un lit de célite et le filtrat est recueilli dans une fiole contenant 20ml d'éthanol chlorhydrique 6N préalablement refroidi et ce filtrat est additionné d' éther isopropylique

On observe alors la précipitation d'un solide jaunâtre que l'on essore sur fritté et que l'on lave avec 2x50 ml d'éther isopropylique. Après séchage sous vide, en présence de P₂O₅ et de pastilles de soude, nous récupérons 623mg d'une poudre gris beige correspondant au composé attendu.

Les analyses RMN (¹H 400 MHz et ¹³C 100,61 MHz D₂O) sont conformes à la structure attendue.

Les ions quasi-moléculaires [M+H]+, [M+Na]+ et [M-H]- de la molécule attendue, C₁₄H₂₀N₄, sont principalement détectés

### Exemple 20 : chlorhydrate de 2-Méthylsulfanyl-pyrazolo[1,5-a]pyridin-3-ylamine

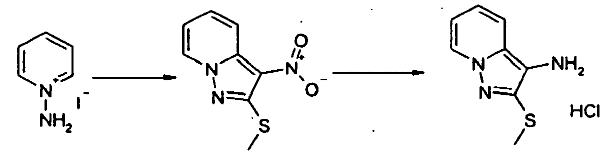

### Synthèse du chlorhydrate de 2-Méthylsulfanyl-pyrazolo[1,5-a]pyridin-3-ylamine

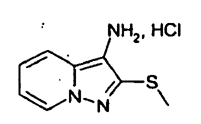

Dans un tricol de 500 ml, surmonté d'un réfrigérant à boules, d'un thermomètre et d'une agitation magnétique, on introduit 200 ml d'éthanol et 15g de poudre de zinc et on porte au reflux.

On ajoute alors au goutte à goutte 5 ml d'acide chlorhydrique puis par portion 5g de 2-(methylsulfanyl)-3-nitropyrazolo[1,5-a]pyridine en 45 minutes.

En fin d'addition, on ajoute au goutte à goutte 5 ml d'acide chlorhydrique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide beige que l'on essore sur fritté et que l'on lave avec 2x50 ml d'éther isopropylique. Après séchage sous vide, en présence de P₂O₅ et de pastilles de soude, nous récupérons 4.9g d'une poudre gris beige correspondant au composé attendu.

Les analyses RMN (¹H 400 MHz et ¹³C 100,61 MHz DMSO d₆) sont conformes à la structure attendue.

Les ions quasi-moléculaites [M+H]+, [M+Na]+ et [M-H]- de la molécule attendue, C₈H₉N₃S, sont principalement détectés

### Exemple 21 : dichlorhydrate de 6,7-Diméthyl-2-méthylsulfanyl-pyrazolo[1,5-a]pyridin-3-ylamine

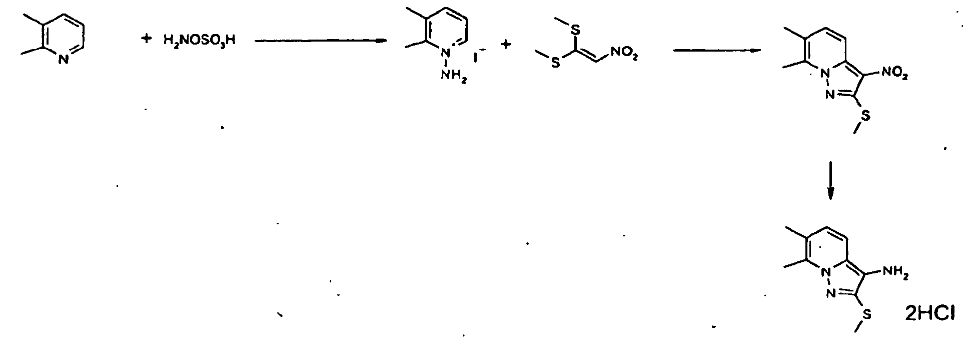

### Synthèse du 1-amino-2,3-diméthylpyridinium iodure

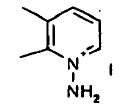

Dans un tricol de 1 litre, on introduit 400 ml d'eau et 64.2g de 2,3-diméthylpyridine que l'on porte à 30°C puis on ajoute 122.6g d' acide hydroxylamine-o-sulfonique.

En fin d'addition, le milieu est porté à 90°C pendant 7 heures. Après refroidissement à température ambiante 28g de carbonate de potassium sont ajoutés par portion sous agitation en une heure.

La solution aqueuse est alors lavée avec 5x300ml d'acétate d'éthyle et le résidu est repris avec 200ml d'éthanol, le solide obtenu est filtré et au filtrat refroidi à -60°C, on ajoute au goutte à goutte 56.9g d'acide iodhydrique à 45%.

L'agitation est poursuivie durant deux heures et le solide formé est essoré sur fritté n°3 et après séchage sous vide, en présence de P₂O₅, on récupère 31.5g g d'iodure de 1-amino-2,3-dimethylpyridinium.correspondant au composé attendu.

HNMR (200 MHz, CDCl₃),□ 8.06 (d, 1H), 7.46 (d, 1H), 2.76 (s, 3H), 2.67 (s, 3H), 2.43 (s, 3H)

### Synthèse du 6,7-dimethyl-2-(méthylsulfanyl)-3-nitropyrazolo[1,5-a]pyridine

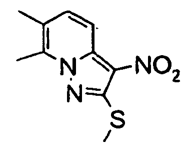

Dans un ballon de 1litre contenant 800ml d'éthanol anhydre, 31.5g d'iodure de 1-amino-2,3-dimethylpyridinium et 20.8 g de 1,1-bis(methylthio)-2-nitroethylene on ajoute 19.1g de triéthylamine au goutte à goutte.

Le milieu est porté au reflux pendant 3 heures puis refroidi à une température inférieure à 10°C. Le solide qui se forme sous agitation est essoré, lavé à l'éthanol et après séchage sous vide, en présence de P₂O₅, on récupère 8.32g de 6,7-dimethyl-2-(méthylsulfanyl)-3-nitropyrazolo[1,5-a]pyridine correspondant au composé attendu.

Les analyses RMN (¹H 400 MHz et ¹³C 100,61 MHz DMSO d₆) et spectrométrie de masse sont conformes à la structure attendue.

### Synthèse du dichlorhydrate de 6,7-Diméthyl-2-méthylsulfanyl-pyrazolo[1,5-a]pyridin-3-ylamine

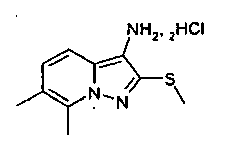

Dans un tricol de 250 ml, surmonté d'un réfrigérant à boules, d'un thermomètre et d'une agitation magnétique, on introduit 100 ml d'éthanol et 10g de poudre de zinc et on porte au reflux.

On ajoute alors au goutte à goutte 1 ml d'acide chlorhydrique à 35% puis par portion 4g de 6,7-dimethyl-2-(méthylsulfanyl)-3-nitropyrazolo[1,5-a]pyridine.

En fin d'addition, le reflux est maintenu 2 heures. La réduction étant terminée, le zinc est éliminé par filtration sous argon sur un lit de célite et le filtrat est recueilli dans une fiole contenant 50ml d'isopropanol chlorhydrique 6N préalablement refroidi.

Le filtrat est concentré au 1/3 de volume et nous observons alors la cristallisation d'un solide gris que l'on essore sur fritté et que l'on lave avec 2x100 ml d'éther isopropylique. Après séchage sous vide, en présence de P₂O₅ et de pastilles de soude, nous récupérons 3.6g d'une poudre grise correspondant au composé attendu.

Les analyses RMN (¹H 400 MHz et ¹³C 100,61 MHz DMSO d₆) sont conformes à la structure attendue.

Les ions quasi-moléculaires [M+H]+, [M+Na]+ et [M-H]- de la molécule attendue, C₁₀H₁₃N₃S, sont principalement détectés.

### Exemple 22 : dichlorhydrate N2-[2-(dimethylamino)ethyl]-6,7-dimethylpyrazolo[1,5-a]pyridine-2,3-diamine

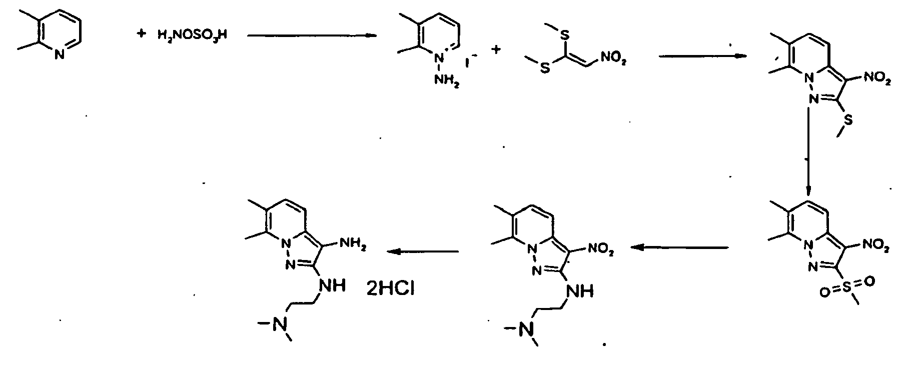

### Synthèse du dichlorhydrate N2-[2-(dimethylamino)ethyl]-6,7-dimethylpyrazolo[1,5-a]pyridine-2,3-diamine

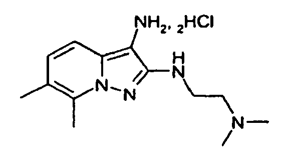

Dans un tricol de 500 ml, surmonté d'un réfrigérant à boules, d'un thermomètre et d'une agitation magnétique, on introduit 200 ml d'éthanol et 5g de poudre de zinc et on porte au reflux.

On ajoute alors au goutte à goutte 1 ml d'acide acétique puis par portion 4g de N'-(6,7-dimethyl-3-nitropyrazolo[1,5-a]pyridin-2-yl)-N,N-dimethylethane-1,2-diamine tout en ajoutant régulièrement de l'acide acétique.(au total 1.5 ml)

En fin de d'addition, le reflux est maintenu 2 heures. La réduction étant terminée, le zinc est éliminé par filtration sous argon sur un lit de célite et le filtrat est recueilli dans une fiole contenant 100ml d'isopropanol chlorhydrique 6N préalablement refroidi.

Un solide blanc cristallise, il est essoré sur fritté puis lavé avec 2x100 ml d'éther isopropylique. Après séchage sous vide, en présence de P₂O₅ et de pastilles de soude, nous récupérons 4.5g d'une poudre blanche correspondant au composé attendu.

Les analyses RMN (¹H 400 MHz et ¹³C 100,61 MHz DMSO d₆) et spectrométrie de masse sont conformes à la structure attendue.

### Exemple 22 : chlorhydrate 2-methoxy-6,7-dimethylpyrazolo[1,5-a]pyridin-3-amine

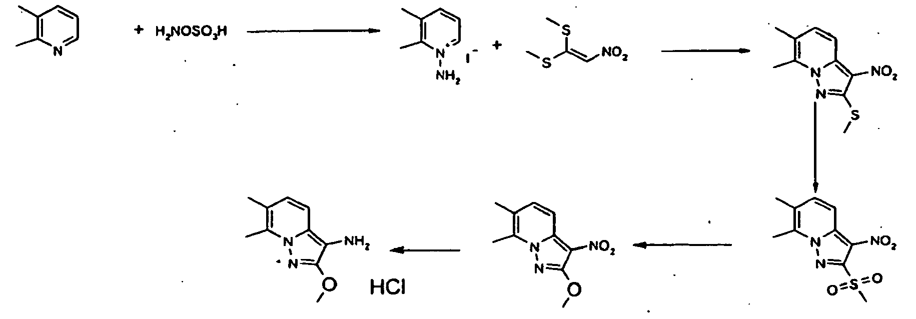

### Synthèse du chlorhydrate 2-methoxy-6,7-dimethylpyrazolo[1,5-a]pyridin-3-amine

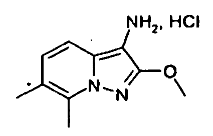

Dans un tricol de 250 ml, surmonté d'un réfrigérant à boules, d'un thermomètre et d'une agitation magnétique, on introduit 100 ml d'éthanol et 1.5g de poudre de zinc et on porte au reflux.

On ajoute alors 3 gouttes d'acide acétique puis par portion 1.5g de 2-methoxy-6,7-dimethyl-3-nitropyrazolo[1,5-a]pyridine tout en ajoutant régulièrement quelques gouttes d'acide acétique.

En fin d'addition, le reflux est maintenu 20 minutes. La réduction étant terminée, le zinc est éliminé par filtration sous argon sur un lit de célite et le filtrat est recueilli dans une fiole contenant 20ml d'isopropanol chlorhydrique 6N préalablement refroidi. Apres évaporation au 1/10 de la solution la cristallisation est amorcée et le solide beige cristallise abondamment par ajout de 50 ml de mélange d'isopropanol et d'éther.

Il est essoré sur fritté puis lavé avec 2x30 ml d'éther. Après séchage sous vide, en présence de P₂O₅ et de pastilles de soude, nous récupérons 700mg d'une poudre blanche correspondant au composé attendu.

Les analyses RMN (¹H 400 MHz et ¹³C 100,61 MHz DMSO d₆) et spectrométrie de masse sont conformes à la structure attendue.

### Exemple 23 : chlorhydrate de 2-isopropoxy-6,7-dimethylpyrazolo[1,5-a]pyridin-3-amine

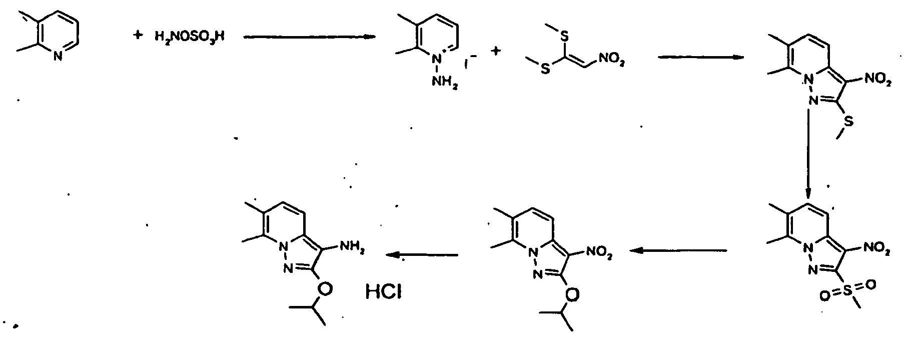

### Synthèse du chlorhydrate de 2-isopropoxy-6,7-dimethylpyrazolo[1,5-a]pyridin-3-amine

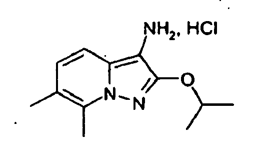

Dans un tricol de 250 ml, surmonté d'un réfrigérant à boules, d'un thermomètre et d'une agitation magnétique, on introduit 100 ml d'éthanol et 1.5g de poudre de zinc et on porte au reflux.

On ajoute alors 0.25 ml d'acide acétique puis par portion 1.5g 2-isopropoxy-6,7-dimethyl-3-nitropyrazolo[1,5-a]pyridine tout en ajoutant régulièrement quelques gouttes d'acide acétique.

En fin d'addition, e reflux est maintenu 1 heure. La réduction étant terminée, le zinc est éliminé par filtration sous argon sur un lit de célite et le filtrat est recueilli dans une fiole contenant 20ml d'isopropanol chlorhydrique 6N préalablement refroidi.

Apres évaporation au 1/10 de la solution on ajoute 50 ml d'heptane et on concentre à nouveau au 1/10 de volume ; on reprend alors avec 50 ml d'éther pour amorcer la cristallisation. Le solide formé est essoré sur fritté. Après séchage sous vide, en présence de P₂O₅ et de pastilles de soude, nous récupérons 560mg d'une poudre beige légèrement rose correspondant au composé attendu.

Les analyses RMN (¹H 400 MHz et ¹³C 100,61 MHz DMSO d₆) et spectrométrie de masse sont conformes à la structure attendue.

### Exemple 25 : N-2-[2-(dimethylamino)ethyl]-7,8-dihydro-6H-cyclopenta[e]pyrazolo[1,5-a]pyridine-2,3-diamine

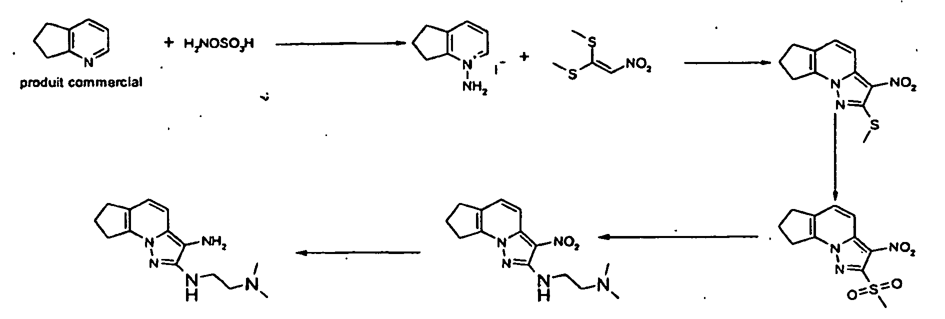

### Synthèse du 1-amino-6,7-dihydro-5H-cyclopenta[b]pyridinium iodide

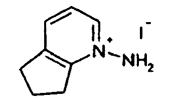

Dans un tricol de 1000 ml, surmonté d'un réfrigérant à boules, d'un thermomètre et d'une agitation mécanique, on introduit 400 ml d'eau, 123g (1,032 mole) 2,3-cyclopentenopyridine puis 46,6g(0,413mole) d'acide hydroxylamine-o-sulfonique par petite portion et on porte au reflux pendant 18 heures.

On refroidit le milieu à la température ambiante et on ajoute doucement 74,2g (0,537mole) de carbonate de potassium et on agite pendant 30 minutes.

La phase aqueuse est lavée avec 4x200ml d'acétate d'éthyle, puis co-évaporée avec du 2-propanol pour obtenir un solide marron que l'on reprend avec 400ml d'éthanol pour éliminer les sels.

La solution d'éthanol brune est introduite dans un tricol de 2 litres muni d'une ampoule isobare et est refroidie à -50°C sous agitation . On coule, goutte à goutte, 67,5ml (0,516mole) d'acide iodhydrique.

En fin de coulée, on laisse la température remonter jusqu'à zéro degré et on essore l'insoluble beige sur fritté. Ce solide est lavé avec 3x150ml d'éther isopropylique. Après séchage sous vide, en présence de P₂O₅, pendant 12 heures, on récupère 27,7g de solide beige correspondant au composé attendu.

Les analyses RMN (¹H 400 MHz et ¹³C 100,61 MHz DMSO d₆) et spectrométrie de masse sont conformes à la structure attendue.

Le cation attendu [C₈H₁₁N₂]+ est principalement détecté à m/z,ESP+=135. Détection d'ions I- en électrospray négatif.

### Synthèse du 2-(méthylsulfanyl)-3-nitro-7,8-dihydro-6H-cyclopenta[e] pyrazolo[1,5-a]pyridine

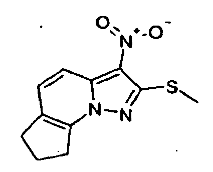

Dans un tricol de 2 litres équipé d'un compte bulle, d'un réfrigérant et d'une agitation mécanique, on introduit 500ml de N-Méthylpyrrolidinone et 65,56g (0,025mole) d'iodure de 1-amino-6,7-dihydro-5H-cyclopenta[b]pyridinium. Sous agitation on ajoute doucement 103,65g de carbonate de potassium en 15 minutes puis en une seule fois 41,33g(0,25mole) de 1,1-bis(méthythio)-2-nitroéthylène.

L'agitation est maintenue pendant 48 heures à la température ambiante et le milieu réactionnel est versé sur 2,5 litres de mélange glace - eau.

L'insoluble vert foncé formé est essoré sur fritté n°3 puis lavé abondamment à l'eau, 3x 200ml d'acétate d'éthyle puis avec 3x200 ml d' éther isopropylique.

Après séchage sous vide, en présence de P₂O₅, nous récupérons 36,77g de solide vert foncé correspondant au composé attendu.

Les analyses RMN (¹H 400 MHz et ¹³C 100,61 MHz DMSO d₆) et spectrométrie de masse sont conformes à la structure attendue.

Les ions quasi moléculaires [M+H]+, [M+Na]+, [2M+Na]+ de la molécule attendue C₁₁H₁₁N₃O₂S sont principalement détectés.

### Synthèse du 2-(méthylsulfonyl)-3-nitro-7,8-dihydro-6H-cyclopenta[e]pyrazolo[1,5-a]pyridine

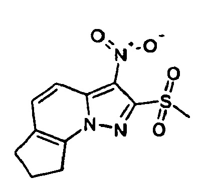

Dans un tricol de 3 litres, équipé d'une agitation mécanique et d'une sonde de température interne, sont chargés successivement 267,6 g d'oxone (3 éq.), 800 ml d'eau et 36,17g de 2-(méthylsulfanyl)-3-nitro-7,8-dihydro-6H-cyclopenta[e]pyrazolo[1,5-a]pyridine (0,145 mole) obtenu précédemment. Le tout est agité à la température ambiante.

Pour compléter la réaction, de l'oxone (89,2g, 1 éq.) est ajouté et après 4 heures sous agitation à la température ambiante, la réaction est terminée.

Le solide formé est essoré et lavé abondamment à l'eau jusqu'à l'obtention d'un filtrat ne contenant plus de peroxydes. Il est ensuite placé sous vide à 40°C sur P₂O₅. 35,31g de produit attendu sont obtenus (solide jaune).

Les analyses RMN (¹H 400 MHz et ¹³C 100,61 MHz DMSO d₆) et spectrométrie de masse sont conformes à la structure attendue.

Les ions quasi moléculaires [M-H]-, [M+H]+, [M+Na]+, [M+NA+CH3OH]+, [2M+Na]+ de la molécule attendue C₁₁H₁₁N₃O₄S sont principalement détectés.

### Synthèse du N,N-diméthyl-N'-(3-nitro-7,8-dihydro-6H-cyclopenta[e]pyrazolo[1,5-a]pyridin-2-yl)éthane-1,2-diamine

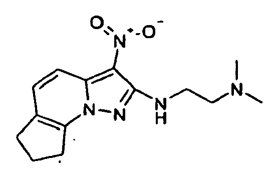

Dans un tricol de 250 ml, surmonté d'un réfrigérant à boules, d'un thermomètre et d'une agitation magnétique, on introduit 50 ml de N-Méthylpyrrolidinone, 10g (0,03555 mole) de 2-(méthylsulfonyl)-3-nitro-7,8-dihydro-6H-cyclopenta[e]pyrazolo[1,5-a]pyridine et 7,8 ml de N,N-diméthyléthylène diamine, on porte à 70°C à l'aide d'un bain d'huile, pendant 4 heures

Le milieu réactionnel est refroidi à température ambiante puis versé sur un mélange de 200g de glace et d'eau .Le composé jaune qui a cristallisé est essoré sur fritté, lavé par 2x100ml d'eau puis avec 3x100ml d'éther isopropylique. Après séchage à 35°C sous vide, en présence de P₂O₅, pendant 12 heures, on récupère 8,32g de solide vert correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires de la molécule attendue C₁₄H₁₉N₅O₂ sont principalement détectés.

### Synthèse du dicholorydrate de N-2-[2-(diméthylamino)ethyl]-7,8-dihydro-6H-cyclopenta[e]pyrazolo[1,5-a]pyridine-2,3-diamine

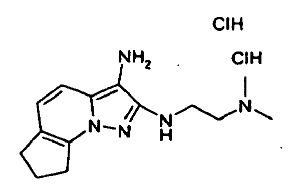

Dans un tricol de 250 ml, surmonté d'un réfrigérant à boules, d'un thermomètre et d'une agitation magnétique, on introduit 150 ml d'éthanol et 2g de poudre de zinc et on porte au reflux.

On ajoute alors au goutte à goutte 2 ml d'acide acétique puis une solution de 5ml d'eau et 2g de N,N-diméthyl-N'-(3-nitro-7,8-dihydro-6H-cyclopenta[e]pyrazolo[1,5-a]pyridin-2-yl)éthane-1,2-diamine.

En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide gris bleu que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 1.92g d'une poudre bleu vert correspondant au composé attendu.

Les analyses RMN (¹H 400 MHz et ¹³C 100,61 MHz DMSO d₆) et spectrophotométrie de masse sont conformes à la structure attendue.

L'ion quasi moléculaire [M+H]+ de la molécule attendue C₁₄H₂₁N₅ est principalement détecté

### Exemple 26 : chlorhydrate de 2-[(3-amino-7,8-dihydro-6H-cyclopenta[e]pyrazolo[1,5-a]pyridin-2-yl)amino]éthanol

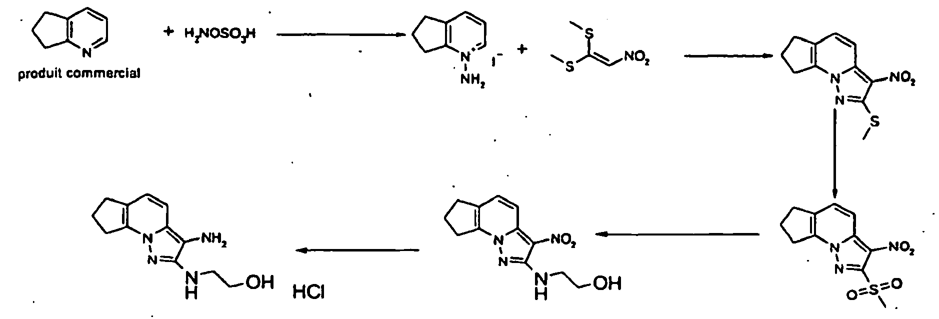

### Synthèse du 2-[(3-nitro-7,8-dihydro-6H-cyclopenta[e]pyrazolo[1,5-a]pyridin-2-yl)amino]ethanol

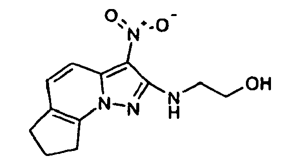

Dans un tricol de 250 ml, surmonté d'un réfrigérant à boules, d'un thermomètre et d'une agitation magnétique, on introduit 50 ml de N-Méthylpyrrolidinone, 10g (0,03555 mole) de 2-(méthylsulfonyl)-3-nitro-7,8-dihydro-6H-cyclopenta[e]pyrazolo[1,5-a]pyridine et 4.29 ml d'éthanolamine, on porte à 70°C à l'aide d'un bain d'huile, pendant 5 heures. Le milieu réactionnel est refroidi à température ambiante puis versé sur un mélange de 200g de glace et d'eau .Le composé jaune qui a cristallisé est essoré sur fritté n°3, lavé à l'eau 2x100ml puis avec 3x100ml d'éther isopropylique. Après séchage à 35°C sous vide, en présence de P₂O₅, pendant 12 heures, on récupère 8,70g de solide vert correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires [M+H]+, [M+Na]+, [M+Na+CH₃OH]+, [M+Na+CH₃CN]+, [2M+Na]+, [M-H]- de la molécule attendue C₁₂H₁₄N₄O₃ sont principalement détectés

### Synthèse du chlorhydrate de 2-[(3-amino-7,8-dihydro-6H-cyclopenta[e]pyrazolo[1,5-a]pyridin-2-yl)amino]éthanol

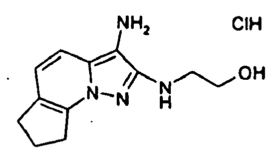

Dans un tricol de 100 ml, surmonté d'un réfrigérant à boules, d'un thermomètre et équipé d'une agitation magnétique, on introduit 50 ml d'éthanol et 2g de poudre de zinc et on porte au reflux.

On ajoute alors au goutte à goutte 2 ml d'acide acétique puis par portion 1g de 2-[(3-nitro-7,8-dihydro-6H-cyclopenta[e]pyrazolo[1,5-a]pyridin-2-yl)amino]éthanol.

En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide gris bleu que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 0.96g d'une poudre bleu vert correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires [M+H]+, [M+Na]+, [M+H+CH₃OH]+, [M-H]-, [M+Cl]- de la molécule attendue C₁₂H₁₆N₄O sont détectés.

### Exemple 27 : chlorhydrate de la 2-méthoxy-7,8-dihydro-6H-cyclopenta[e]pyrazolo[1,5-a]pyridin-3-amine

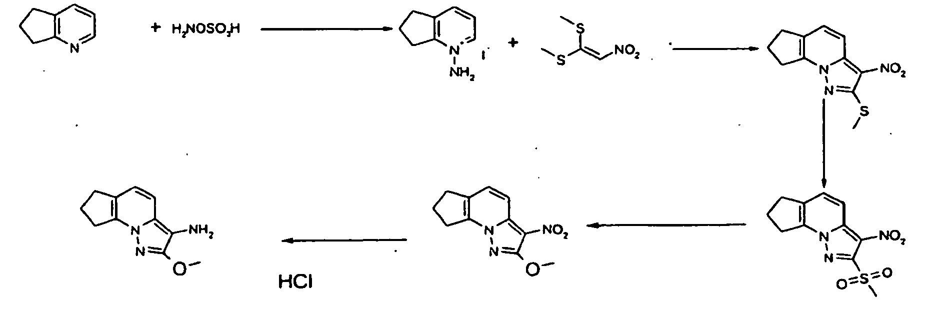

### Synthèse du 2-methoxy-3-nitro-7,8-dihydro-6H-cyclopenta[e]pyrazolo[1,5-a]pyridine

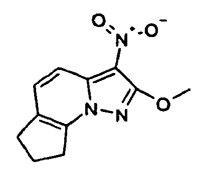

Dans un tricol de 250 ml, surmonté d'un réfrigérant à boules, d'un thermomètre et d'une agitation magnétique, on introduit 35ml de méthanol, 10g (0,03555 mole) de 2-(méthylsulfonyl)-3-nitro-7,8-dihydro-6H-cyclopenta[e]pyrazolo[1,5-a]pyridine et 33 ml de méthylate de sodium à 30%, on agite pendant 18 heures.

Le composé qui a cristallisé est essoré sur fritté, lavé par 2x25ml d'eau 10ml de méthanol puis avec 3x50ml d'éther isopropylique. Après séchage à 35°C sous vide, en présence de P₂O₅, pendant 12 heures, on récupère 7.82g de solide beige correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires [M+H]+, [M+Na]+, [M+Na+CH₃OH]+, [2M+Na]+ sont principalement détectés

### Synthèse du chlorhydrate de la 2-méthoxy-7,8-dihydro-6H-cyclopenta[e]pyrazolo[1,5-a]pyridin-3-amine.

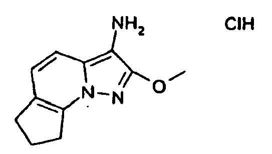

Dans un tricol de 500 ml, surmonté d'un réfrigérant à boules, d'un thermomètre et d'une agitation magnétique, on introduit 50 ml d'éthanol et 5g de poudre de zinc et on porte au reflux.

On ajoute alors au goutte à goutte 5 ml d'acide acétique puis par portion 5g de 2-méthoxy-3-nitro-7,8-dihydro-6H-cyclopenta[e]pyrazolo[1,5-a]pyridine. En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

On observe alors la précipitation d'un solide bleu gris que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 3.92g d'une poudre bleu gris correspondant au composé attendu.

Les analyses RMN (¹H 400 MHz et ¹³C 100,61 MHz DMSO d₆) et spectrophotométrie de masse sont conformes à la structure attendue.

Le cation attendu [C₁₅H₂₂N₅O₂]+ est principalement détecté.

### Exemple 28 : chlorhydrate de 2-isopropoxy-7,8-dihydro-6H-cyclopenta[e]pyrazolo[1,5-a]pyridin-3-amine

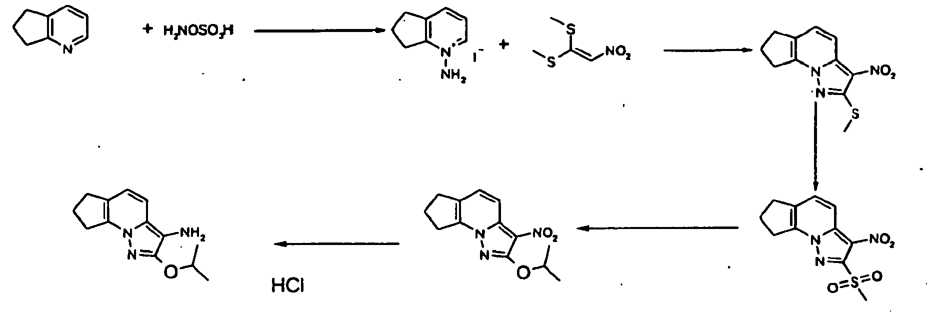

### Synthèse du 2-isopropoxy-3-nitro-7,8-dihydro-6H-cyclopenta[e]pyrazolo[1,5-a]pyridine

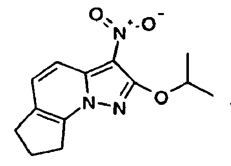

Dans un tricol de 250 ml, surmonté d'un réfrigérant à boules, d'un thermomètre et d'une agitation magnétique, on introduit 100ml de THF, 10g (0,03555 mole) 2-(méthylsulfonyl)-3-nitro-7,8-dihydro-6H-cyclopenta[e]pyrazolo[1,5-a]pyridine et 7.29g (0.08887 mole) de sodium isopropoxide par portion, on agite pendant 30 minutes. On verse sur 300ml d'eau et le composé qui a cristallisé est essoré sur fritté n°3, lavé à l'eau abondamment puis avec 4x50ml d'éther isopropylique. Après séchage à 35°C sous vide, en présence de P₂O₅, pendant 12 heures, on récupère 3.70g de solide jaune foncé correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires [M+H]+, [M+Na]+, [M+Na+CH₃OH]+, [2M+Na]+ sont principalement détectés

### Synthèse du chlorhydrate de 2-isopropoxy-7,8-dihydro-6H-cyclopenta[e]pyrazolo[1,5-a]pyridin-3-amine

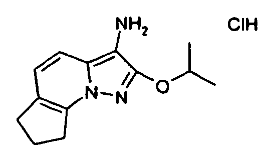

Dans un tricol de 250 ml, surmonté d'un réfrigérant à boules, d'un thermomètre et d'une agitation magnétique, on introduit 150 ml d'éthanol et 3g de poudre de zinc et on porte au reflux.

On ajoute alors au goutte à goutte 2 ml d'acide acétique puis par portion 3g de 2-isopropoxy-3-nitro-7,8-dihydro-6H-cyclopenta[e]pyrazolo[1,5-a]pyridine. En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et le filtrat est recueilli dans une fiole contenant 50ml d'isopropanol chlorhydrique 6N préalablement refroidi.

Le filtrat est concentré au 1/3 puis repris plusieurs fois à l'éther, On observe alors la cristallisation lente d'un solide vert clair que l'on essore sur fritté n°3 et que l'on lave avec 2x50 ml d'éther. Après séchage sous vide, en présence de P₂O₅ et de pastilles de soude, nous récupérons 1.67g d'une poudre rose pale correspondant au composé attendu.

Les analyses RMN (¹H 400 MHz et ¹³C 100,61 MHz DMSO d₆) et spectrophotométrie de masse sont conformes à la structure attendue.

Les ions quasi moléculaires [M+H]+, [M+Na]+, [M+H+CH₃OH] de la molécule attendue C₁₀H₁₃N₃O sont détectés.

### Exemple 29 : Chlorhydrate de 2-(2-piperidin-1-ylethoxy)pyrazolo[1,5-a]pyridin-3-amine

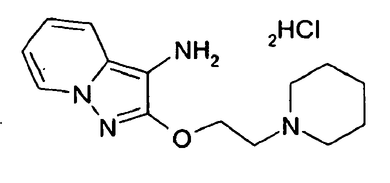

### Synthèse générale

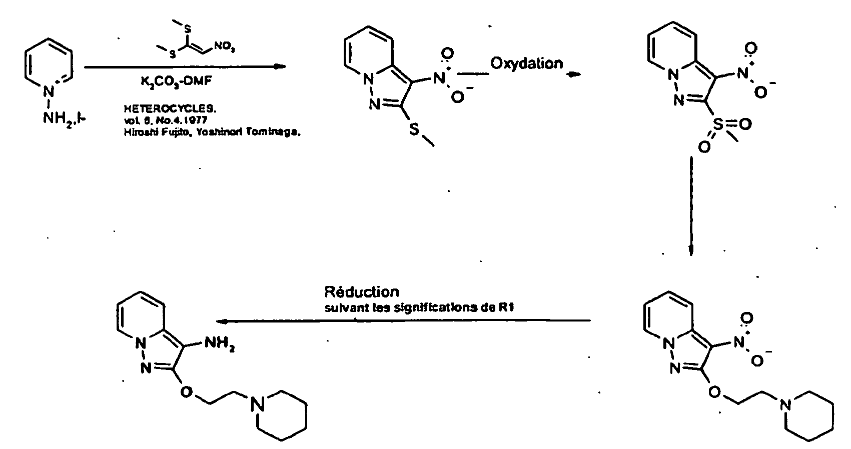

### Synthèse du 3-nitro-2-(2-piperidin-1-ylethoxy)pyrazolo[1,5-a]pyridine

Dans un tricol de 250 ml, surmonté d'un réfrigérant à boules, d'un thermomètre et d'une agitation magnétique, on introduit 10ml d'eau et 13.8 ml(0,103mole) de N-(2-hydroxyethyl)pipéridine que l'on refroidit à zéro degrés puis une solution issue de 8.29 g(0,207mole) de soude et 20 ml d'eau est ajoutée en 15 minutes.

Cette solution est agitée à cette même température pendant 15 minutes puis une solution issue de 100ml de N-methylpyrrolidinone et 10g (0,04145 mole) 2-(methylsulfonyl)-3-nitropyrazolo[1,5-a]pyridine est ajoutée au goutte à goutte en 20 minutes.

Le milieu violet hétérogène obtenu est agité pendant 4 heures puis à température ambiante. Le solide formé est essoré lavé à l'eau jusqu'à pH neutre puis avec 4x50ml d'éther isopropylique. Après séchage à 35°C sous vide, en présence de P2O5, pendant 12 heures, on récupère 3.70g de solide beige correspondant au composé attendu.

Les analyses RMN (1H 400 MHz et ¹³C 100,61 MHz DMSO d6) et spectrophotométrie de masse sont conformes à la structure attendue.

Les ions quasi moléculaires [M+H]+, [M+Na]+, [M+Na+CH3OH]+, [2M+Na]+ sont principalement détectés

### Synthèse du chlorhydrate de 2-(2-piperidin-1-ylethoxy)pyrazolo[1,5-a]pyridin-3-amine

Dans un tricol de 100 ml, surmonté d'un réfrigérant à boules, d'un thermomètre et d'une agitation magnétique, on introduit 30 ml d'éthanol et 2.75g de poudre de zinc et on porte au reflux. On ajoute alors au goutte à goutte 550 micro litre d'acide acétique puis par portion on ajoute 2,75g de 3-nitro-2-(2-piperidin-1-ylethoxy)pyrazolo[1,5-a]pyridine En fin d'ajout, on ajoute 2 gouttes d'acide acétique et le reflux est maintenu 4 heures.

Lorsque la réduction est terminée, le zinc est éliminé par filtration sous argon sur un litde célite et le filtrat est recueilli dans une fiole contenant 100ml d'isopropanol chlorhydrique 6N préalablement refroidi.

Le filtrat est concentré au 1/3 puis repris plusieurs fois à l'éther, On observe alors la cristallisation lente d'un solide violet que l'on essore sur fritté n°3 et que l'on lave avec le minimum d'isopropanol puis avec 3x30 ml d'éther . Après séchage sous vide, en présence de P2O5 et de pastilles de soude, nous récupérons 1.71g d'une poudre rose pale correspondant au composé attendu.

Les analyses RMN (1H 400 MHz et 13C 100,61 MHz DMSO d6) et spectrophotométrie de masse sont conformes à la structure attendue.

Les ions quasi moléculaires [M+H]+, [M+Na]+, [M+H+CH3OH] de la molécule attendue C14H20N4O sont détectés.

### Exemple 30 : Chlorhydrate de 2-[2-(dimethylamino)ethoxy]pyrazolo[1,5-a]pyridin-3-amine

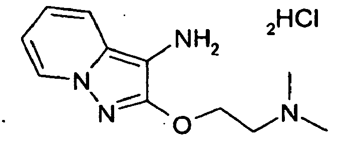

### Synthèse générale

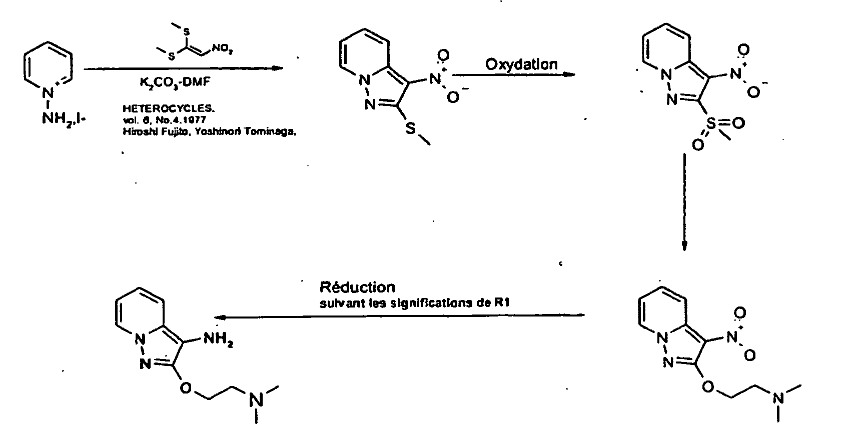

### Synthèse du N,N-dimethyl-2-[(3-nitropyrazolo[1,5-a]pyridin-2-yl)oxy]ethanamine

Dans un tricol de 250 ml, surmonté d'un réfrigérant à boules, d'un thermomètre et d'une agitation magnétique, on introduit 10ml d'eau et 10,3ml(0,103mole) de N, N-dimethylethanolamine que l'on refroidit à zéro degrés puis une solution issue de 8.29 g(0,207mole) de soude et 20 ml d'eau est ajoutée en 15 minutes.

Cette solution est agitée à cette même température pendant 15 minutes puis une solution issue de 100ml de N-methylpyrrolidinone et 10g (0,04145 mole) 2-(methylsulfonyl)-3-nitropyrazolo[1,5-a]pyridine est ajoutée au goutte à goutte en 20 minutes.

Le milieu est légèrement violet obtenu est agité pendant 6 heures puis à température ambiante. Le milieu réactionnel est versé sur 500 ml d'eau et le solide beige formé est essoré lavé à l'eau jusqu'à pH neutre puis avec 4x50ml d'éther isopropylique. Après séchage à 35°C sous vide, en présence de P2O5, pendant 12 heures, on récupère 5,01g de solide beige correspondant au composé attendu.

Les analyses RMN (1H 400 MHz et 13C 100,61 MHz DMSO d6) et spectrophotométrie de masse sont conformes à la structure attendue.

Les ions quasi moléculaires [M+H]+, [M+Na]+, [M+Na+CH3OH]+, [2M+Na]+ sont principalement détectés

### Synthèse du chlorhydrate de 2-[2-(dimethylamino)ethoxy]pyrazolo[1,5-a]pyridin-3-amine

Dans un tricol de 100 ml, surmonté d'un réfrigérant à boules, d'un thermomètre et d'une agitation magnétique, on introduit 20 ml d'éthanol et 2g de poudre de zinc et on porte au reflux. On ajoute alors deux gouttes d'acide acétique puis par portion on ajoute 2g de N,N-dimethyl-2-[(3-nitropyrazolo[1,5-a]pyridin-2-yl)oxy]ethanamine. En fin d'ajout, on ajoute 2 gouttes d'acide acétique et le reflux est maintenu 17 heures.

Lorsque la réduction est terminée, le zinc est éliminé par filtration sous argon sur un lit de célite et le filtrat est recueilli dans une fiole contenant 100ml d'isopropanol chlorhydrique 6N préalablement refroidi.

Le filtrat est concentré au 1/3 puis repris plusieurs fois à l'éther, On observe alors la cristallisation lente d'un solide violet que l'on essore sur fritté n°3 et que l'on lave avec le minimum d'isopropanol puis avec 3x30 ml d'éther . Après séchage sous vide, en présence de P2O5 et de pastilles de soude, nous récupérons 2,80g d'une poudre violet pale correspondant au composé attendu.

Les analyses RMN (1H 400 MHz et 13C 100,61 MHz DMSO d6) et spectrophotométrie de masse sont conformes à la structure attendue.

Les ions quasi moléculaires [M+H]+, [M+Na]+, [M+H+CH3OH] de la molécule attendue C11H16N4O sont détectés.

### EXEMPLES DE TEINTURE

Les compositions tinctoriales suivantes sont préparées

| | | | | | | | |
|---|---|---|---|---|---|---|---|
| 2-isopropoxypyrazolo[ 1,5-a]pyridin-3-amine chlorhydrate | 10⁻³ mole | 10⁻³ mole | 10⁻³ mole | 10⁻³ mole | 10⁻³ mole | 10⁻³ mole | 10⁻³ mole |
| Benzene-1,3-diol | 10⁻³ mole | | | | | | |
| 5-Amino-2-méthyl-phenol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Dimethyl-1H-pyrazolo[5,1-c][1,2 ,4]triazole | | | | | 10⁻³ mole | | |
| 2-(2,4-Diamino-phenoxy)-ethanol,chlorhydrate | | | | | | 10⁻³ mole | |
| 3-Amino-2-chicro-6-methyl-phenol,chlorhydrate | | | | | | | 10⁻³ mole |
| Support de teinture (1) | (*) | (*) | (*) | (*) | (*) | (*) | (*) |
| Eau déminéralisée q.s.p. | 100g | 100g | 100g | 100g | 100g | 100g | 100g |
| **Nuance observée** | rouge intense | rouge chromatique intense | rouge intense | violet-rouge intense | rouge chromatique | rouge intense | rouge chromatique intense |

| | | | | |
|---|---|---|---|---|
| 2-[3-(dimethylamino)pyrrolidin-1-yl]pyrazolo[1,5-a]pyridin-3-amine chlorhydrate | 10⁻³ mole | 10⁻³ mole | 10⁻³ mole | 10⁻³ mole |
| 5-Amino-2-methyl-phenol | 10⁻³ mole | | | |
| 1H-Indol-6-ol | | 10⁻³ mole | | |
| 3,6-Dimethyl-1H-pyrazolo[5,1-c][1,2,4]triazole | | | 10⁻³ mole | |
| 2-(2,4-Diamino-phenoxy)-ethanol,chlorhydrate | | | | 10⁻³ mole |
| Support de teinture (2) | (*) | (*) | (*) | (*) |
| Eau déminéralisée q.s.p. | 100g | 100g | 100g | 100g |
| **Nuance observée** | bleu intense | brun orangé | rouge | gris vert-bleu intense |

| | | |
|---|---|---|
| 2-[3-(dimethylamino)pyrrolidin-1-yl]pyrazolo[1,5-a]pyridin-3-amine chlorhydrate | 10⁻³ mole | 10⁻³ mole |
| 5-Amino-2-methyl-phenol | 10⁻³ mole | |
| 2-(2,4-Diamino-phenoxy)-ethanol,chlorhydrate | | 10⁻³ mole |
| Support de teinture (1) | (*) | (*) |
| Eau déminéralisée q.s.p. | 100g | 1 00g |
| **Nuance observée** | gris bleu | gris vert-bleu |

| | | | | | | |
|---|---|---|---|---|---|---|
| 2-methoxy-7-methylpyrazolo[1,5-a]pyridin-3-amine chlorhydrate | 10⁻³ mole | 10⁻³ mole | 10⁻³ mole | 10⁻³ mole | 10⁻³ mole | 10⁻³ mole |
| 5-Amino-2-methyl-phenol | 10⁻³ mole | | | | | |
| 1H-Indol-6-ol | | 10⁻³ mole | | | | |
| 2-Amino-pyridin-3-ol | | | 10⁻³ mole | | | |
| 3,6-Dimethyl-1H-pyrazolo[5,1-c][1,2 ,4]triazole | | | | 10⁻³ mole | | |
| 2-(2,4-Diamino-phenoxy)-ethanol,chlorhydrate | | | | | 10⁻³ mole | |
| 3-Amino-2-chloro-6-methyl-phenol,chlorhydrate | | | | | | 10⁻³ mole |
| Support de teinture (2) | (*) | (*) | (*) | (*) | (*) | (*) |
| Eau déminéralisée q.s.p. | 100g | 100g | 100g | 100g | 100g | 100g |
| **Nuance observée** | rouge chromatique intense | rouge intense rouge | brun | rouge chromatique | rouge intense | rouge chromatique intense |

| | | | | | | |
|---|---|---|---|---|---|---|
| 2-methoxy-7-methylpyrazolo[1,5-a]pyridin-3-amine chlorhydrate | 10⁻³ mole | 10⁻³ mole | 10⁻³ mole | 10⁻³ mole | 10⁻³ mole | 10⁻³ mole |
| 5-Amino-2-methyl-phenol | 10⁻³ mole | | | | | |
| 1H-Indol-6-ol | | 10⁻³ mole | | | | |
| 2-Amino-pyridin-3-ol | | | 10⁻³ mole | | | |
| 3,6-Dimethyl-1H-pyrazolo[5,1-c][1,2 ,4]triazole | | | | 10⁻³ mole | | |
| 2-(2,4-Diamino-phenoxy)-ethanol,chlorhydrate | | | | | 10⁻³ mole | |
| 3-Amino-2-chloro-6-methyl-phenol,chlorhydrate | | | | | | 10⁻³ mole |
| Support de teinture (1) | (*) | (*) | (*) | (*) | (*) | (*) |
| Eau déminéralisée | 100g | 100g q.s.p. | 100g | 100g | 100g | 100g |
| **Nuance observée** | rouge chromatique intense | brun rouge intense | brun rouge | rouge chromatique | rouge intense | rouge chromatique intense |

| | | |
|---|---|---|
| 2-pyrrolidin-1-ylpyrazolo[1,5-a]pyridin-3-amine chlorhydrate | 10⁻³ mole | 10⁻³ mole |
| 5-Amino-2-methyl-phenol | 10⁻³ mole | |
| 2-(2,4-Diamino-phenoxy)-ethanol,chlorhydrate | | 10⁻³ mole |
| Support de teinture (2) | (*) | (*) |
| Eau déminéralisée q.s.p. | 100g | 100g |
| **Nuance observée** | gris vert | gris vert-bleu intense |

| | | |
|---|---|---|
| 2-pyrrolidin-1-ylpyrazolo[1,5-a]pyridin-3-amine chlorhydrate | 10⁻³ mole | 10⁻³ mole |
| 5-Amino-2-methyl-phenol | 10⁻³ mole | |
| 2-(2,4-Diamino-phenoxy)-ethanol,chlorhydrate | | 10⁻³ mole |
| Support de teinture (1) | (*) | (*) |
| Eau déminéralisée q.s.p. | 100g | 100g |
| **Nuance observée** | vert-bleu | gris vert-bleu |

| | | | | | | |
|---|---|---|---|---|---|---|
| N2-[3-(1H-imidazol-1-yl)propyl]pyrazolo[1,5-a]pyridine-2,3-diamine chlorhydrate | 10⁻³ mole | 10⁻³ mole | 10⁻³ mole | 10⁻³ mole | 10⁻³ mole | 10⁻³ mole |
| Benzene-1,3-diol | 10⁻³ mole | | | | | |
| 5-Amino-2-methyl-phenol | | 10⁻³ mole | | | | |
| 1H-Indol-6-ol | | | 10⁻³ mole | | | |
| 3,6-Dimethyl-1H-pyrazolo[5,1-c][1,2 ,4]triazole | | | | 10⁻³ mole | | |
| 2-(2,4-Diamino-phenoxy)-ethanol,chlorhydrate | | | | | 10⁻³ mole | |
| 3-Amino-2-chloro-6-methyl-phenol,chlorhydrate | | | | | | 10⁻³ mole |
| Support de teinture (2) | (*) | (*) | (*) | (*) | (*) | (*) |
| Eau déminéralisée q.s.p. | 100g | 100g | 100g | 100g | 100g | 100g |
| **Nuance observée** | gris vert | gris vert-bleu | gris vert | gris vert | vert-bleu | vert-bleu |

| | | | |
|---|---|---|---|
| N2-[3-(1H-imidazol-1-yl)propyl]pyrazolo[1,5-a]pyridine-2,3-diamine chlorhydrate | 10⁻³ mole | 10⁻³ mole | 10⁻³ mole |
| 5-Amino-2-methyl-phenol | 10⁻³ mole | | |
| 2-(2,4-Diamino-phenoxy)-ethanol,chlorhydrate | | 10⁻³ mole | |
| 3-Amino-2-chloro-6-methyl-phenol,chlorhydrate | | | 10⁻³ mole |
| Support de teinture (1) | (*) | (*) | (*) |
| Eau déminéralisée q.s.p. | 100g | 100g | 100g |
| **Nuance observée** | gris bleu | gris vert | vert-bleu |

| | | | | |
|---|---|---|---|---|
| 2-(4-methylpiperazin-1-yl)pyrazolo[1,5-a]pyridin-3-amine chlorhydrate | 10⁻³ mole | 10⁻³ mole | 10⁻³ mole | 10⁻³ mole |
| 5-Amino-2-methyl-phenol | 10⁻³ mole | | | |
| 1H-Indol-6-ol | | 10⁻³ mole | | |
| 2-(2,4-Diamino-phenoxy)-ethanol,chlorhydrate | | | 10⁻³ mole | |
| 3-Amino-2-chloro-6-methyl-phenol,chlorhydrate | | | | 10⁻³ mole |
| Support de teinture (2) | (*) | (*) | (*) | (*) |
| Eau déminéralisée q.s.p. | 100g | 100g | 100g | 100g |
| **Nuance observée** | gris violet-rouge | orangé | gris vert-bleu intense | gris bleu |

| | | | |
|---|---|---|---|
| 2-(4-methylpiperazin-1-yl)pyrazolo[1,5-a]pyridin-3-amine chlorhydrate | 10⁻³ mole | 10⁻³ mole | 10⁻³ mole |
| 5-Amino-2-methyl-phenol | 10⁻³ mole | | |
| 2-(2,4-Diamino-phenoxy)-ethanol,chlorhydrate | | 10⁻³ mole | |
| 3-Amino-2-chloro-6-methyl-phenol,chlorhydrate | | | 10⁻³ mole |
| Support de teinture (1) | (*) | (*) | (*) |
| Eau déminéralisée q.s.p. | 100g | 100g | 100g |
| **Nuance observée** | gris violet | gris vert-bleu | gris bleu |

| | |
|---|---|
| 2-[(3-aminopyrazolo[1,5-a]pyridin-2-yl)amino]ethanol chlorhydrate | 10⁻³ mole |
| 2-(2,4-Diamino-phenoxy)-ethanol,chlorhydrate | 10⁻³ mole |
| Support de teinture (2) | (*) |
| Eau déminéralisée q.s.p. | 100g |
| **Nuance observée** | gris vert-bleu |

| | | |
|---|---|---|
| 2-[(3-aminopyrazolo[1,5-a]pyridin-2-yl)amino]ethanol chlorhydrate | 10⁻³ mole | 10⁻³ mole |
| 2-(2,4-Diamino-phenoxy)-ethanol,chlorhydrate | 10⁻³ mole | |
| 3-Amino-2-chloro-6-methyl-phenol,chlorhydrate | | 10⁻³ mole |
| Support de teinture (1) | (*) | (*) |
| Eau déminéralisée q.s.p. | 100g | 100g |
| **Nuance observée** | vert-bleu intense | bleu |

| | |
|---|---|
| 1-[(3-aminopyrazolo[1,5-a]pyridin-2-yl)amino]propan-2-ol chlorhydrate | 10⁻³ mole |
| 2-(2,4-Diamino-phenoxy)-ethanol,chlorhydrate | 10⁻³ mole |
| Support de teinture (2) | (*) |
| Eau déminéralisée q.s.p. | 100g |
| **Nuance observée** | gris vert |

| | | | |
|---|---|---|---|
| 1-[(3-aminopyrazolo[1,5-a]pyridin-2-yl)amino]propan-2-ol chlorhydrate | 10⁻³ mole | 10⁻³ mole | 10⁻³ mole |
| 5-Amino-2-methyl-phenol | 10⁻³ mole | | |
| 2-(2,4-Diamino-phenoxy)-ethanol,chlorhydrate | | 10⁻³ mole | |
| 3-Amino-2-chloro-6-methyl-phenol,chlorhydrate | | | 10⁻³ mole |
| Support de teinture (1) | (*) | (*) | (*) |
| Eau déminéralisée q.s.p. | 100g | 100g | 100g |
| **Nuance observée** | gris bleu | gris vert | vert-bleu |

| | |
|---|---|
| N2-ethylpyrazolo[1,5-a]pyridine-2,3-diamine chlorhydrate | 10⁻³ mole |
| 2-(2,4-Diamino-phenoxy)-ethanol,chlorhydrate | 10⁻³ mole |
| Support de teinture (2) | (*) |
| Eau déminéralisée q.s.p. | 100g |
| **Nuance observée** | gris jaune-vert |

| | |
|---|---|
| N2-ethylpyrazolo[1,5-a]pyridine-2,3-diamine chlorhydrate | 10⁻³ mole |
| 3-Amino-2-chloro-6-methyl-phenol,chlorhydrate | 10⁻³ mole |
| Support de teinture (1) | (*) |
| Eau déminéralisée q.s.p. | 100g |
| **Nuance observée** | vert-bleu |

| | | | | |
|---|---|---|---|---|
| 2-[(3-amino-7,8-dihydro-6H-cyclopenta[e]pyrazolo[1,5-a]pyridin-2-yl)amino]ethanol chlorhydrate | 10⁻³ mole | 10⁻³ mole | 10⁻³ mole | 10⁻³ mole |
| 5-Amino-2-methyl-phenol | 10⁻³ mole | | | |
| 1H-Indol-6-ol | | 10⁻³ mole | | |
| 2-(2,4-Diamino-phenoxy)-ethanol,chlorhydrate | | | 10⁻³ mole | |
| 3-Amino-2-chloro-6-methyl-phenol,chlorhydrate | | | | 10⁻³ mole |
| Support de teinture (1) | (*) | (*) | (*) | (*) |
| Eau déminéralisée q.s.p. | 100g | 100g | 100g | 100g |
| **Nuance observée** | vert | jaune-vert | vert | vert-bleu |

| | | | | | | | |
|---|---|---|---|---|---|---|---|
| 2-[(3-amino-7,8-dihydro-6H-cyclopenta[e]pyrazolo[1,5-a]pyridin-2-yl)amino]ethanol chlorhydrate | 10⁻³ mole | 10⁻³ mole | 10⁻³ mole | 10⁻³ mole | 10⁻³ mole | 10⁻³ mole | 10⁻³ mole |
| Benzene-1,3-diol | mole | | | | | | |
| 5-Amino-2-methyl-phenol | | mole | | | | | |
| 1H-Indol-6-ol1H-Indol-6-ol | | | 10⁻³ mole | | | | |
| 2-Amino-pyridin-3-ol | | | | 10⁻³ mole | | | |
| 3,6-Dimethyl-1H-pyrazolo[5,1-c][1,2 ,4]triazole | | | | | 10⁻³ mole | | |
| 2-(2,4-Diamino-phenoxy)-ethanol,chlorhydrate | | | | | | 10⁻³ mole | |
| 3-Amino-2-chloro-6-methyl-phenol,chlorhydrate | | | | | | | 10⁻³ mole |
| Support de teinture (2) | (*) | (*) | (*) | (*) | (*) | (*) | (*) |
| Eau déminéralisée q.s.p. | 100g | 100g | 100g | 100g | 100g | 100g | 100g |
| **Nuance observée** | vert | vert | gris vert | vert | vert | gris vert | vert |

| | | | | | | |
|---|---|---|---|---|---|---|
| 2-methoxy-7,8-dihydro-6H-cyclopenta[e]pyrazolo[1,5-a]pyridin-3-amine chlorhydrate | 10⁻³ mole | 10⁻³ mole | 10⁻³ mole | 10⁻³ mole | 10⁻³ mole | 10⁻³ mole |
| Benzene-1,3-diol | 10⁻³ mole | | | | | |
| 1H-Indol-6-ol | | 10⁻³ mole | | | | |
| 2-Amino-pyridin-3-ol | | | 10⁻³ mole | | | |
| 3,6-Dimethyl-1H-pyrazolo[5,1-c][1,2 ,4]triazole | | | | 10⁻³ mole | | |
| 2-(2,4-Diamino-phenoxy)-ethanol,chlorhydrate | | | | | 10⁻³ mole | |
| 3-Amino-2-chloro-6-methyl-phenol,chlorhydrate | | | | | | 10⁻³ mole |
| Support de teinture (1) | (*) | (*) | (*) | (*) | (*) | (*) |
| Eau déminéralisée q.s.p. | 100g | 100g | 100g | 100g | 100g | 100g |
| **Nuance observée** | gris violet-rouge intense | gris rouge intense | gris | rouge chromatique intense | brun rouge intense | rouge intense |

| | | | | | | | |
|---|---|---|---|---|---|---|---|
| 2-methoxy-7,8-dihydro-6H-cyclopenta[e]pyrazolo[1,5-a]pyridin-3-amine chlorhydrate | 10⁻³ mole | 10⁻³ mole | 10⁻³ mole | 10⁻³ mole | 10⁻³ mole | 10⁻³ mole | 10⁻³ mole |
| Benzene-1,3-diol | 10⁻³ mole | | | | | | |
| 5-Amino-2-methyl-phenol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Dimethyl-1H-pyrazolo[5,1-c][1,2 ,4]triazole | | | | | 10⁻³ mole | | |
| 2-(2,4-Diamino-phenoxy)-ethanol,chlorhydrate | | | | | | 10⁻³ mole | |
| 3-Amino-2-chloro-6-methyl-phenol,chlorhydrate | | | | | | | 10⁻³ mole |
| Support de teinture (2) | (*) | (*) | (*) | (*) | (*) | (*) | (*) |
| Eau déminéralisée q.s.p. | 100g | 100g | 100g | 100g | 100g | 100g | 100g |
| **Nuance observée** | gris violet intense | rouge intense | brun rouge ntense | gris intense | rouge intense | brun rouge intense | rouge intense |

| | | | |
|---|---|---|---|
| N2-[2-(dimethylamino)ethyl]-7,8-dihydro-6H-cyclopenta[e]pyrazolo[1,5-a]pyridine-2,3-diamine chlorhydrate | 10⁻³ mole | 10⁻³ mole | 10⁻³ mole |
| 5-Amino-2-methyl-phenol | 10⁻³ mole | | |
| 2-(2,4-Diamino-phenoxy)-ethanol,chlorhydrate | | 10⁻³ mole | |
| 3-Amino-2-chloro-6-methyl-phenol,chlorhydrate | | | 10⁻³ mole |
| Support de teinture (1) | (*) | (*) | (*) |
| Eau déminéralisée q.s.p. | 100g | 100g | 100g |
| **Nuance observée** | vert-bleu | vert | vert-bleu |

| | | | | | | | |
|---|---|---|---|---|---|---|---|
| N2-[2-(dimethylamino)ethyl]-7,8-dihydro-6H-cyclopenta[e]pyrazolo[ 1,5-a]pyridine-2,3-diamine chlorhydrate | 10⁻³ mole | 10⁻³ mole | 10⁻³ mole | 10⁻³ mole | 10⁻³ mole | 10⁻³ mole | 10⁻³ mole |
| Benzene-1,3-diol | 10⁻³ mole | | | | | | |
| 5-Amino-2-methyl-phenol | | 10⁻³ mole | | | | | |
| 1H-Indol-ol | | | 10⁻³ mole | | | | |
| 2-Amino-pyridin-3-ol | | | | 10⁻³ mole | | | |
| 3,6-Dimethyl-1H-pyrazolo[5,1-c][1,2 ,4]triazole | | | | | 10⁻³ mole | | |
| 2-(2,4-Diamino-phenoxy)-ethanol,chlorhydrate | | | | | | 10⁻³ mole | |
| 3-Amino-2-chloro-6-methyl-phenol,chlorhydrate | | | | | | | 10⁻³ mole |
| Support de teinture (2) | (*) | (*) | (*) | (*) | (*) | (*) | (*) |
| Eau déminéralisée q.s.p. | 100g | 100g | 100g | 100g | 100g | 100g | 100g |
| **Nuance observée** | vert chromatique | vert | vert | vert chromatique | vert-bleu | vert-bleu | vert-bleu intense |

| | | | | |
|---|---|---|---|---|
| N2-[2-(diethylamino)ethyl]pyrazolo[1,5-a]pyridine-2,3-diamine chlorhydrate | 10⁻³ mole | 10⁻³ mole | 10⁻³ mole | 10⁻³ mole |
| 5-Amino-2-methyl-phenol | 10⁻³ mole | | | |
| 2-Amino-pyridin-3-ol | | 10⁻³ mole | | |
| 2-(2,4-Diamino-phenoxy)-ethanol,chlorhydrate | | | 10⁻³ mole | |
| 3-Amino-2-chloro-6-methyl-phenol,chlorhydrate | | | | 10⁻³ mole |
| Support de teinture (2) | (*) | (*) | (*) | (*) |
| Eau déminéralisée q.s.p. | 100g | 100g | 100g | 100g |
| **Nuance observée** | violet-bleu | gris | bleu intense | violet-bleu intense |

| | | | | |
|---|---|---|---|---|
| N2-[2-(diethylamino)ethyl]pyrazolo[1,5-a]pyridine-2,3-diamine chlorhydrate | 10⁻³ mole | 10⁻³ mole | 10⁻³ mole | 10⁻³ mole |
| 5-Amino-2-methyl-phenol | 10⁻³ mole | | | |
| 1H-Indol-6-ol | | 10⁻³ mole | | |
| 2-(2,4-Diamino-phenoxy)-ethanol,chlorhydrate | | | 10⁻³ mole | |
| 3-Amino-2-chloro-6-methyl-phenol,chlorhydrate | | | | 10⁻³ mole |
| Support de teinture (1) | (*) | (*) | (*) | (*) |
| Eau déminéralisée q.s.p. | 100g | 100g | 100g | 100g |
| **Nuance observée** | violet-bleu intense | brun rouge | bleu intense | violet-bleu intense |

| | | | | | |
|---|---|---|---|---|---|
| N2-[2-(diisopropylamino)ethyl]pyrazoloo[1,5-a]pyridine-2,3-diamine chlorhydrate | 10⁻³ mole | 10⁻³ mole | 10⁻³ mole | 10⁻³ mole | 10⁻³ mole |
| 5-Amino-2-methyl-phenol | 10⁻³ mole | | | | |
| 1H-Indol-6-ol | | 10⁻³ mole | | | |
| 3,6-Dimethyl-1H-pyrazolo[5,1-c][1,2 ,4]thiazole | | | 10⁻³ mole | | |
| 2-(2,4-Diamino-phenoxy)-ethanol,chlorhydrate | | | | 10⁻³ mole | |
| 3-Amino-2-chloro-6-methyl-phenol,chlorhydrate | | | | | 10⁻³ mole |
| Support de teinture (2) | (*) | (*) | (*) | (*) | (*) |
| Eau déminéralisée q.s.p. | 100g | 100g | 100g | 100g | 100g |
| **Nuance observée** | violet-bleu intense | brun orangé | rouge | bleu intense | violet-bleu intense |

| | | | | | | |
|---|---|---|---|---|---|---|
| N2-[2-(diisopropylamino)ethyl]pyrazolo[1,5-a]pyridine-2,3-diamine chlorhydrate | 10⁻³ - mole | 10⁻³ mole | 10⁻³ mole | 10⁻³ mole | 10⁻³ mole | 10⁻³ mole |
| Benzene-1,3-diol | 10⁻³ mole | | | | | |
| 5-Amina-2-methyl-phenol | | 10⁻³ mole | | | | |
| 1H-indol-6-ol | | | 10⁻³ mole | | | |
| 3,6-Dimethyl-1H-pyrazolo[5,1-c][1,2 ,4]triazole | | | | 10⁻³ mole | | |
| 2-(2,4-Diamino-phenoxy)-ethanol,chlorhydrate | | | | | 10⁻³ mole | |
| 3-Amino-2-chloro-6-methyl-phenol,chlorhydrate | | | | | | 10⁻³ mole |
| Support de teinture (1) | (*) | (*) | (*) | (*) | (*) | (*) |
| Eau déminéralisée q.s.p. | 100g | 100g | 100g | 100g | 100g | 100g |
| **Nuance observée** | brun | violet intense | brun rouge intense | rouge chromatique | bleu intense | violet-bleu chromatique intense |

| | | | | |
|---|---|---|---|---|
| N2-(2-pyridin-3-ylethyl)pyrazolo[1,5-a]pyridine-2,3-diamine chlorhydrate | 10⁻³ mole | 10⁻³ mole | 10⁻³ mole | 10⁻³ mole |
| 5-Amino-2-methyl-phenol | 10⁻³ mole | | | |
| 1H-Indol-6-ol | | 10⁻³ mole | | |
| 2-(2,4-Diamino-phenoxy)-ethanol,chlorhydrate | | | 10⁻³ mole | |
| 3-Amino-2-chloro-6-methyl-phenol,chlorhydrate | | | | 10⁻³ mole |
| Support de teinture (2) | (*) | (*) | (*) | (*) |
| Eau déminéralisée q.s.p. | 100g | 100g | 100g | 100g |
| **Nuance observée** | gris vert-bleu | brun jaune | vert-bleu intense | bleu intense |

| | | | | | | |
|---|---|---|---|---|---|---|
| N2-(2-pyridin-3-ylethyl)pyrazolo[1,5-a]pyridine-2,3-diamine chlorhydrate | 10⁻³ mole | 10⁻³ mole | 10⁻³ mole | 10⁻³ mole | 10⁻³ mole | 10⁻³ mole |
| Benzene-1,3-diol | 10⁻³ mole | | | | | |
| 5-Amino-2-methyl-phenol | | 10-3 mole | | | | |
| 1H-Indol-6-ol | | | 10⁻³ mole | | | |
| 3,6-Dimethyl-1H-pyrazolo[5,1-c][1,2 ,4]triazole | | | | 10⁻³ mole | | |
| 2-(2,4-Diamino-phenoxy)-ethanol,chlorhydrate | | | | | 10⁻³ mole | |
| 3-Amino-2-chloro-6-methyl-phenol,chlorhydrate | | | | | | 10⁻³ mole |
| Support de teinture (1) | (*) | (*) | (*) | (*) | (*) | (*) |
| Eau déminéralisée q.s.p. | 100g | 100g | 100g | 100g | 100g | 100g |
| **Nuance observée** | gris vert-bleu intense | violet-bleu intense | brun rouge intense | rouge | bleu intense | bleu intense |

| | | | |
|---|---|---|---|
| N2-(2-pyridin-4-ylethyl)pyrazolo[1,5-a]pyridine-2,3-diamine chlorhydrate | 10⁻³ mole | 10⁻³ mole | 10⁻³ mole |
| 5-Amino-2-methyl-phenol | 10⁻³ mole | | |
| 2-(2,4-Diamino-phenoxy)-ethanol,chlorhydrate | | 10⁻³ mole | |
| 3-Amino-2-chloro-6-methyl-phenol,chlorhydrate | | | 10⁻³ mole |
| Support de teinture (2) | (*) | (*) | (*) |
| Eau déminéralisée q.s.p. | 100g | 100g | 100g |
| **Nuance observée** | gris | vert-bleu intense | bleu |

| | | | | | |
|---|---|---|---|---|---|
| N2-(2-pyridin-4-ylethyl)pyrazolo[1,5-a]pyridine-2,3-diamine chlorhydrate | 10⁻³ mole | 10⁻³ mole | 10⁻³ mole | 10⁻³ mole | 10⁻³ mole |
| Benzene-1,3-diol | 10⁻³ mole | | | | |
| 5-Amino-2-methyl-phenol | | 10⁻³ mole | | | |
| 1H-Indol-6-ol | | | 10⁻³ mole | | |
| 2-(2,4-Diamino-phenoxy)-ethanol,chlorhydrate | | | | 10⁻³ mole | |
| 3-Amino-2-chloro-6-methyl-phenol,chlorhydrate | | | | | 10⁻³ mole |
| Support de teinture (1) | (*) | (*) | (*) | (*) | (*) |
| Eau déminéralisée q.s.p. | 100g | 100g | 100g | 100g | 100g |
| **Nuance observée** | gris | violet-bleu intense | brun rouge | vert-bleu intense | bleu |

| | | | | | | |
|---|---|---|---|---|---|---|
| 2-isopropoxypyrazolo[1,5-a]pyridin-3-amine chlorhydrate | 10⁻³ mole | 10⁻³ mole | 10⁻³ mole | 10⁻³ mole | 10⁻³ mole | 10⁻³ mole |
| Benzene-1,3-diol | 10⁻³ mole | | | | | |
| 5-Amino-2-methyl-phenol | | 10⁻³ mole | | | | |
| 1H-Indol-6-ol | | | 10⁻³ mole | | | |
| 2-Amino-pyridin-3-ol | | | | 10⁻³ mole | | |
| 2-(2,4-Diamino-phenoxy)-ethanol,chlorhydrate | | | | | 10⁻³ mole | |
| 3-Amino-2-chloro-6-methyl-phenol,chlorhydrate | | | | | | 10⁻³ mole |
| Support de teinture (2) | (*) | (*) | (*) | (*) | (*) | (*) |
| Eau déminéralisée q.s.p. | 100g | 100g | 100g | 100g | 100g | 100g |
| **Nuance observée** | brun rouge | rouge chromatique intense | rouge | rouge | Rouge intense | rouge chromatique intense |

| | | | | | | |
|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g (*): support de teinture (2) pH 9,5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2.94 g | | | | | | |

Au moment de l'emploi, chaque composition est mélangée avec un poids égal d'eau oxygénée à 20 volumes (6% en poids). On obtient un pH final de 7, resp. 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les résultats obtenus sont reportés dans les tableaux ci-dessus.

## Revendications

1. Composition de teinture des fibres kératiniques comprenant, dans un milieu approprié à la teinture, à titre de base de coloration d'oxydation, un dérivé 3-amino pyrazolo-[1,5-a]-pyridine de formule (I) ainsi que ses sels et solvates: 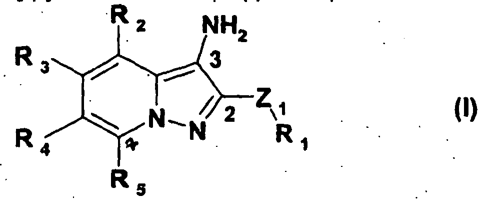 dans laquelle
• Z₁ représente un atome d'oxygène, un groupement NR₆, lorsque Z₁ représente NR₆ alors R₁ et R₆ peuvent former ensemble avec l'atome d'azote auquel ils sont rattachés un hétérocycle saturé, insaturé ou aromatique de 5 à 8 chaînons, éventuellement substitué,
Z₁ peut aussi représenter un radical divalent S, SO, SO₂ lorsque R₁ représente CH₃,
• R₁ est choisi parmi un radical alkyle, un radical alkyle substitué par un hydroxy, un radical alkyle substitué par un radical amino ou (di)alkylamino, un radical alkyle substitué par un hétérocycle azoté,
• R₆ peut être choisi parmi un atome d'hydrogène, un radical alkyle ou un radical alkyle substitué par un radical hydroxy, un radical amino, un radical alkylamino, un radical dialkyle amino, un radical alkyle substitué par un hétérocycle azoté,
• R₂, R₃, R₄, R₅, indépendamment, représentent :
■ un atome d'hydrogène,
■ un radical alkyle en C₁-C₄ éventuellement substitué,
• R₂, R₃, R₄, R₅, peuvent former deux à deux avec des radicaux adjacents un (hétéro)cycle saturé ou insaturé, éventuellement substitué,
à l'exception du 2-méthoxy-pyrazolo[1.5-a]pyridine-3-ylamine et du 2-morpholino-pyrazolo[1,5-a]pyridine-3-ylamine respectivement de formules suivantes :

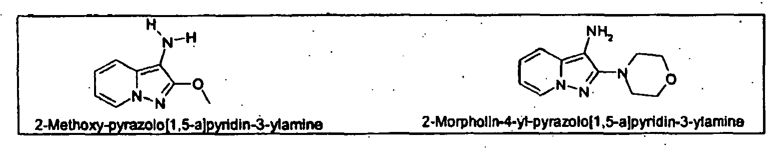

2. Composition selon la revendication 1 dans laquelle Z₁ représente un atome d'oxygène, un radical NR₆ ou un radical NR₆ formant avec R₁ un hétérocycle.

3. Composition selon la revendication 1 ou 2 dans laquelle le radical alkyle de R1 ou R6 comprend de 1 à 6 atomes de carbone.

4. Composition selon la revendication 1 dans laquelle. R₆ forme avec R₁ un hétérocycle avec l'atome d'azote auquel ils sont rattachés, alors l'hétérocycle peut être choisi parmi les imidazoles, les pipérazines, les pyrrolidines, les diazépanes, ces hétérocycles pouvant être substitués ou non.

5. Composition selon l'une quelconque des revendications précédentes dans laquelle les radicaux R₄ et R₅ forment ensemble un (hétéro)cycle saturé ou insaturé de 5 à 8 chaînons.

6. Composition selon la revendication 5 dans laquelle R4 et R5 forment ensemble un radical cyclopentane.

7. Composition selon l'une quelconque des revendications 1 à 6 dans laquelle le composé de formule (I) est choisi parmi les composés ci-dessous et les sels d'addition ou solvates correspondants :

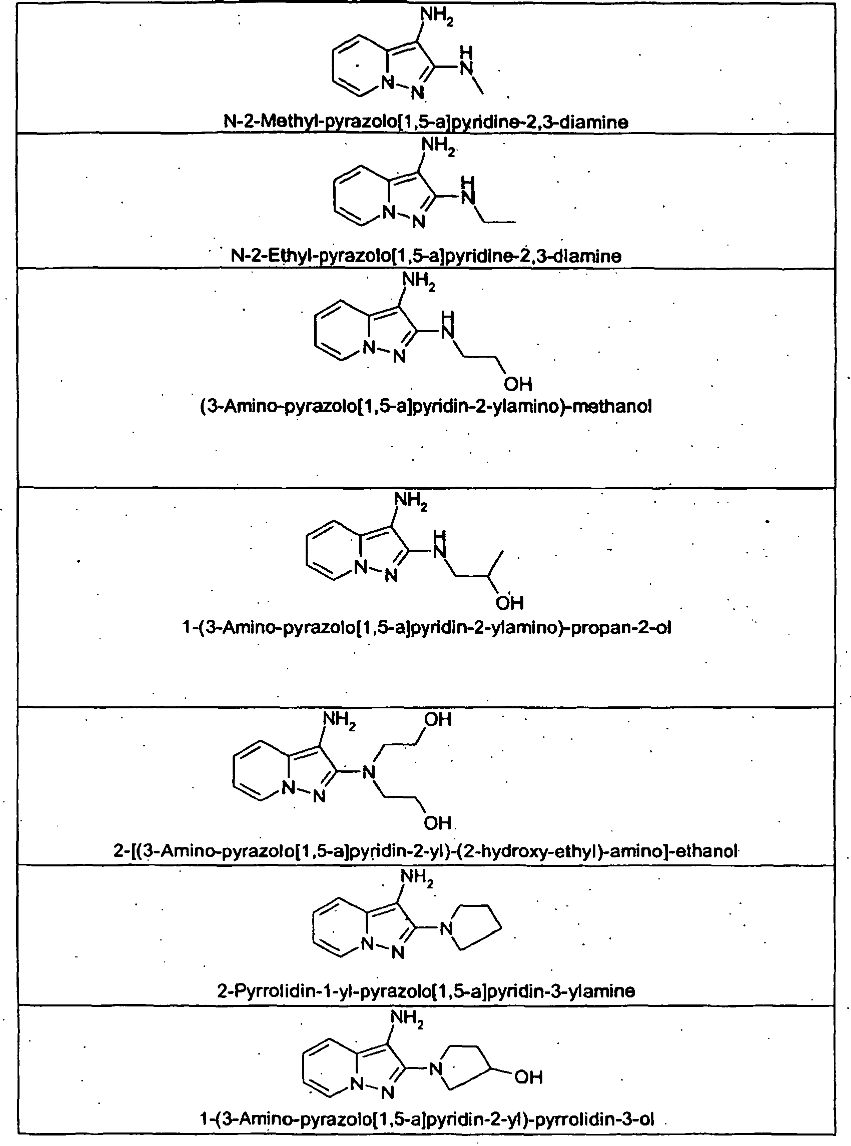

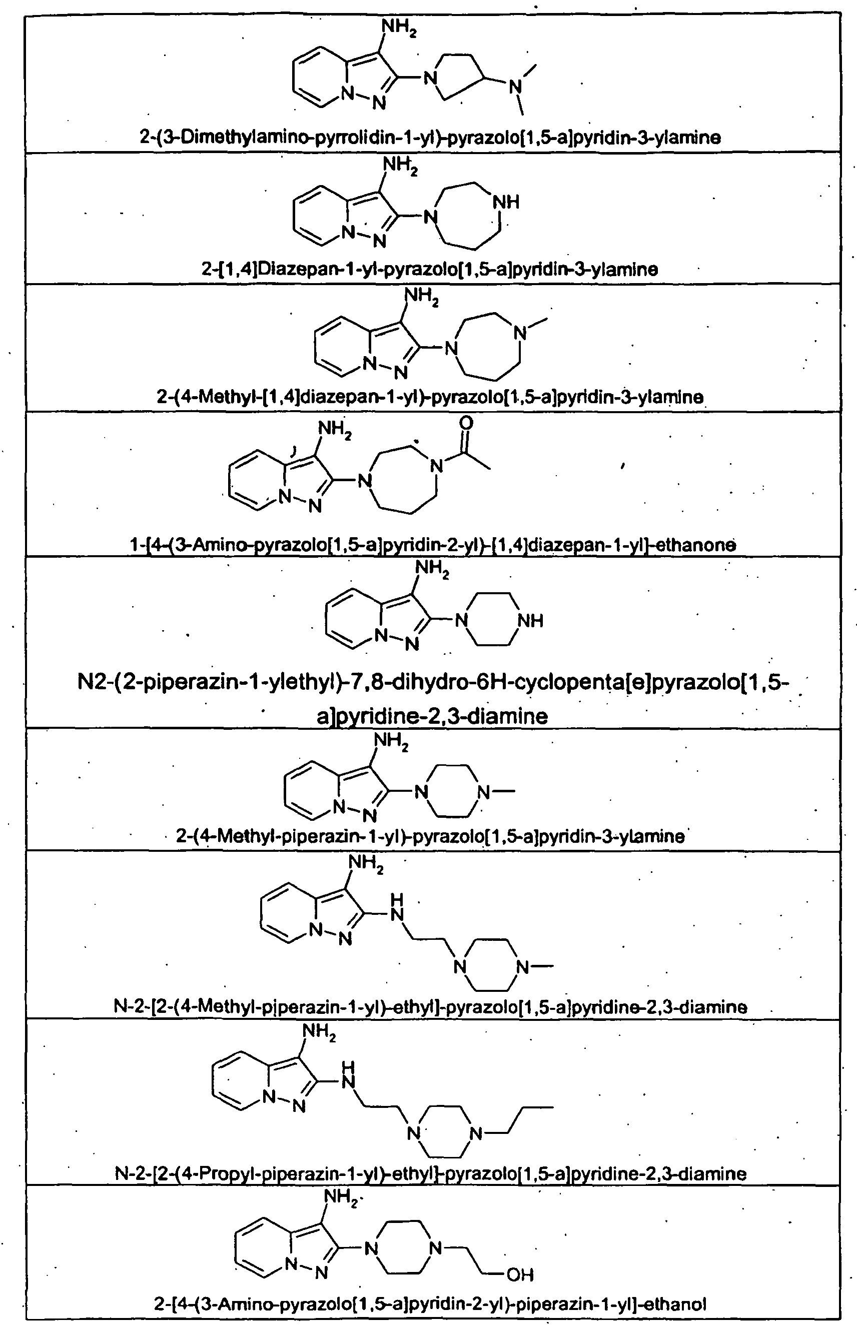

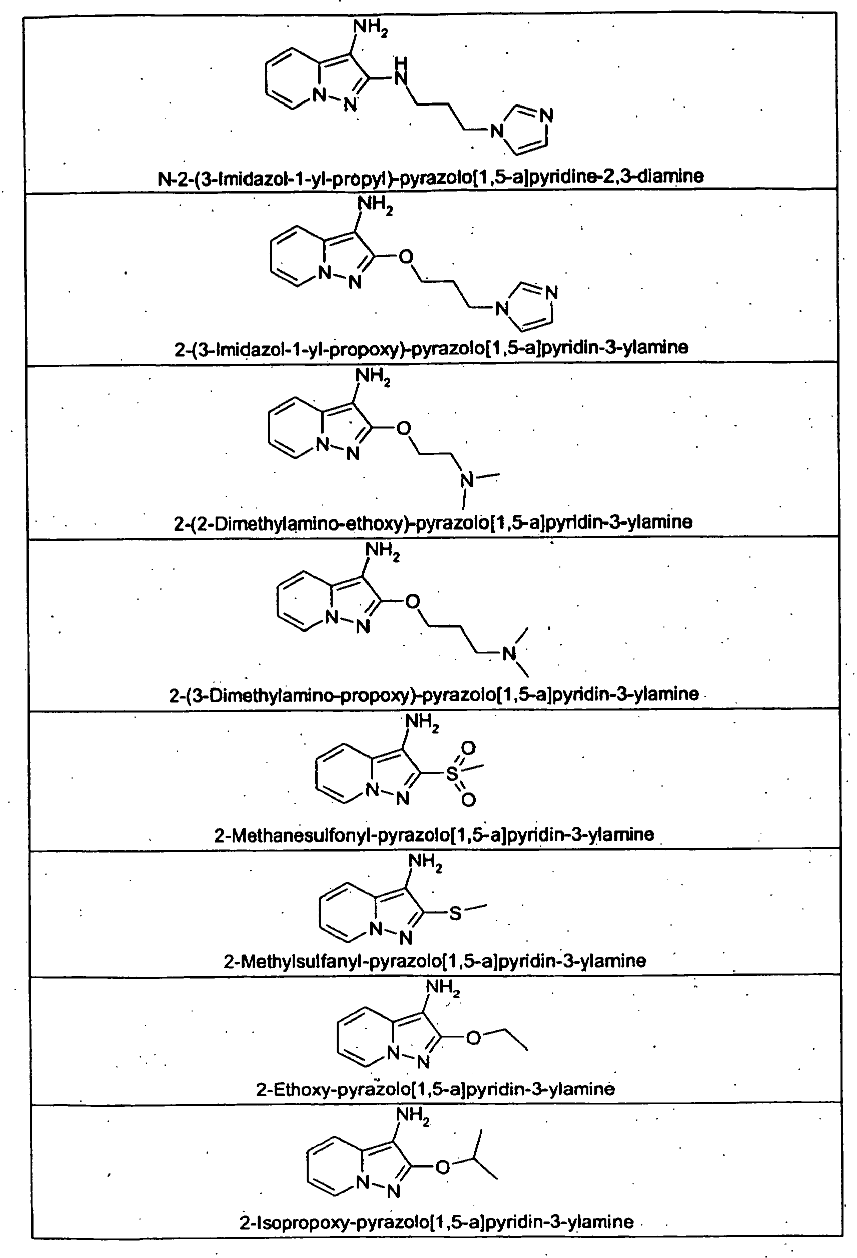

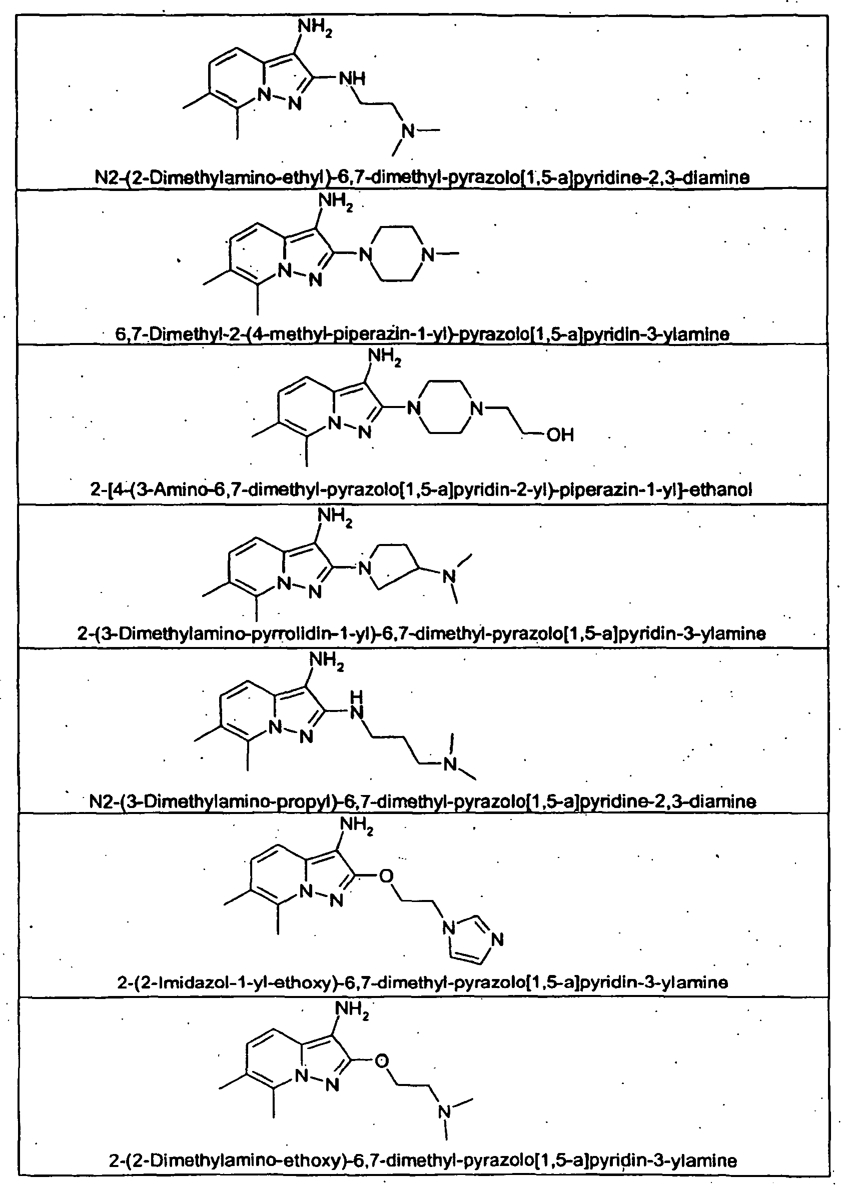

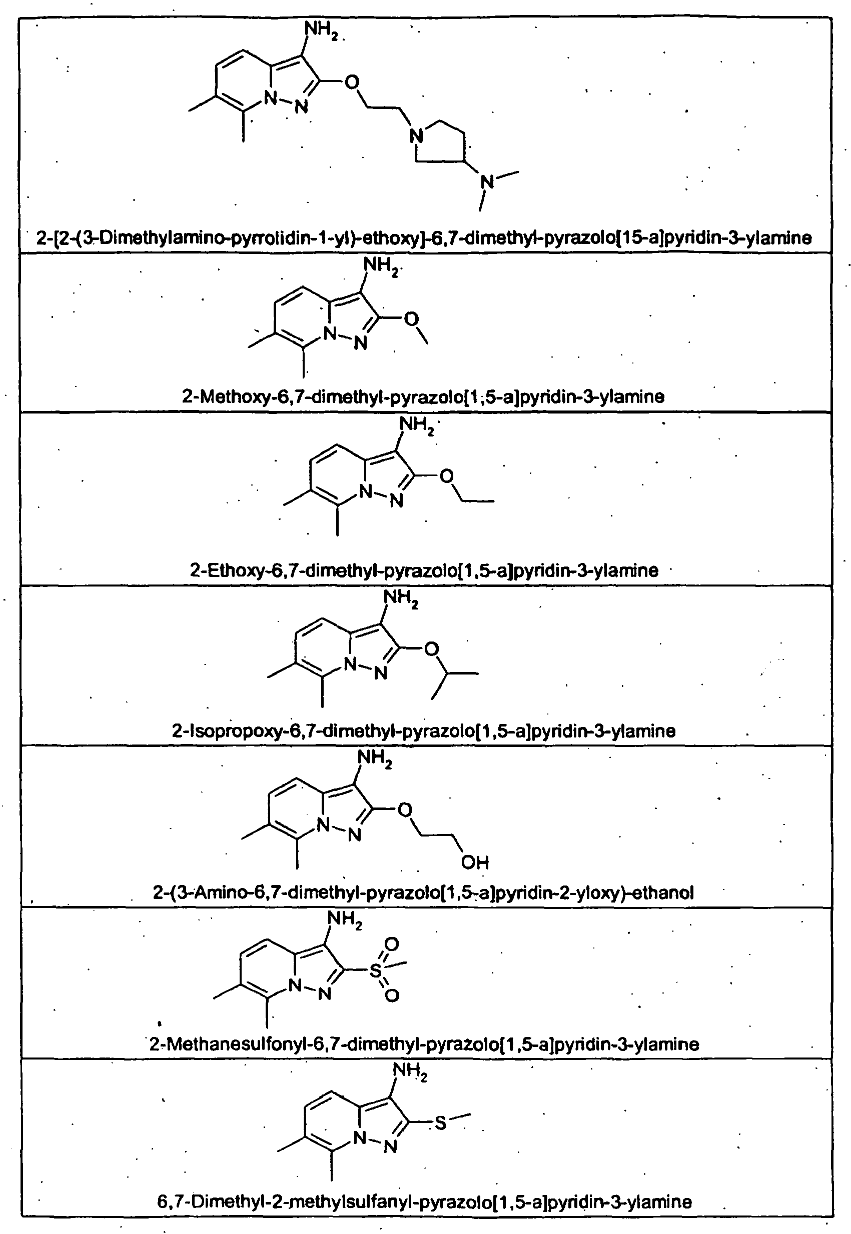

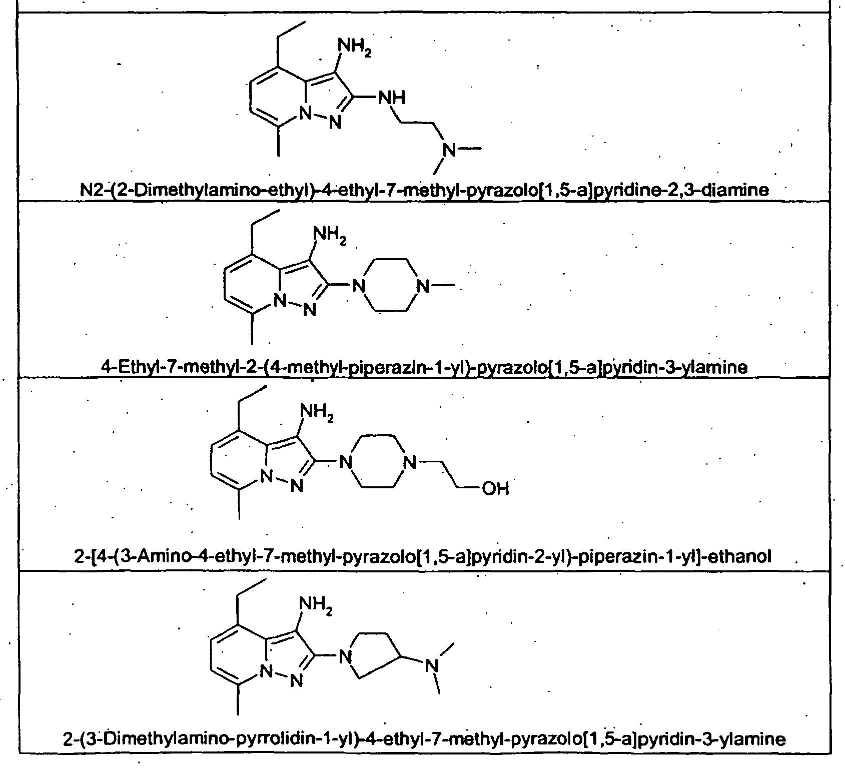

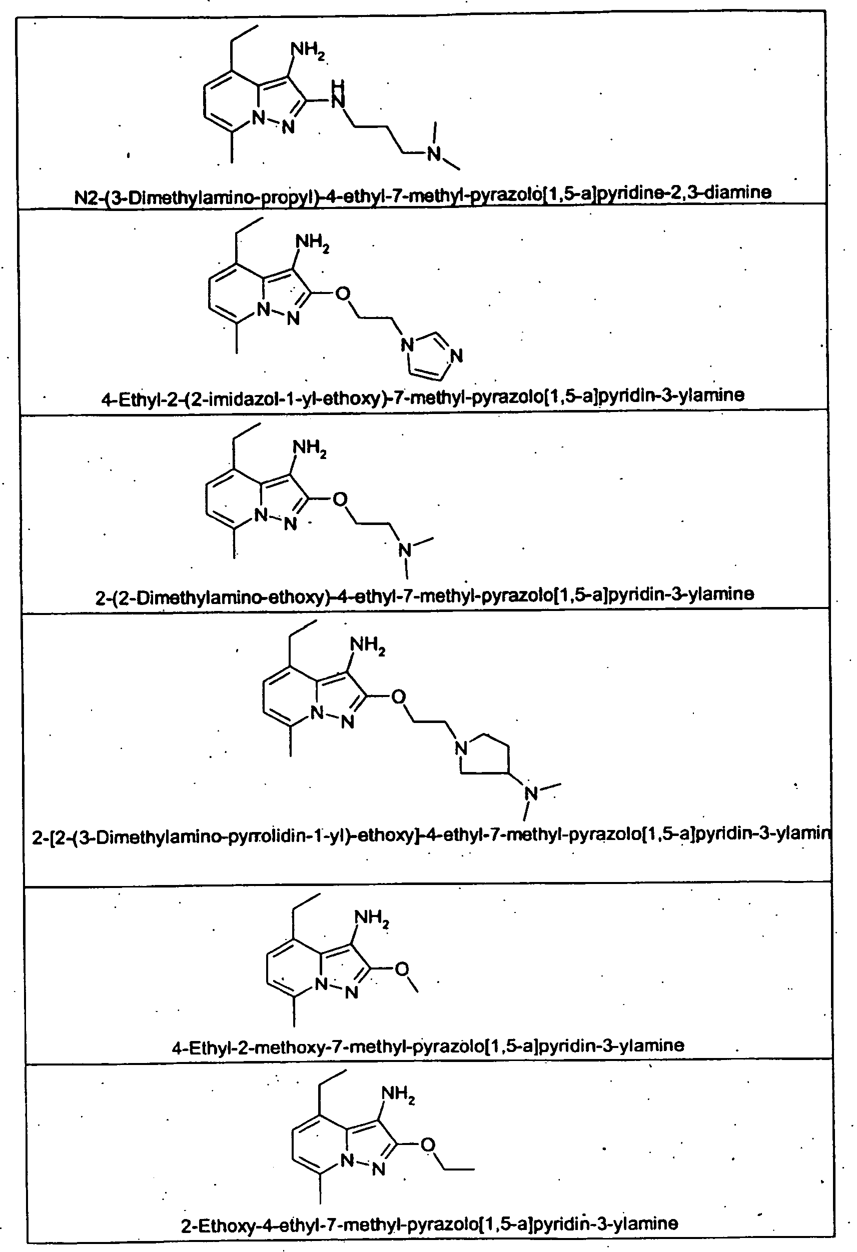

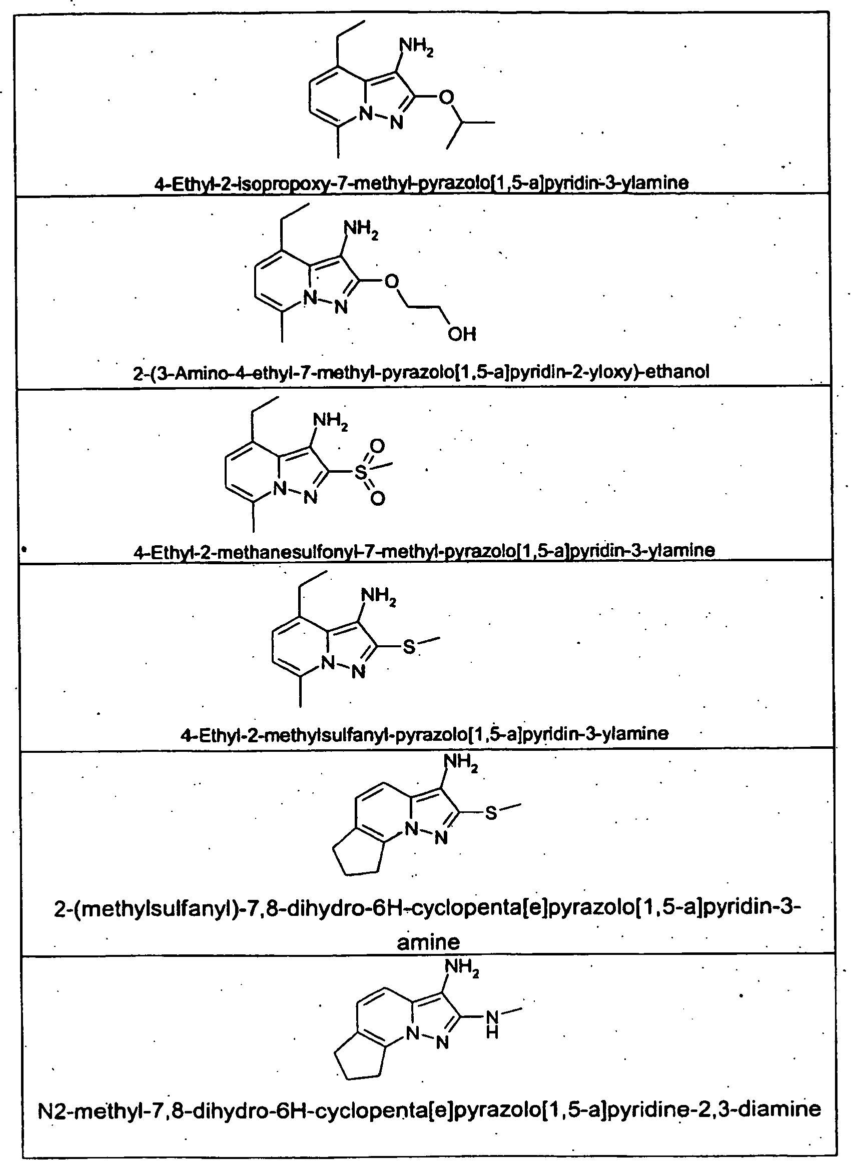

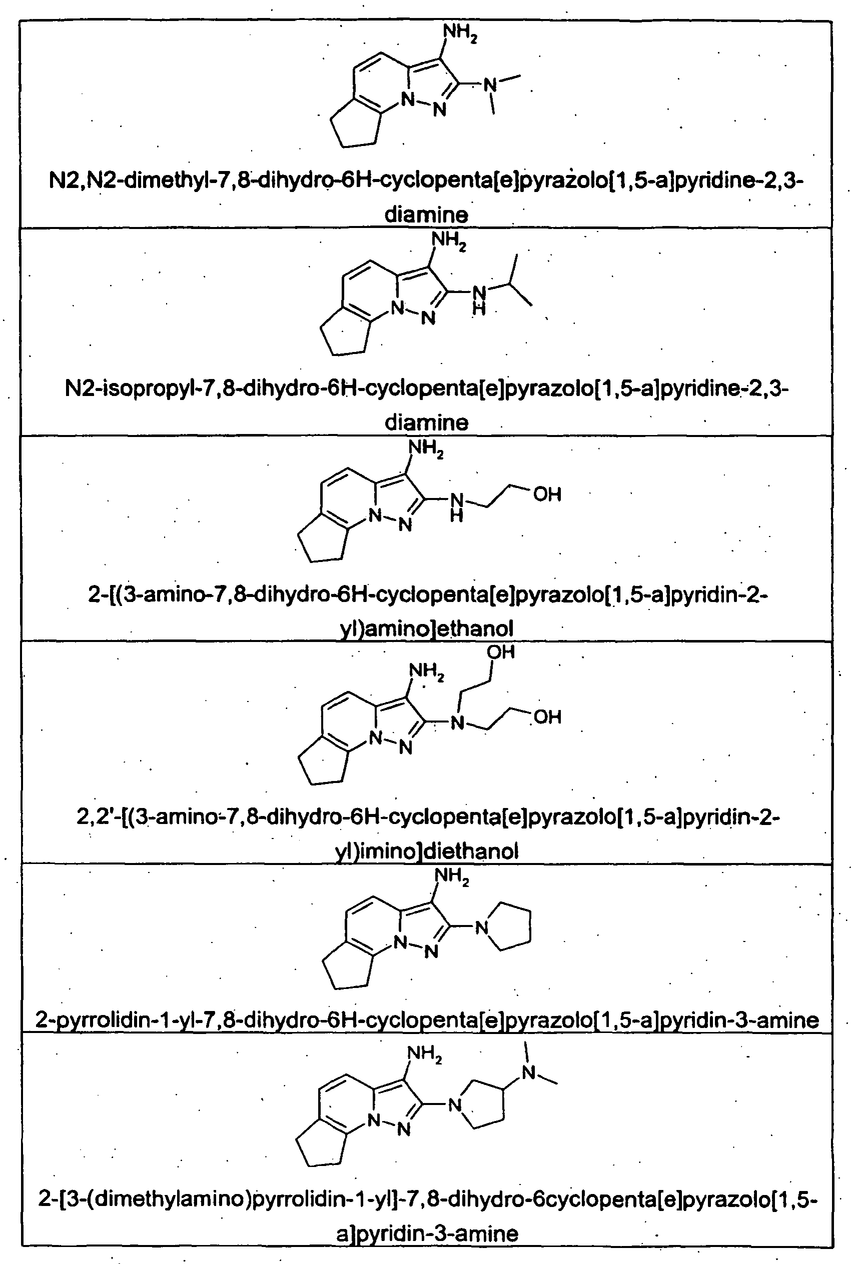

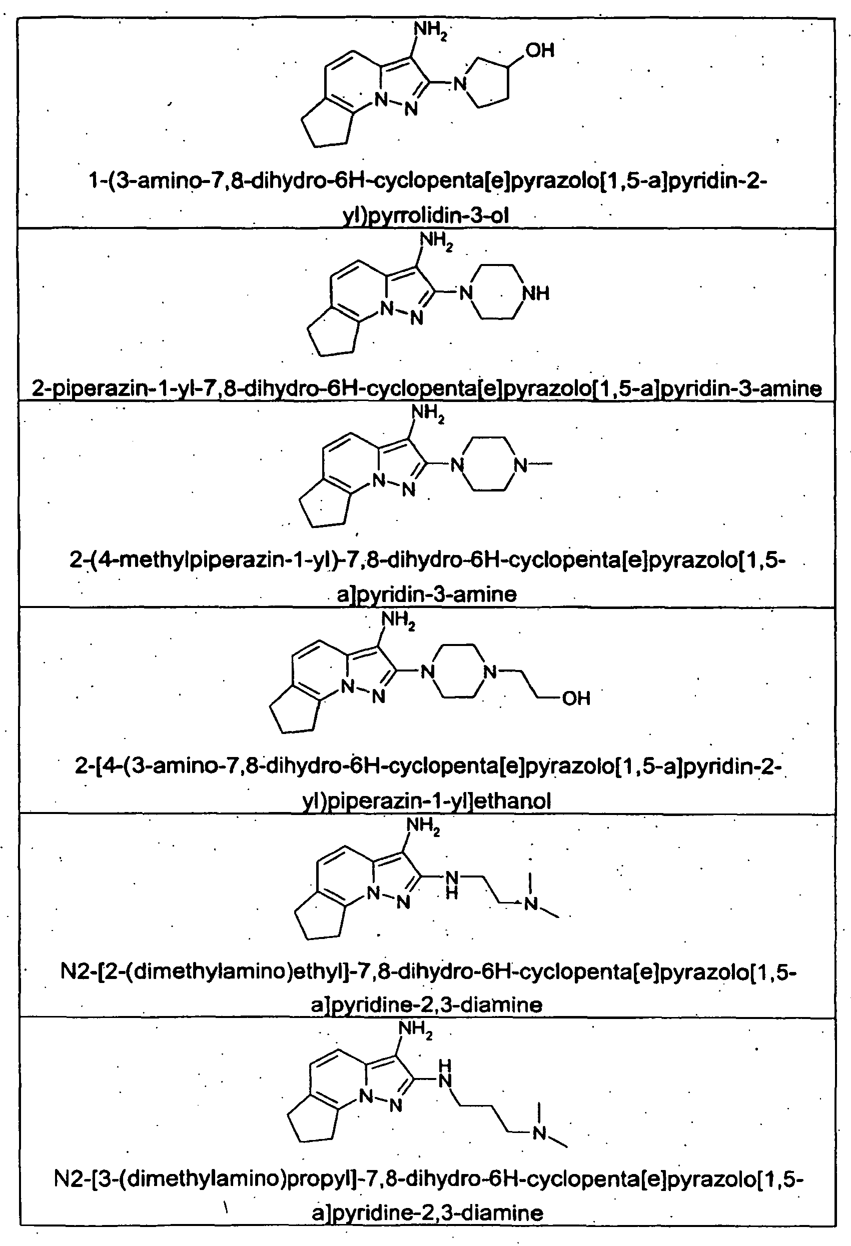

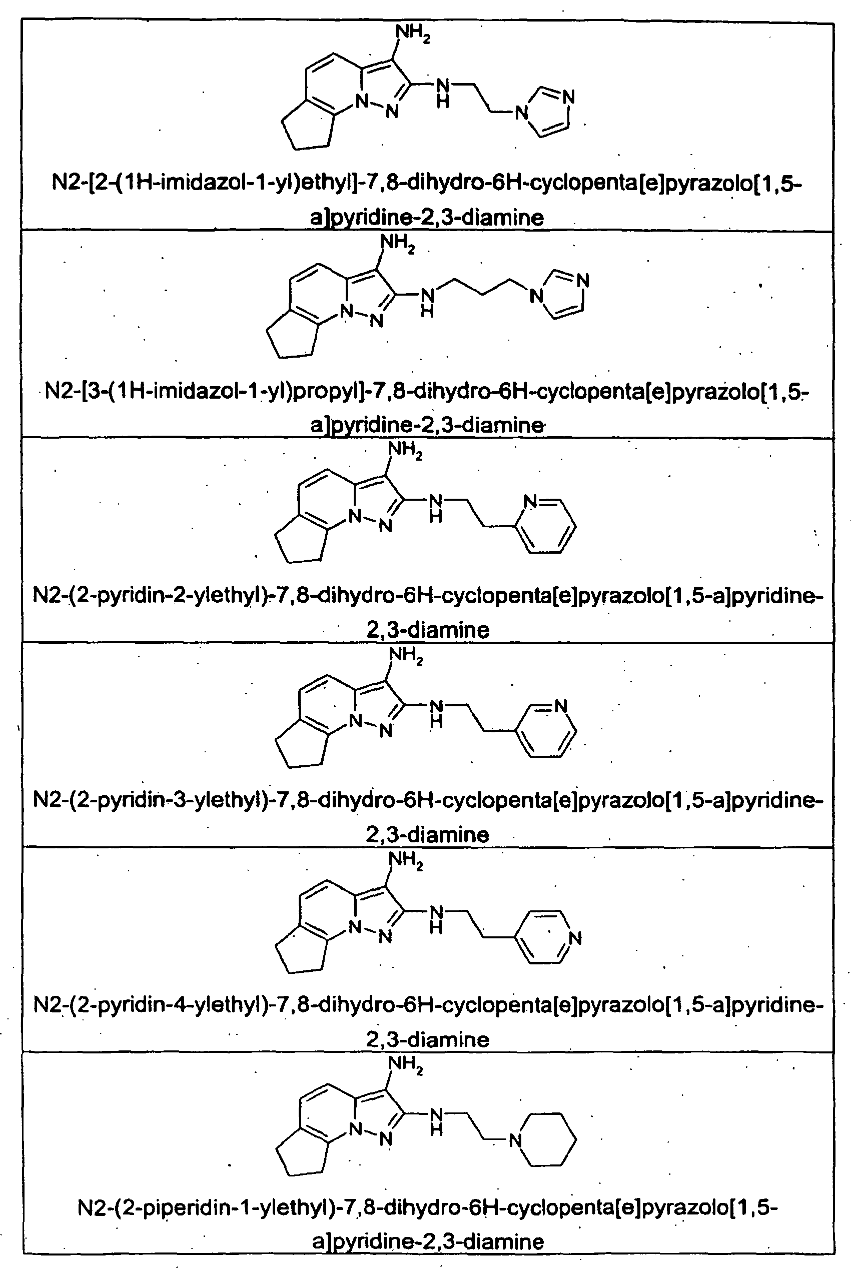

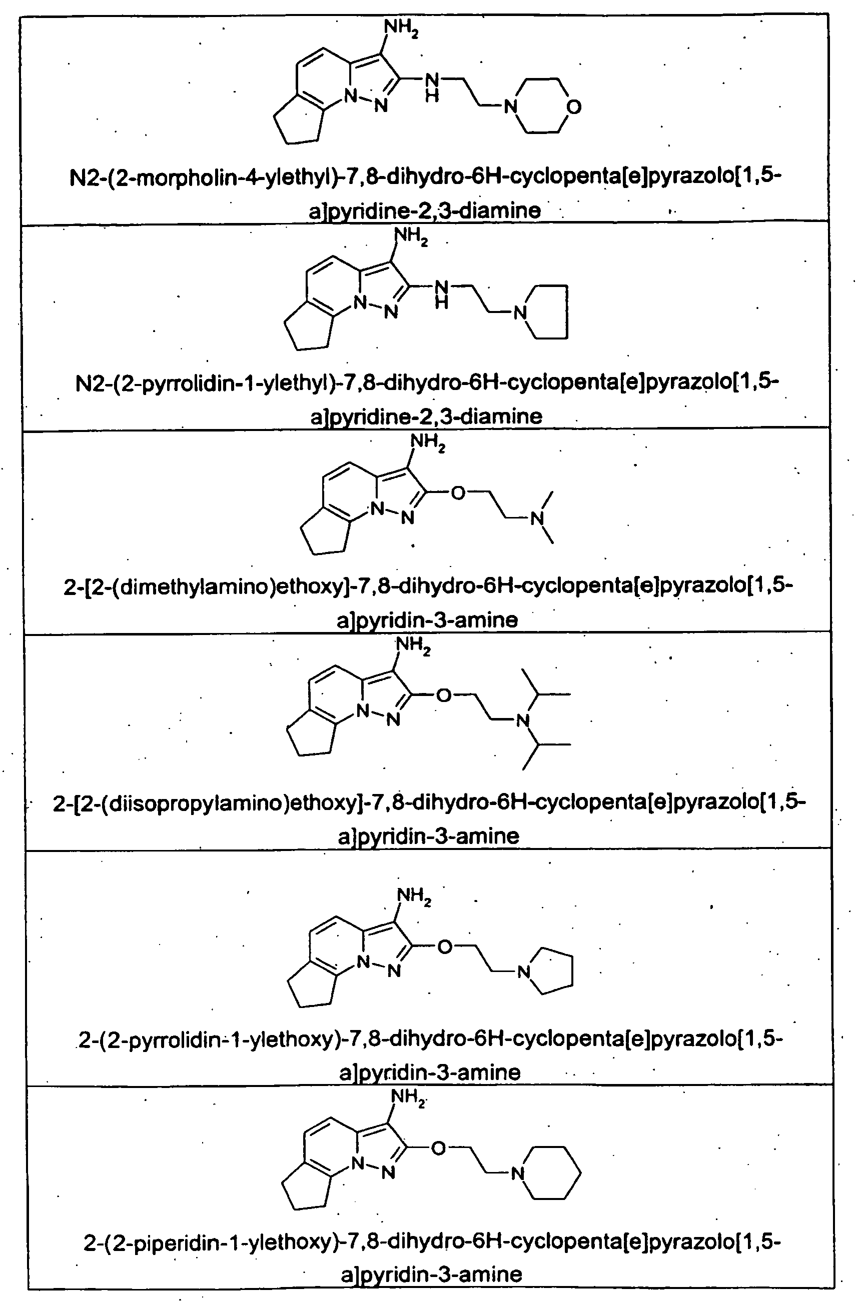

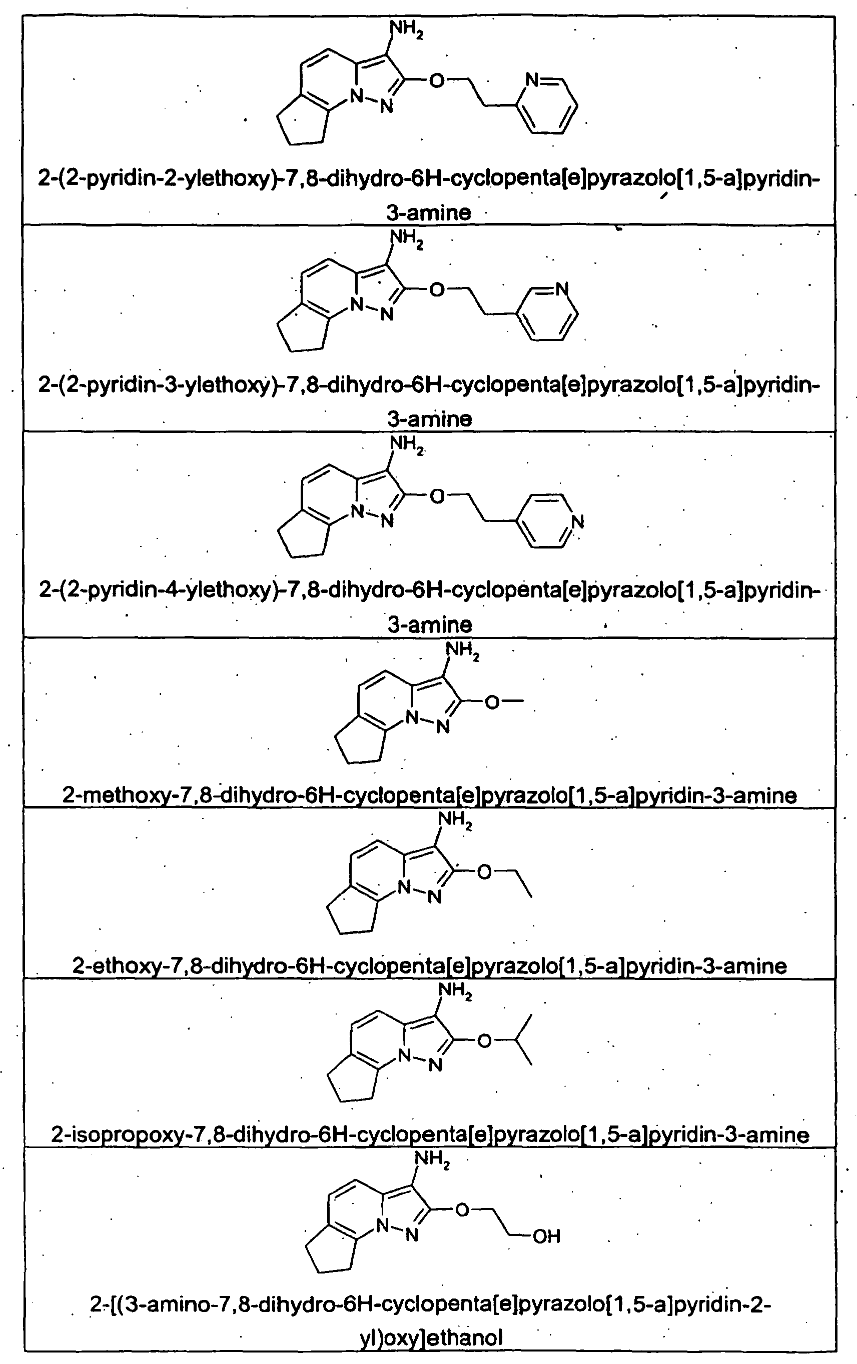

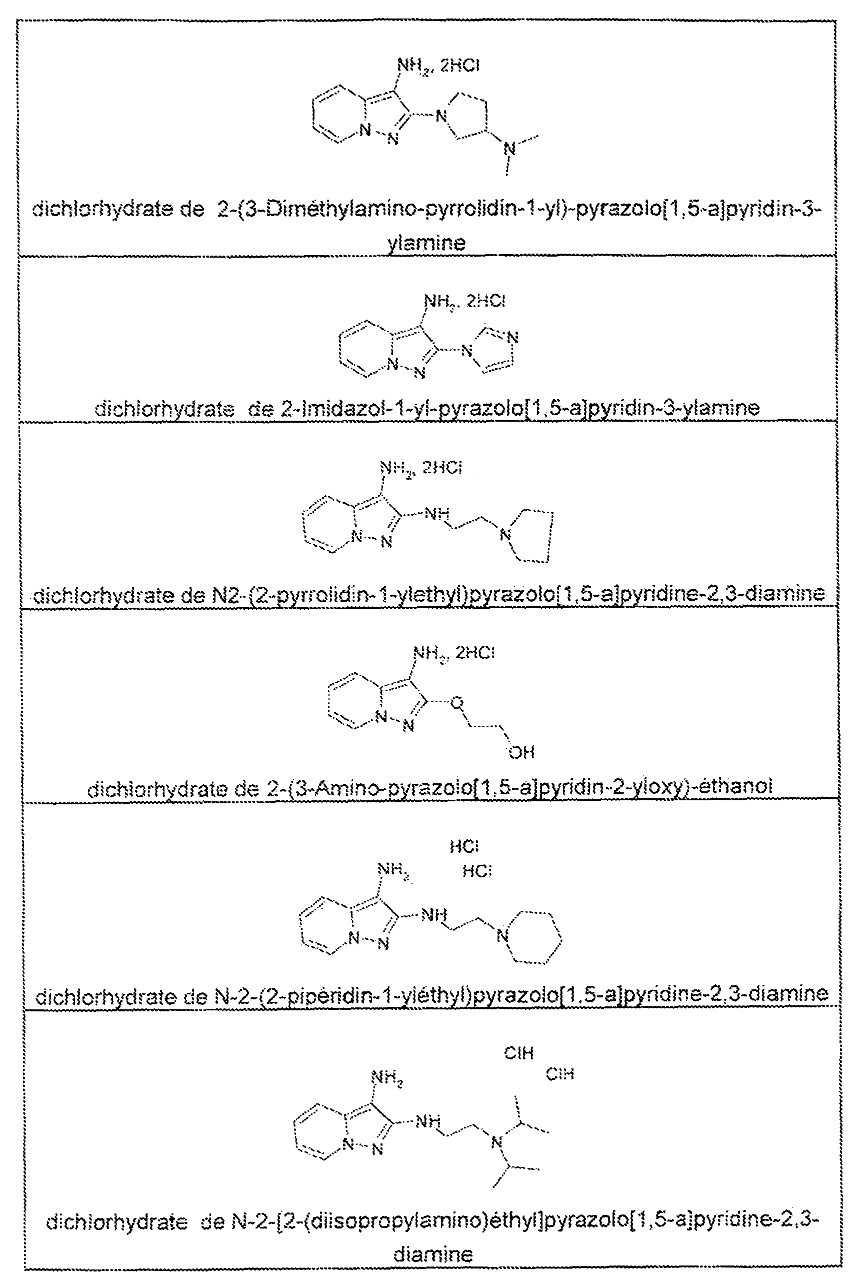

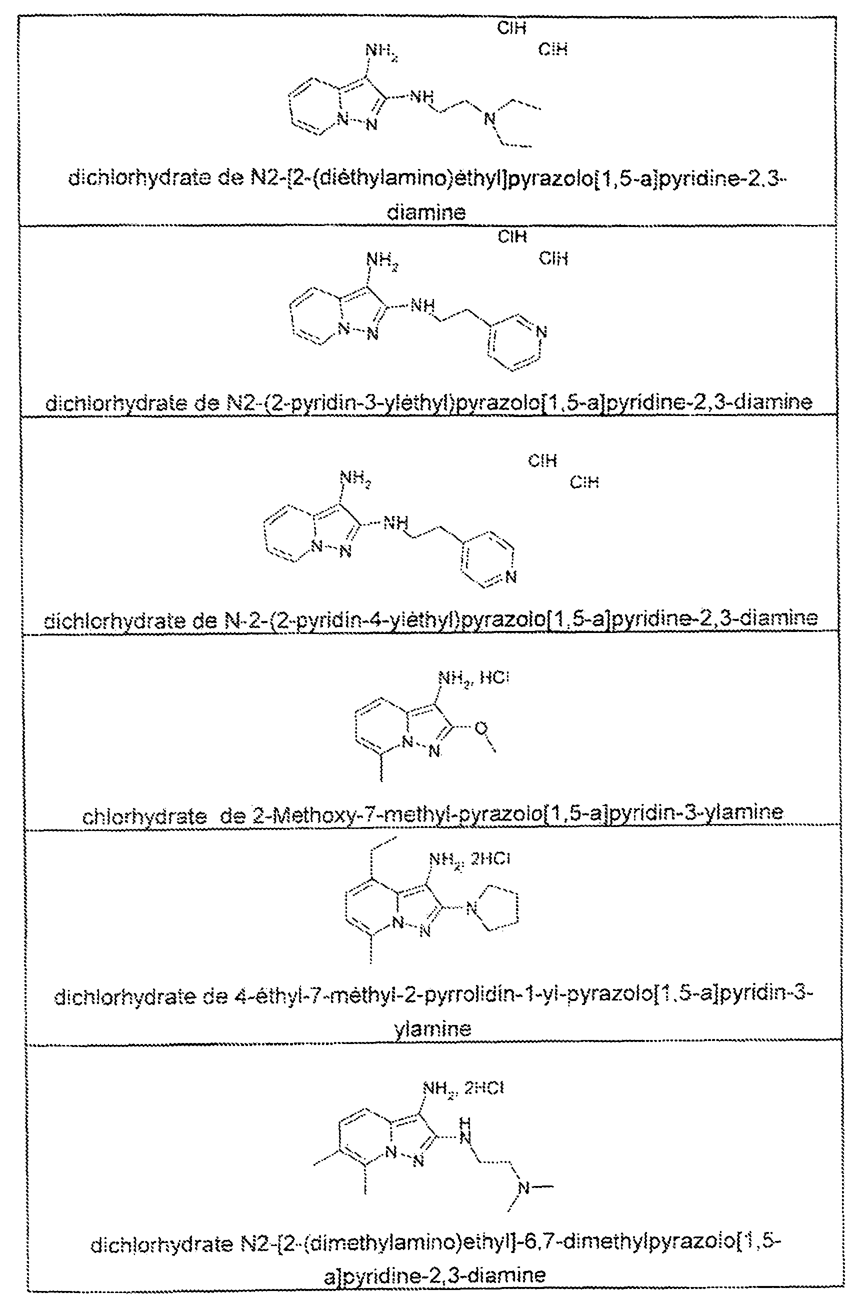

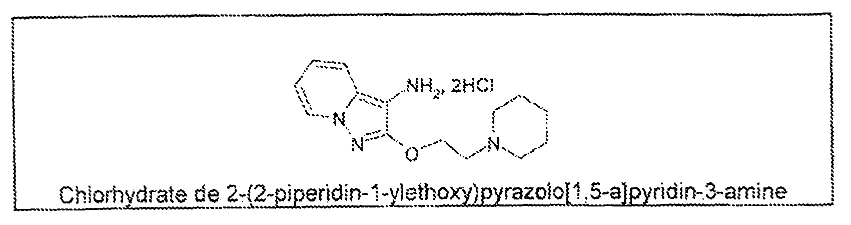

8. Composition selon la revendication 7 dans laquelle le composé de formule (I) est choisi parmi :
le dichlorhydrate de N2-éthylpyrazolo[1,5-a]pyridine-2,3-diamine,
le dichlorydrate de 2-(3-Amino-pyrazolo[1,5-a]pyridin-2-ylamino)-éthanol,
le dichlorhydrate de 1-(3-Amino-pyrazolo[1,5-a]pyridin-2-ylamino)-propan-2-ol,
le dichlorhydrate de N-2-(3-Imidazol-1-yl-propyl)-pyrazolo[1,5-a]pyridine-2,3-diamine,
le dichlorhydrate de 2-Pyrrolidin-1-yl-pyrazolo[1,5-a]pyridin-3-ylamine,
le dichlorhydrate de 2-(3-Diméthylamino-pyrrolidin-1-yl)-pyrazolo[1,5-a]pyridin-3-ylamine,
le dichlorhydrate de 2-Imidazol-1-yl-pyrazolo[1,5-a]pyridin-3-ylamine,
le dichlorhydrate de N2-(2-pyrrolidin-1-yléthyl)pyrazolo[1,5-a]pyridine-2,3-diamine,
le dichlorhydrate de 2-(3-Amino-pyrazolo[1,5-a]pyridin-2-yloxy)-éthanol,
le dichlorhydrate de 2-éthoxy--pyrazolo[1,5-a]pyridin-3-ylamine
le chlorhydrate de 2-Méthoxy-7-méthyl-pyrazolo[1,5-a]pyridin-3-ylamine,
le dichlorhydrate de 4-éthyl-2-méthoxy-7-methyl-pyrazolo[1,5-a]pyridin-3-ylamine,
le dichlorhydrate de 4-éthyl-7-methyl-2-pyrrolidin-1-yl-pyrazolo[1,5-a]pyridin-3-ylamine,
le dichlorhydrate de N2-(2-pipéridin-1-ylethyl)pyrazolo[1,5-a]pyridine-2,3-diamine,
le dichlorhydrate de N2-[2-(diisopropylamino)éthyl]pyrazolo[1,5-a]pyridine-2,3-diamine,
le dichlorhydrate de N2-[2-(diéthylamino)ethyl]pyrazolo[1,5-a]pyridine-2,3-diamine,
le dichlorhydrate de N2-(2-pyridin-3-yléthyl)pyrazolo[1,5-a]pyridine-2,3-diamine,
le dichlorhydrate de N2-(2-pyridin-4-yléthyl)pyrazolo[1,5-a]pyridine-2,3-diamine,
le dichlorhydrate de N2-(2-pyridin-2-yléthyl)pyrazolo[1,5-a]pyridine-2,3-diamine diamine,
le N2-(2-pipérazin-1-ylethyl)pyrazolo[1,5-a]pyridine-2,3-diamine,
le dichlorhydrate de N2-[2-(4-méthylpiperazin-1-yl)éthyl]pyrazolo[1,5-a]pyridine-2,3-diamine,
le dichlorhydrate de N2-(2-morpholin-4-yléthyl)pyrazolo[1,5-a]pyridine-2,3-diamine,
le dichlorhydrate de N2-[2-(diméthylamino)éthyl]-7,8-dihydro-6H-cyclopenta[e]pyrazolo[1,5-a]pyridine-2,3-diamine,
le chlorhydrate de 2-[(3-amino-7,8-dihydro-6H-cyclopenta[e]pyrazolo[1,5-a]pyridin-2-yl)amino]éthanol,
le chlorhydrate de 2-méthoxy-7,8-dihydro-6H-cyclopenta[e]pyrazolo[1,5-a]pyridin-3-amine,
le chlorhydrate de 2-isopropoxy-7,8-dihydro-6H-cyclopenta[e]pyrazolo[1,5-a]pyridin-3-amine,
le dichlorhydrate de 2-[2-(diméthylamino)ethoxy]pyrazolo[1,5-a]pyridin-3-amine,
le dichlorhydrate de 2-(2-pyrrolidin-1-ylethoxy)pyrazolo[1,5-a]pyridin-3-amine,
le dichlorhydrate de 2-(2-piperidin-1-yléthoxy)pyrazolo[1,5-a]pyridin-3-amine,
le dichlorhydrate de 2-(-morpholin-4-yléthoxy)pyrazolo[1,5-a]pyridin-3-amine,
le dichlohrydrate de 2-[2-(diisopropylamino)éthoxy]pyrazolo[1,5-a]pyridin-3-amine,
le dichlorhydrate de 2-(2-pyridin-2-yléthoxy)pyrazolo[1,5-a]pyridin-3-amine,
le dichlorhydrate de 2-(2-pyridin-3-yléthoxy)pyrazolo[1,5-a]pyridin-3-amine,
le dichlorhydrate de 2-(2-pyridin-4-yléthoxy)pyrazolo[1,5-a]pyridin-3-amine,

9. Composition selon l'une quelconque de revendications précédentes dans laquelle la quantité de chacune des bases d'oxydation de formule (I) est comprise entre 0,001 à 10 % en poids environ du poids total de la composition tinctoriale.

10. Composition selon l'une quelconque des revendications précédentes comprenant de plus un coupleur choisi parmi les méta-phénylènediamines, les méta-aminophénols, les méta-diphénols, les coupleurs naphtaléniques, les coupleurs hétérocycliques et leurs sels d'addition, ainsi que leurs mélanges.

11. Composition selon la revendication 10 dans laquelle la quantité de chacun des coupleurs est comprise entre 0,001 et 10 % en poids du poids total de la composition tinctoriale.

12. Procédé de teinture des fibres kératiniques, **caractérisé en ce qu'**une composition telle que définie dans l'une quelconque des revendications 1 à 11 est appliquée sur les fibres kératiniques en présence d'un agent oxydant pendant un temps suffisant pour développer la coloration désirée.

13. Procédé selon la revendication 12 dans lequel l'agent oxydant est choisi parmi le peroxyde d'hydrogène, le peroxyde d'urée, les bromates de métaux alcalins, les persels, les peracides et les enzymes oxydases.

14. Dispositif à plusieurs compartiments dans lequel un premier compartiment contient une composition tinctoriale telle que définie dans l'une quelconque des revendications 1 à 11 et un deuxième compartiment contient un agent oxydant.

15. Composés de formule (1) tels que définis selon l'une quelconque des revendications 1 à 11 à l'exception des composés 2-méthylsulfanyl-pyrazolo[1,5-a]pyridin-3-ylamine et 2,3-diamino-pyrazolo[1,5-a]pyridine.

16. Procédé de préparation d'un composé de formule (I) tel que défini dans les compositions selon l'une quelconque des revendications 1 à 8 qui comprend les étapes suivantes : 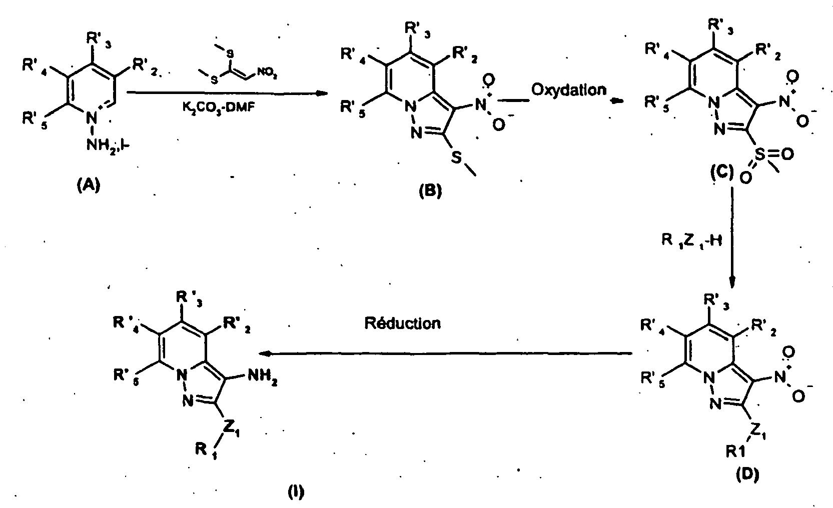 dans lequel R'₂, R'₃, R'₄ et R'₅ ont les mêmes significations que R₂, R₃, R₄ et R₅ ou en sont des précurseurs.

17. Composés intermédiaires de formules intermédiaires (A) et (B) tels que définis ci-dessus dans lesquels au moins un des radicaux R'₂, R'₃, R'₄ ou R'₅ est différent d'un atome d'hydrogène à l'exclusion du composé pour lequel R'₂ = Me lorsque tous les autres substituants sont l'hydrogène

18. Composés intermédiaires de formules (C), (D) tels que définis à la revendication 16.

## Claims

1. Composition for dyeing keratin fibres comprising, in a medium suitable for dyeing, as an oxidation dyeing base, a 3-aminopyrazolo[1,5-a]-pyridine derivative of formula (I) as well as its salts and solvates: 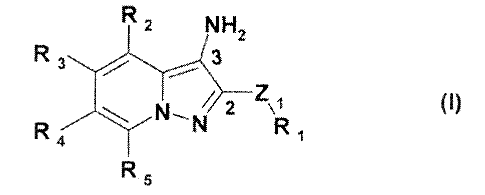 in which
• Z₁ represents an oxygen atom, a group NR₆, when Z₁ represents NR₆ then R₁ and R₆ can form, together with the nitrogen atom to which they are attached, a saturated, unsaturated or aromatic heterocycle with 5 to 8 ring members, optionally substituted, Z₁ can also represent a divalent radical S, SO, SO₂ when R₁ represents CH₃,
• R₁ is selected from an alkyl radical, an alkyl radical substituted with a hydroxy, an alkyl radical substituted with an amino or (di)alkylamino radical, an alkyl radical substituted with a nitrogen-containing heterocycle,
• R₆ can be selected from a hydrogen atom, an alkyl radical or an alkyl radical substituted with a hydroxy radical, an amino radical, an alkylamino radical, a dialkylamino radical, an alkyl radical substituted with a nitrogen-containing heterocycle,
• R₂, R₃, R₄, R₅, independently, represent:
■ a hydrogen atom,
■ a C₁-C₄ alkyl radical, optionally substituted,
• R₂, R₃, R₄, R₅, can form, two by two with adjacent radicals, a saturated or unsaturated (hetero)cycle, optionally substituted,
with the exception of 2-methoxypyrazolo[1,5 -a]pyridin-3-ylamine and 2-morpholino-pyrazolo[1,5-a]pyridin-3-ylamine respectively of the hollowing formulae:

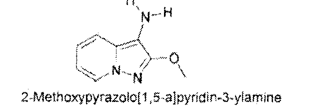

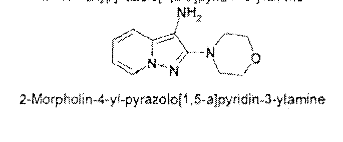

2. Composition according to Claim 1 in which Z₁ represents an oxygen atom, a radical NR₆ or a radical NR₆ forming a heterocycle with R₁.

3. Composition according to Claim 1 or 2 in which the alkyl radical of R₁ or R₆ has from 1 to 6 carbon atoms.

4. Composition according to Claim 1 in which R₆ forms, with R₁, a heterocycle with the nitrogen atom to which they are attached, then the heterocycle can be selected from the imidazoles, piperazines, pyrrolidines, diazepanes, and these heterocycles can be substituted or unsubstituted.

5. Composition according to any one of the preceding claims in which the radicals R₄ and R₅ together form a saturated or unsaturated (hetero)cycle with 5 to 8 ring members.

6. Composition according to Claim 5 in which R₄ and R₅ together form a cyclopentane radical.

7. Composition according to any one of Claims 1 to 6 in which the compound of formula (I) is selected from the compounds listed hereunder and the corresponding salts of addition or solvates:

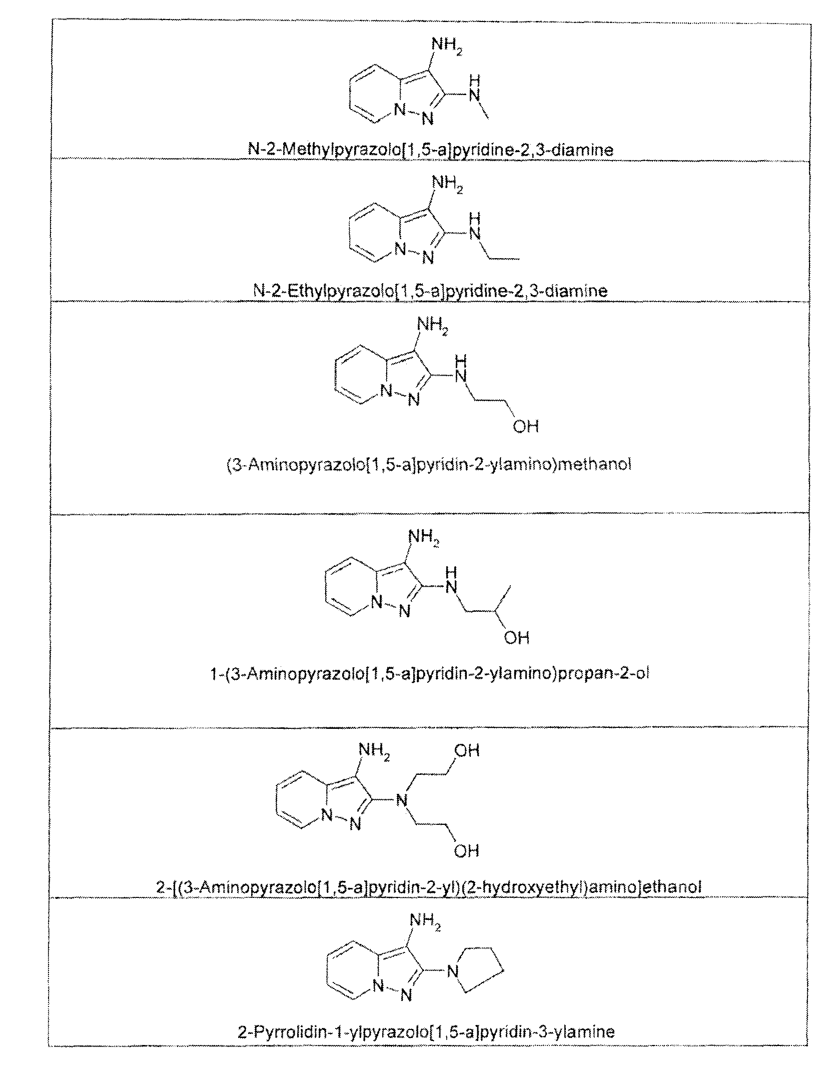

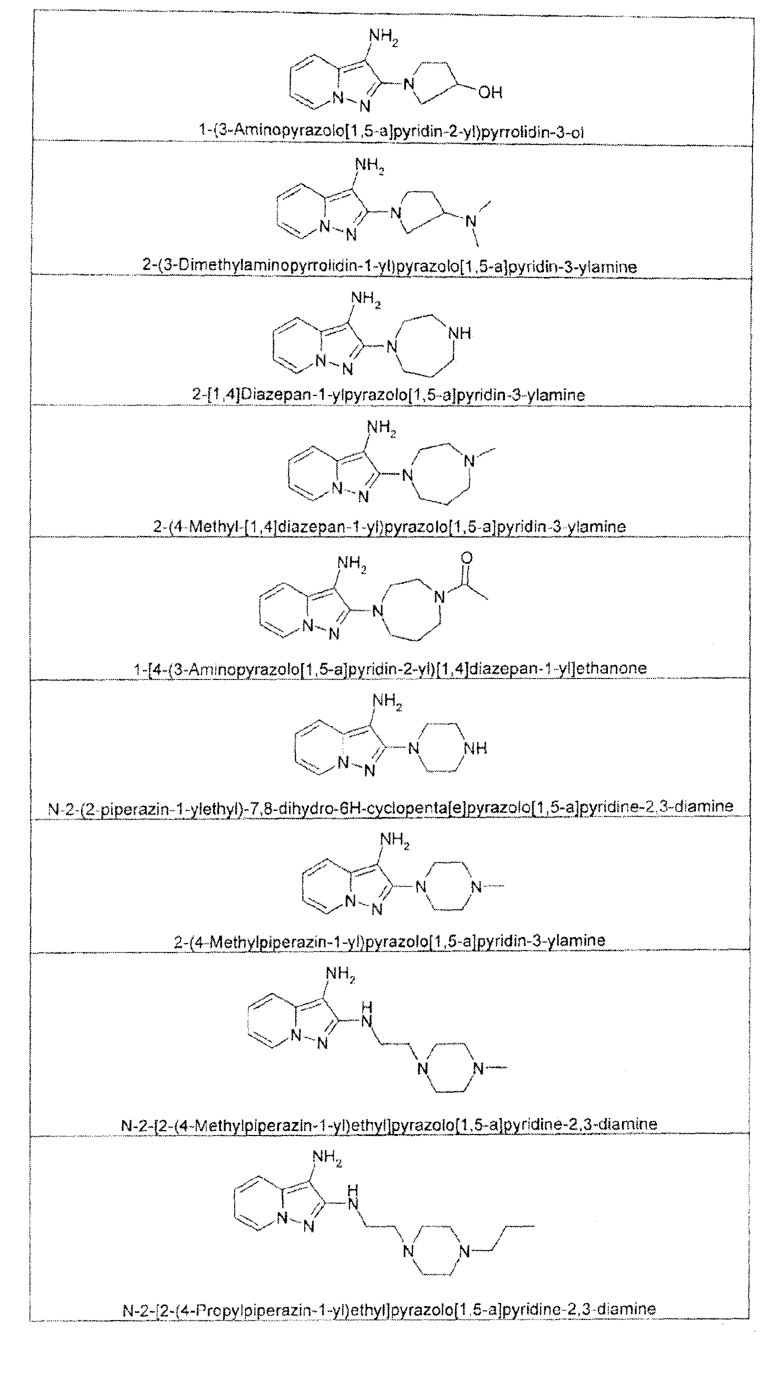

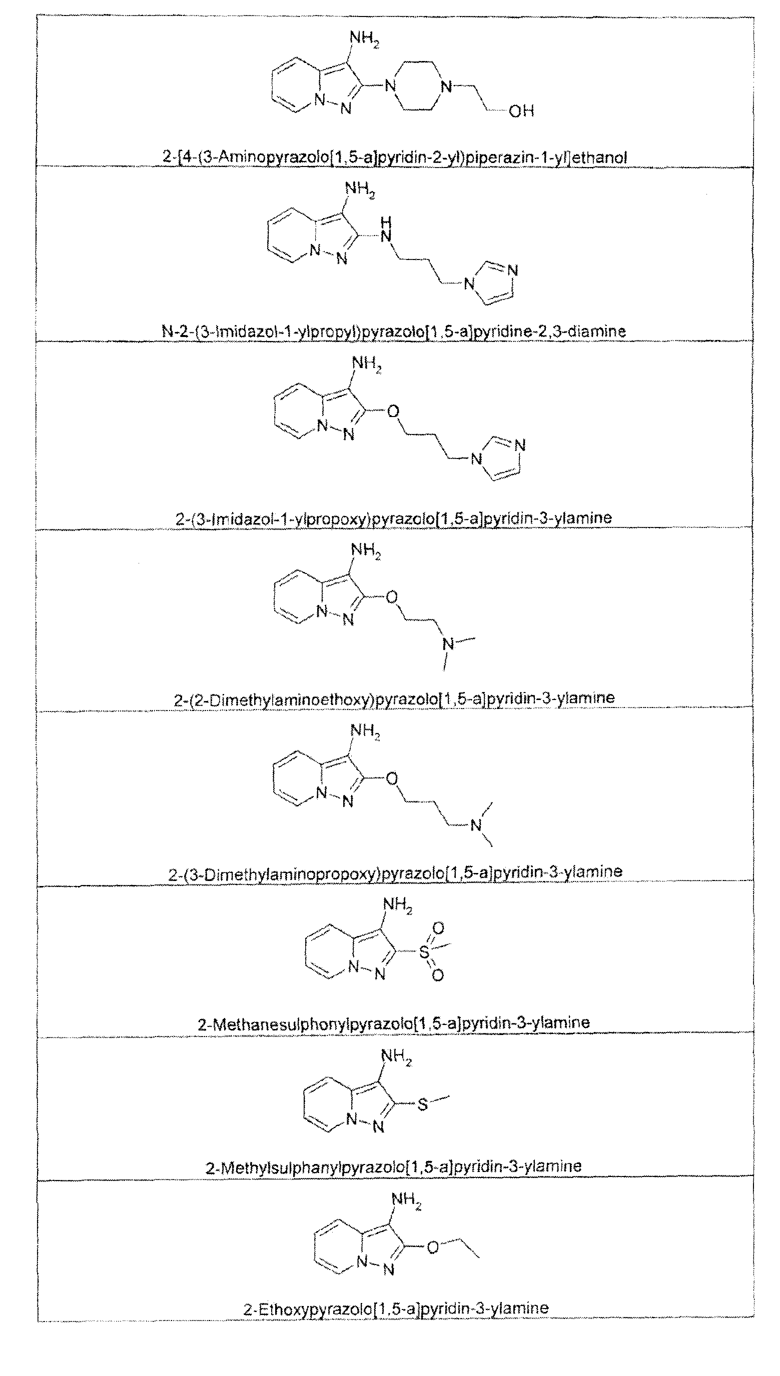

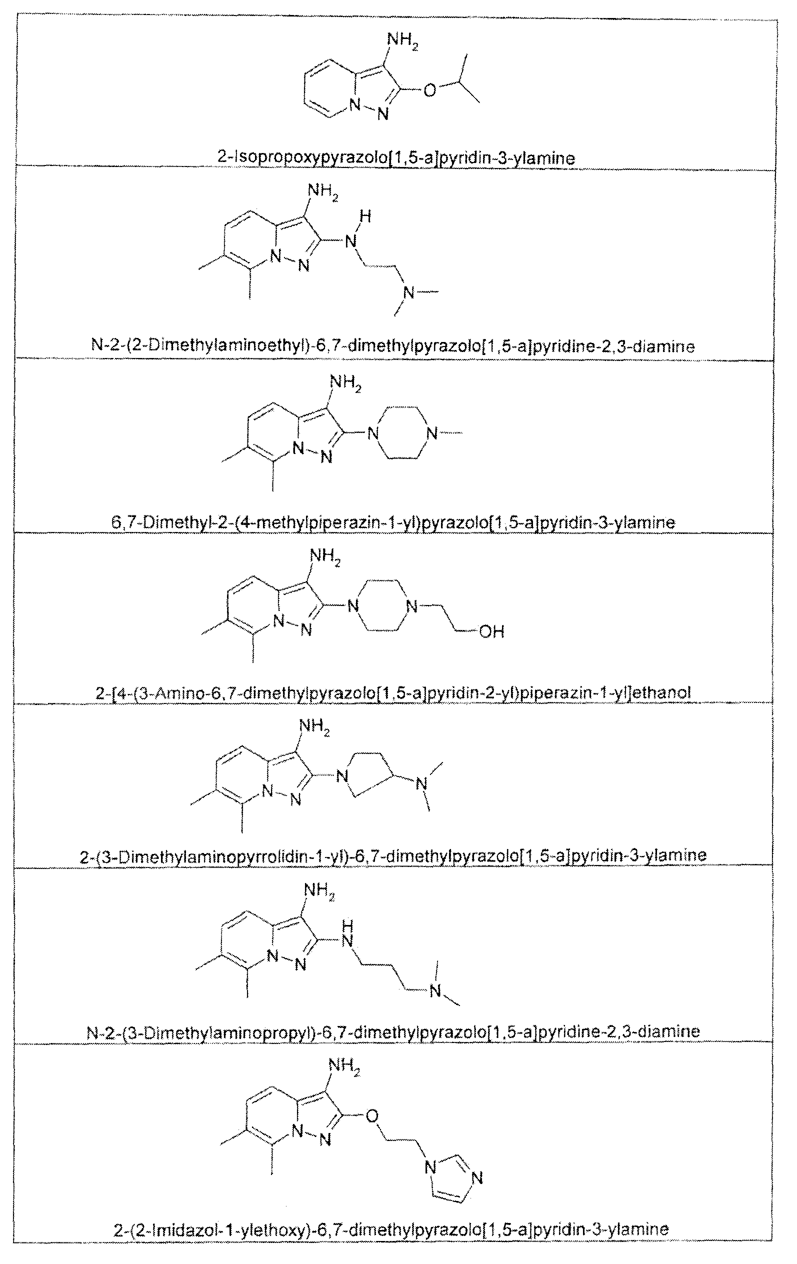

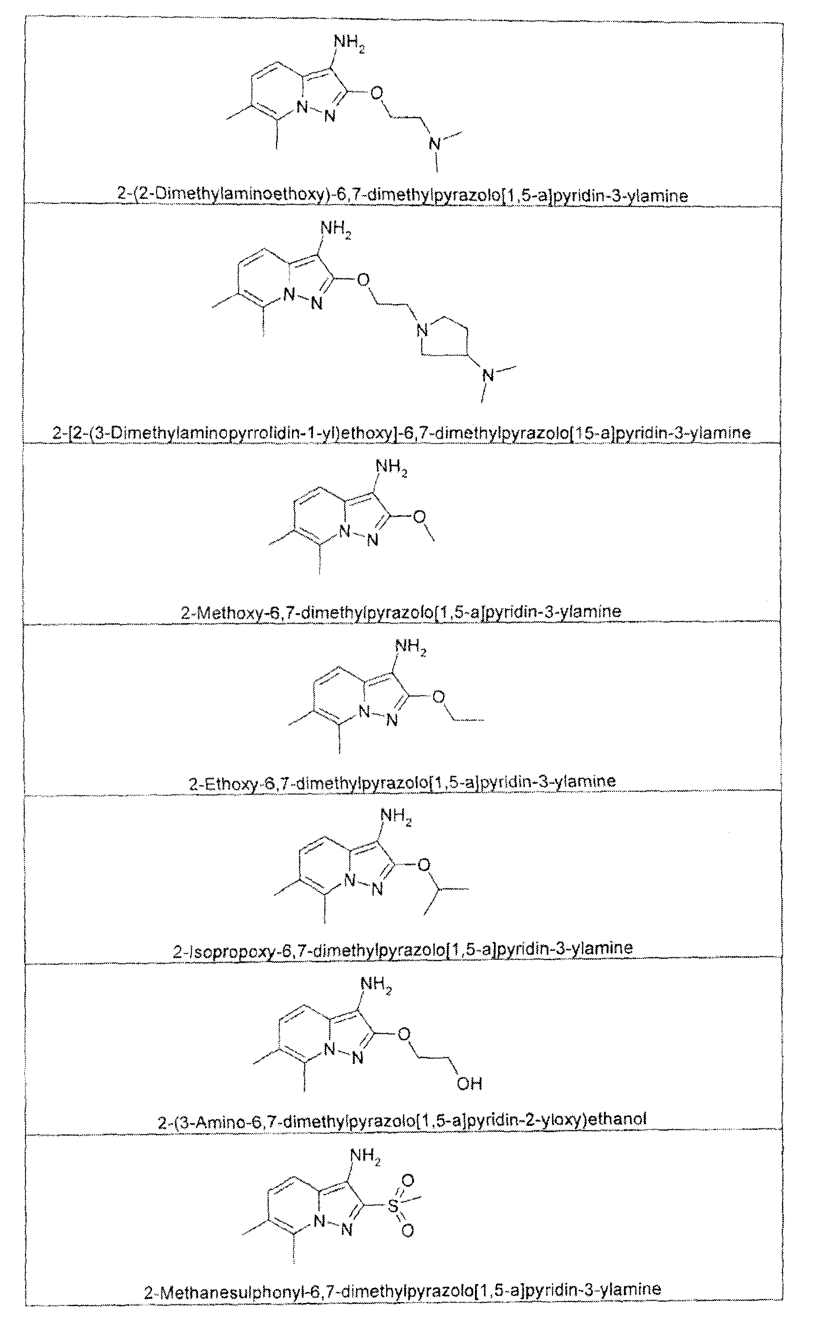

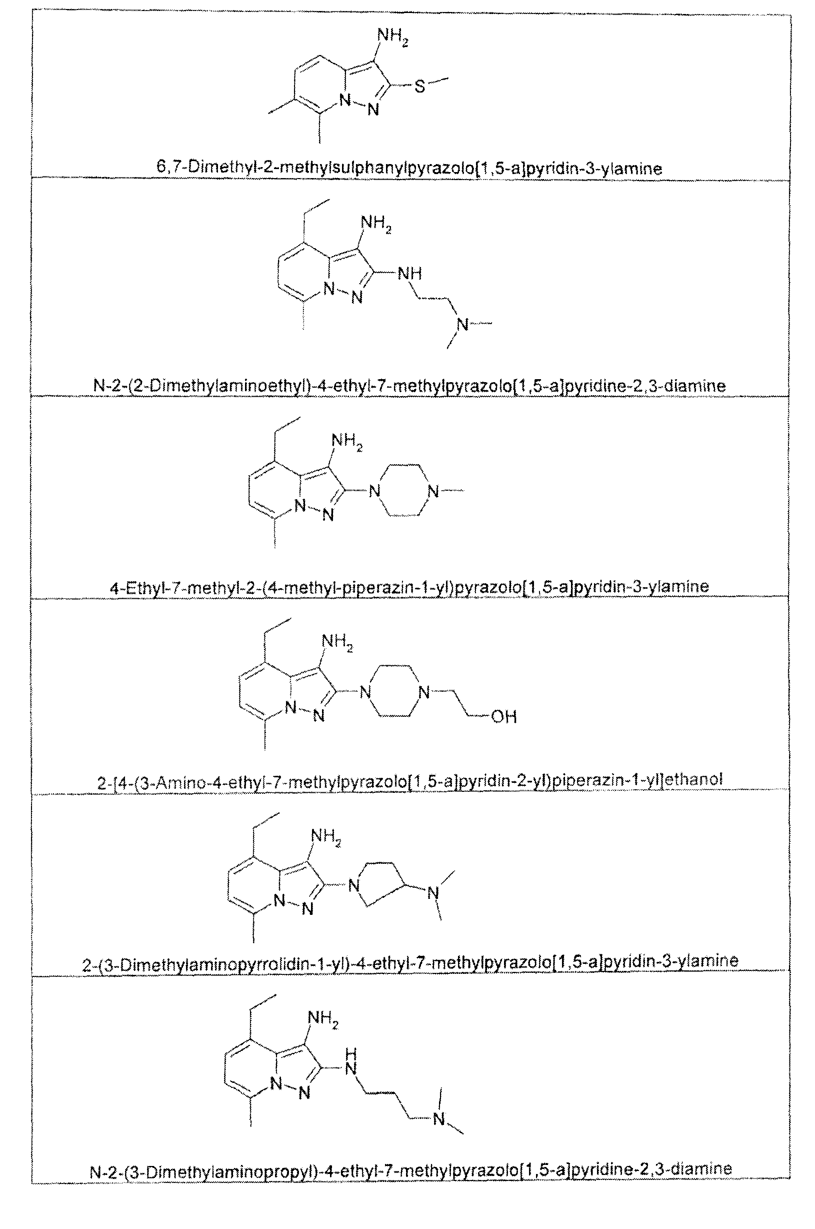

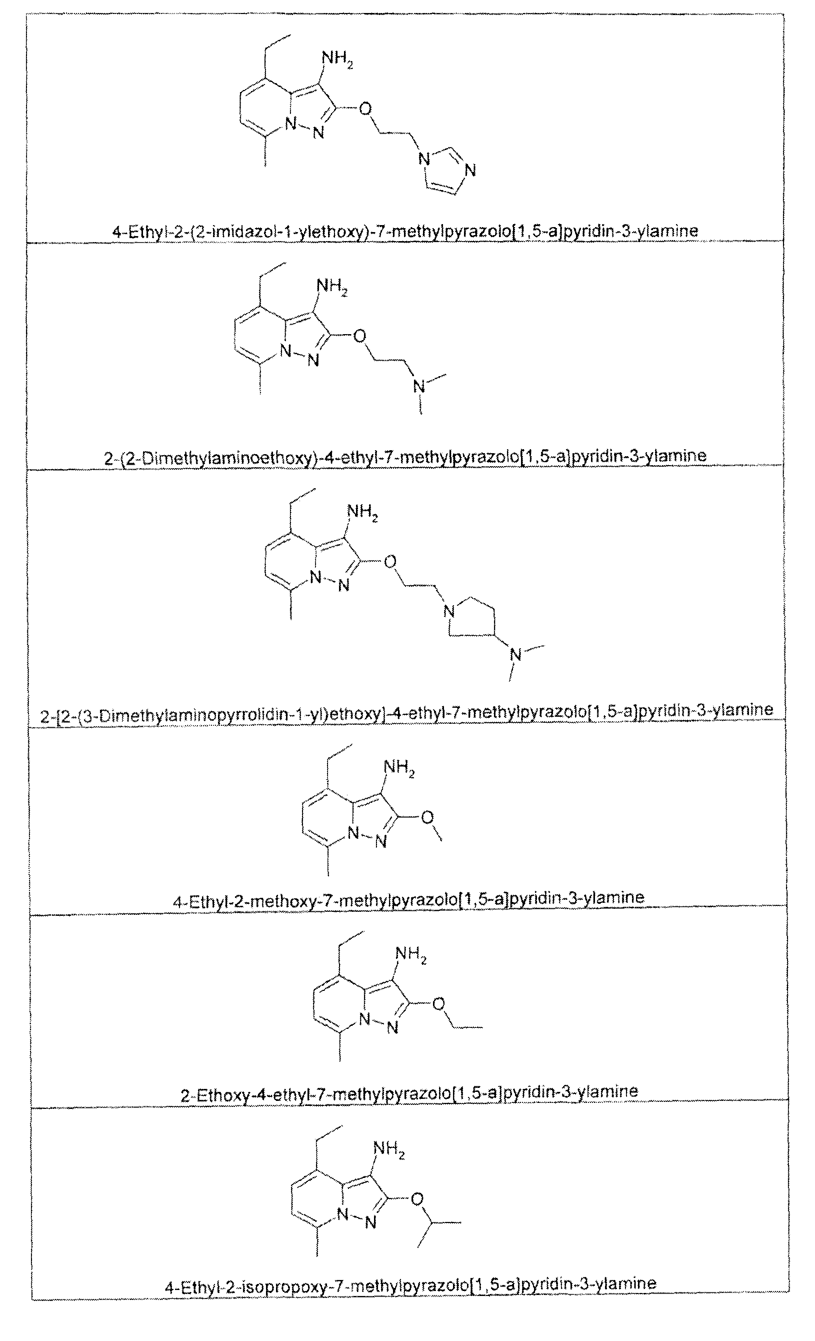

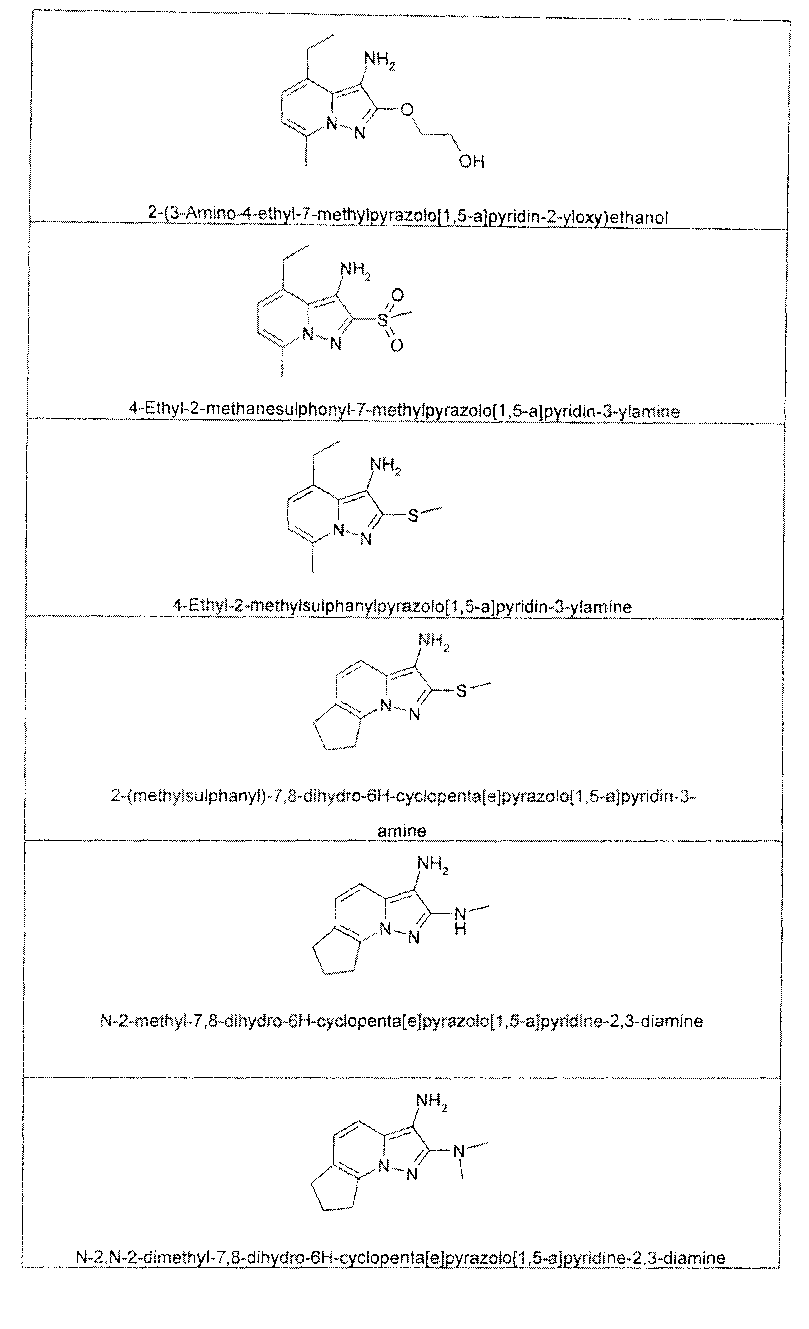

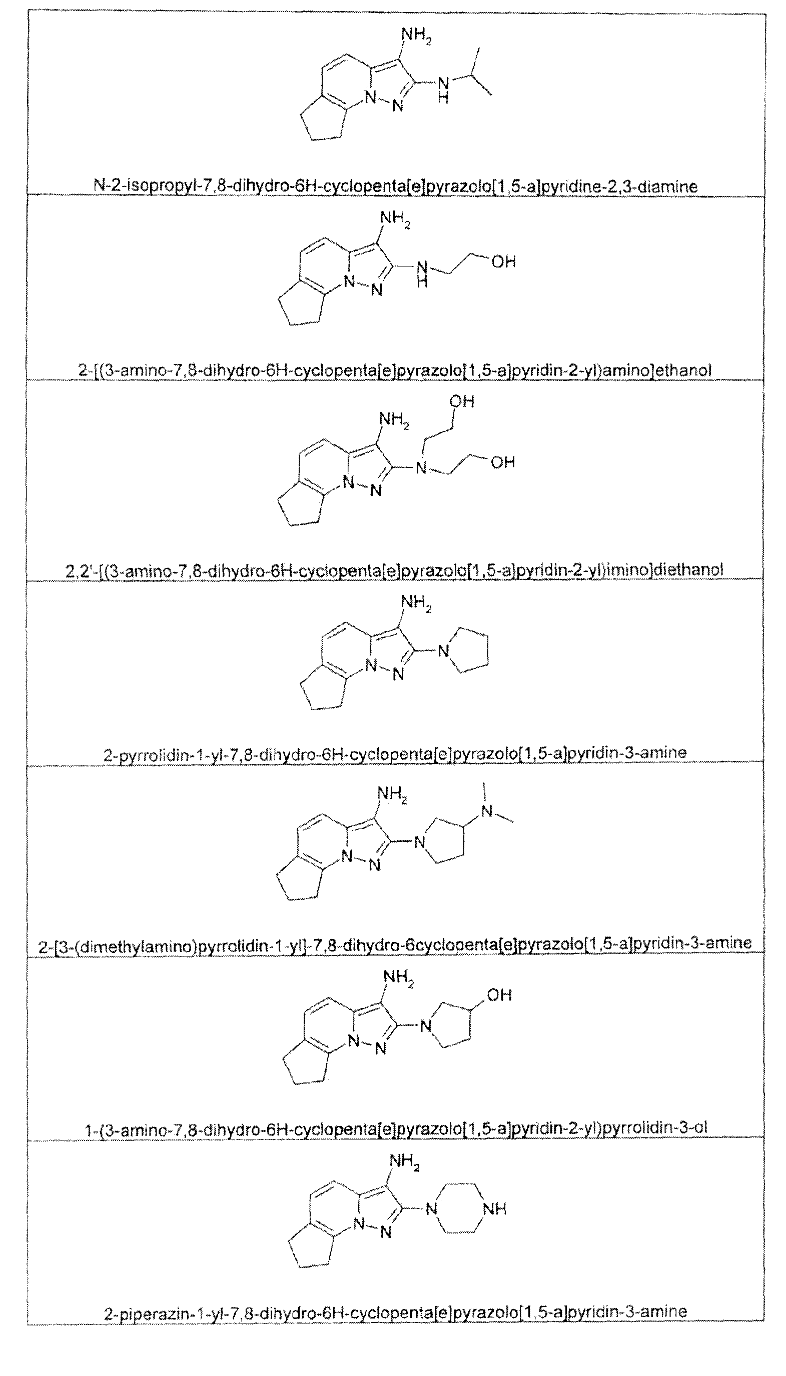

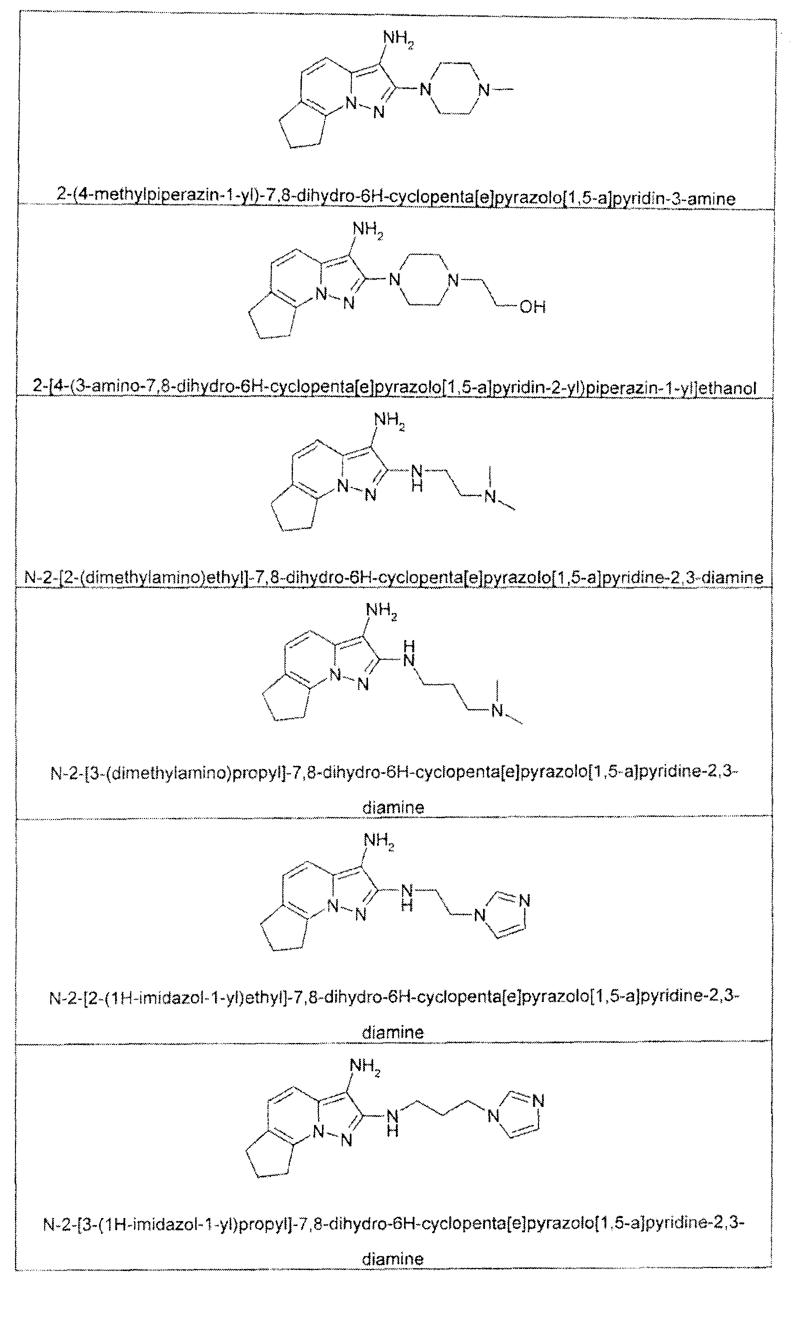

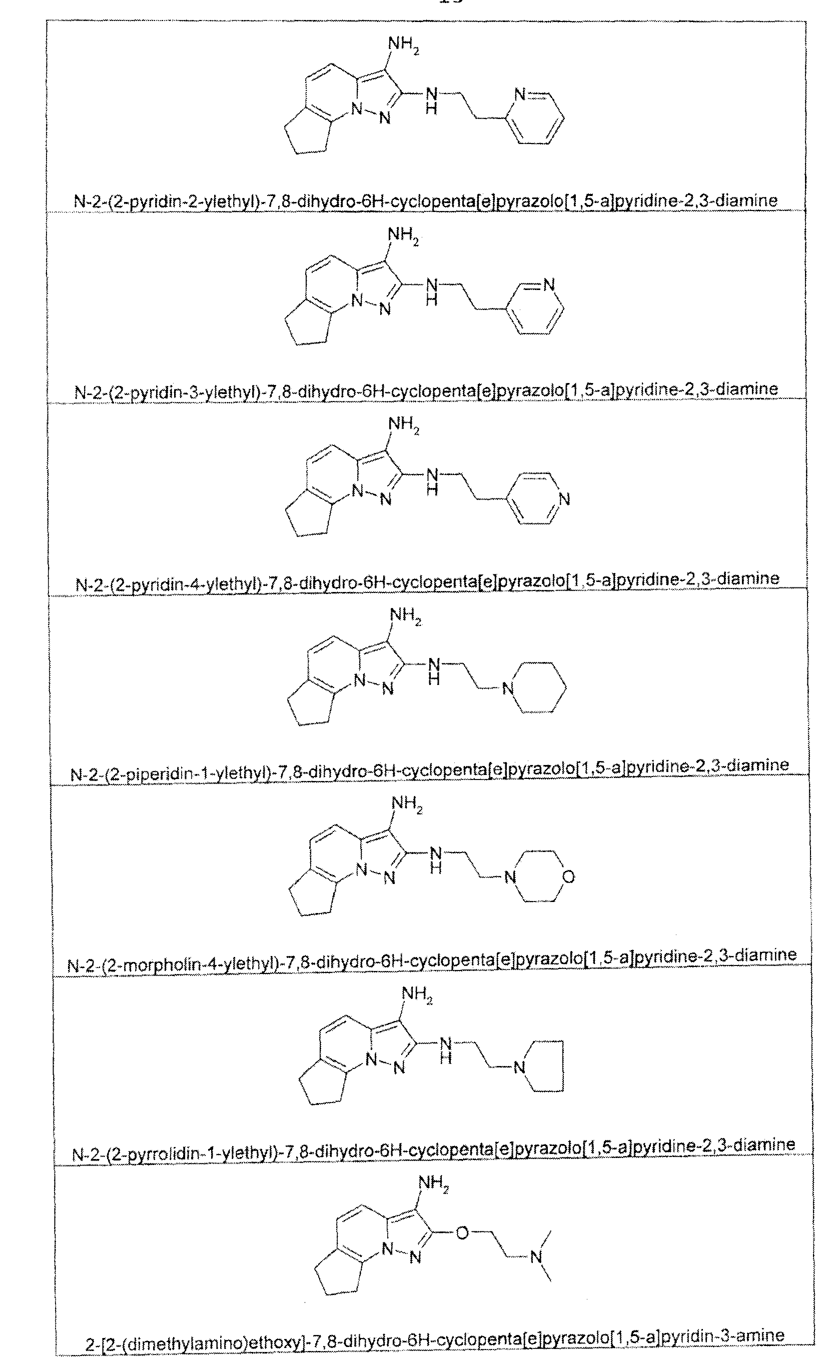

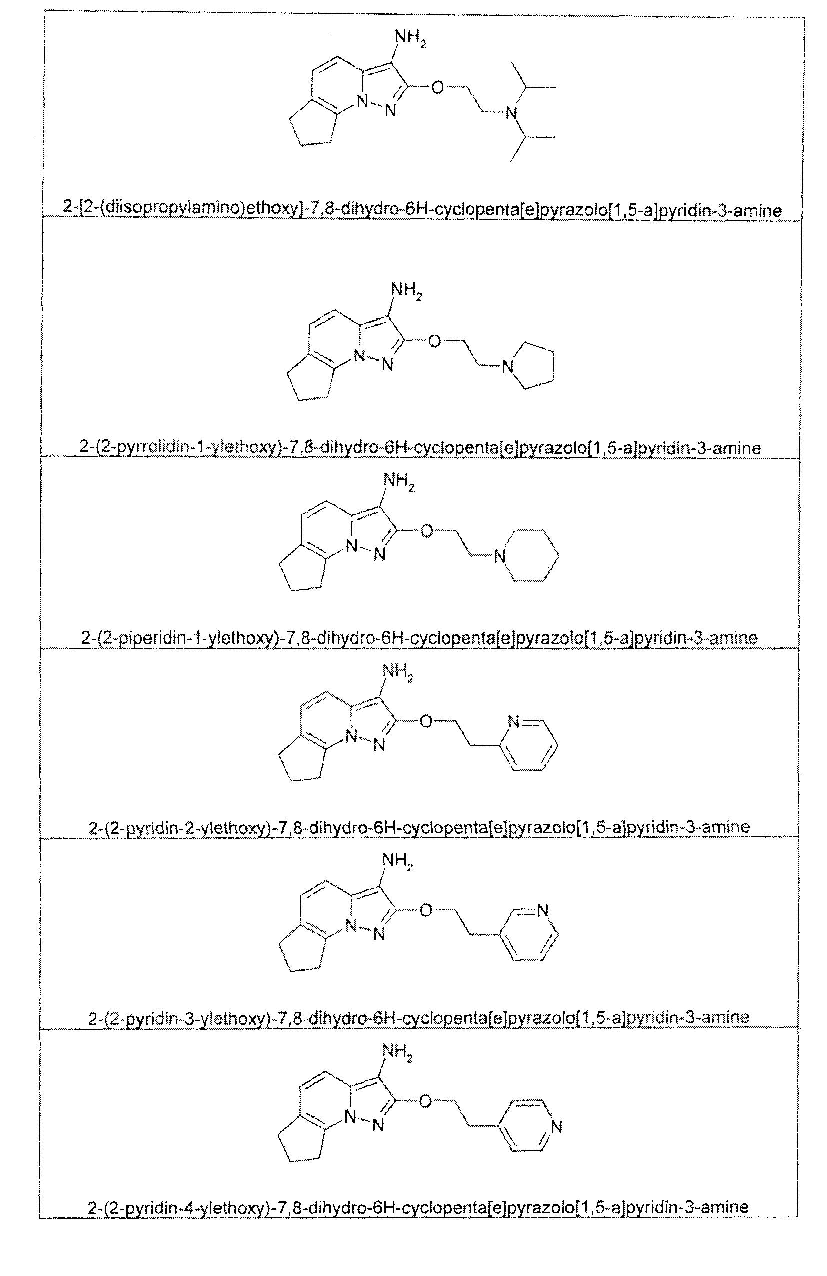

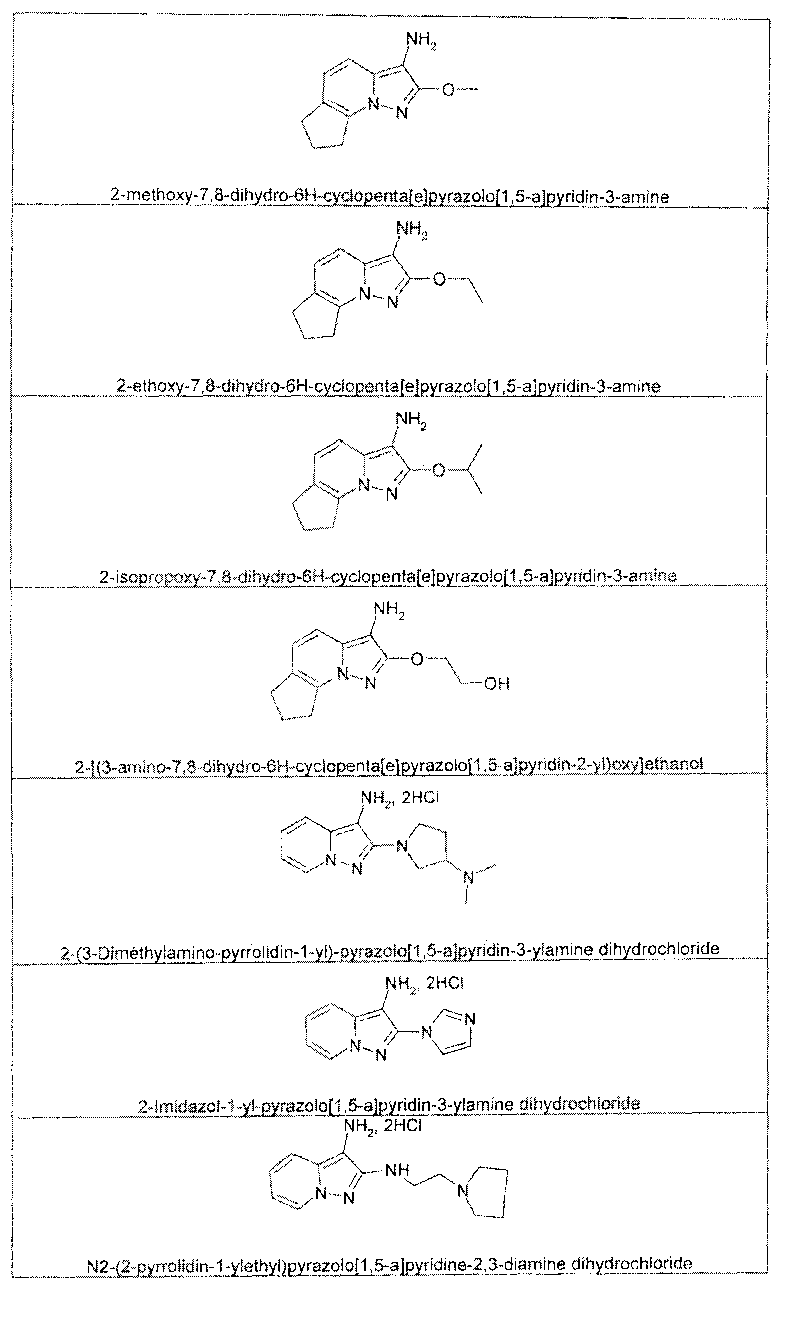

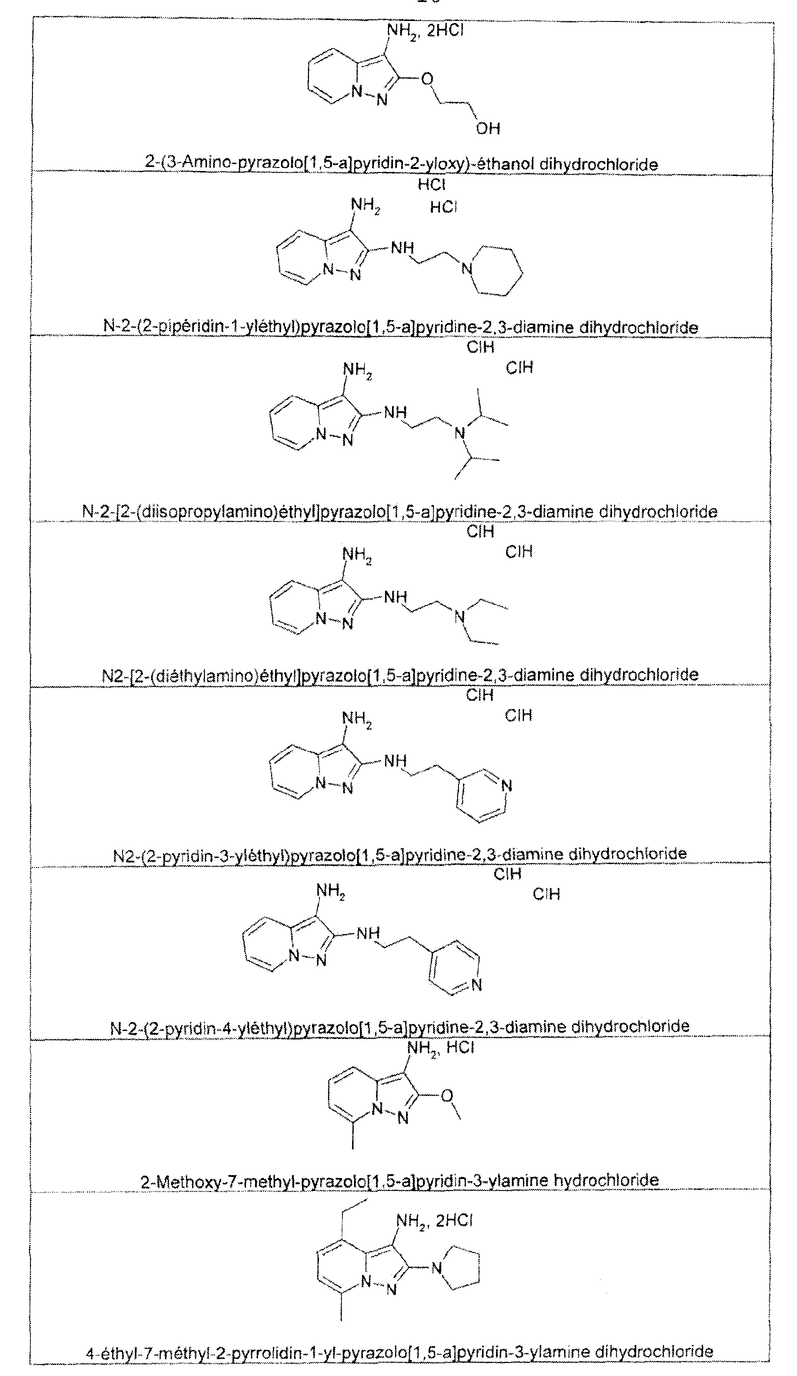

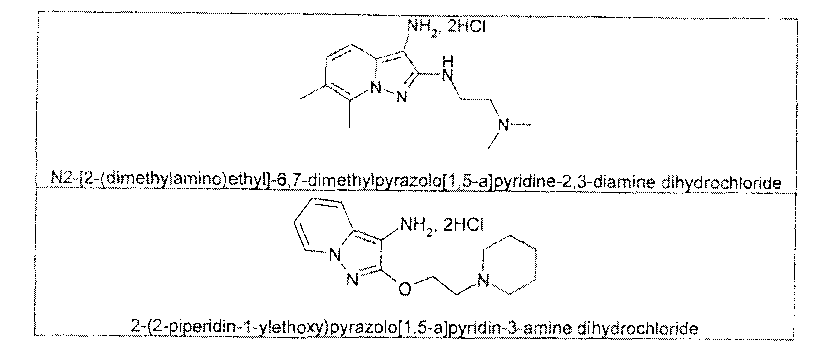

8. Composition according to Claim 7 in which the compound of formula (I) is selected from:
N-2-ethylpyrazolo[1,5-a]pyridine-2,3-diamine dihydrochloride,
2-(3-aminopyrazolo[1,5-a]pyridin-2-ylamino)ethanol dihydrochloride,
1-(3-aminopyrazolo[1,5-a]pyridin-2-ylamino)propan-2-ol dihydrochloride,
N-2-(3-imidazol-1-ylpropyl)pyrazolo[1,5-a]pyridine-2,3-diamine dihydrochloride,
2-pyrrolidin-1-ylpyrazolo[1,5.a] pyridin-3-ylamine dihydrochloride,
2-(3-dimethylaminopyrrolidin-1-yl)pyrazolo[1,5-a]pyridin-3-ylamine dihydrochloride,
2-imidazol-1-ylpyrazolo[1,5-a]pyridin-3-ylamine dihydrochloride,
N-2-(2-pyrrolidin-1-ylethyl)pyrazolo[1,5-a]pyridine-2,3-diamine dihydrochloride,
2-(3-aminopyrazolo[1,5-a]pyridin-2-yloxy)ethanol dihydrochloride,
2-ethoxypyrazolo[1,5-a]pyridin-3-ylamine dihydrochloride,
2-methoxy-7-methylpyrazolo[1,5-a]pyridin-3-ylamine hydrochloride,
4-ethyl-2-methoxy-7-methylpyrazolo[1,5-a]pyridin-3-ylamine dihydrochloride,
4-ethyl-7-methyl-2-pyrrolidin-1-ylpyrazolo[1,5-a]pyridin-3-ylamine dihydrochloride,
N-2-(2-piperidin-1-ylethyl)pyrazolo[1,5-a]pyndine-2,3-diamine dihydrochloride,
N-2-[2-(diisopropylamino)ethyl]pyrazolo[1,5.a]pyridine-2,3-diamine dihydrochloride,
N-2-[2-(diethylamino)ethyl]pyrazolo[1,5-a]pyridine-2,3-diamine dihydrochloride,
N-2-(2-pyridin-3-ylethyl)pyrazolo[1,5-a]pyridine-2,3-diamine dihydrochloride,
N-2-(2-pyridin-4-ylethyl)pyrazolo[1,5-a]pyridine-2,3-diamine dihydrochloride,
N-2-(2-pyridin-2-ylethyl)pyrazolo [1,5-a]pyridine-2,3-diamine, diamine dihydrochloride,
N-2-(2-piperazin-1-ylethyl)pyrazolo[1,5-a]pyridine-2,3-diamine,
N-2-[2-(4-methylpiperazin-1-yl)ethyl]pyrazolo[1,5-a]pyridine-2,3-diamine dihydrochloride, N-2-(2-morpholin-4-ylethyl)pyrazolo[1,5-a]pyridine-2,3-diamine, dihydrochloride,
N-2-[2-(dimethylamino)ethyl]-7,8-dihydro-6H-cyclopenta[e]pyrazolo[1,5-a]pyridine-2,3-diamine dihydrochloride,
2-[(3-amino-7,8-dihydro-6H-cyclopenta[e]pyrazolo[1,5-a]pyridin-2-yl)amino]ethanol hydrochloride, 2-methoxy-7,8-dihydro-6H-cyclopenta[e]pyrazolo[1,5-a]pyridin-3-amine hydrochloride,
2-isopropoxy-7,8-dihydro-6H-cyclopenta[e]pyrazolo[1,5-a]pyridin-3-amine hydrochloride,
2-[2-(dimethylamino)ethoxy]pyrazolo[1,5-a]pyridin-3-amine dihydrochloride,
2-(2-pyrrolidin-1-ylethoxy)pyrazolo[1,5-a]pyridin-3-amine dihydrochloride,
2-(2-piperidin-1-ylethoxy)pyrazolo[l,5-a]pyridin-3-amine dihydrochloride,
2-(2-morpholin-4-ylethoxy)pyrazolo[1,5-a]pyridin-3-amine dihydrochloride,
2-[2-(diisopropylamino)ethoxylpyrazolo[1,5-a]pyridin-3-amine dihydrochloride,
2-(2-pyridin-2-ylethoxy)pyrazolo[1,5-a]pyridin-3-amine dihydrochloride,
2-(2-pyridin-3-ylethoxy)pyrazolo[1,5-a]pyridin-3-amine dihydrochloride,
2-(2-pyridin-4-ylethoxy)pyrazolo[1,5-a]pyridin-3-amine dihydrochloride.

9. Composition according to any one of the preceding claims in which the amount of each of the oxidation bases of formula (I) is between about 0.001 and 10 wt.% of the total weight of the dyeing composition.

10. Composition according to any one of the preceding claims, additionally comprising a coupler selected from the meta-phenylenediamines, meta-aminophenols, meta-diphenols, naphthalenic couplers, heterocyclic couplers and their salts of addition, as well as their mixtures.

11. Composition according to Claim 10 in which the amount of each of the couplers is between 0.001 and 10 wt.%- of the total weight of the dyeing composition.

12. Method for dyeing keratin fibres, **characterized in that** a composition as defined in any one of Claims 1 to 11 is applied to keratin fibres in the presence of an oxidizing agent for a time that is sufficient to develop the desired coloration.

13. Method according to Claim 12 in which the oxidizing agent is selected from hydrogen peroxide, urea peroxide, alkali metal bromates, per-salts, peracids and oxidase enzymes.

14. Kit with several compartments in which a first compartment contains a dyeing composition as defined in any one of Claims 1 to 11 and a second compartment contains an oxidizing agent.

15. Compounds of formula (I) as defined in any one of Claims 1 to 11 with the exception of the compounds 2-methylsulphanylpyrazolo[1,5-a]pyridin-3-ylamine and 2,3-diaminopyrazolo-[1,5-a]pyridine.

16. Method of preparation of a compound of formula (I) as defined in the compositions according to any one of Claims 1 to 8 which comprises the following stages: 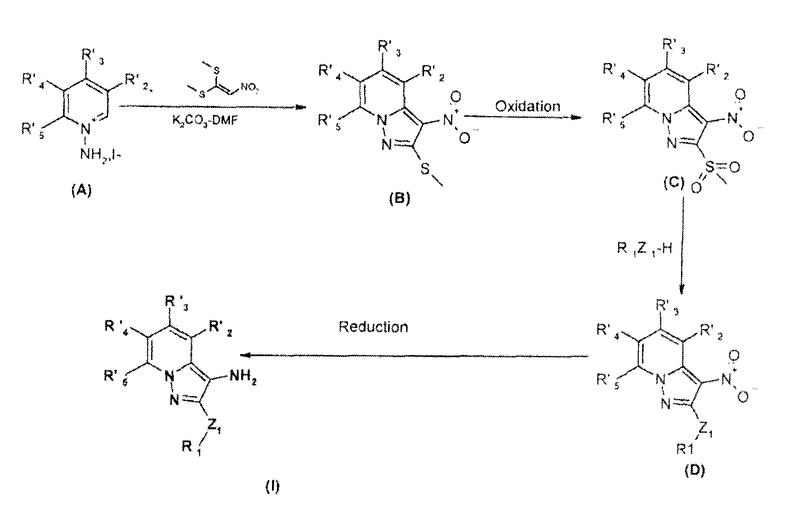 in which R'₂, R'₃, R'₄ and R'₅ have the same meanings as R₂, R₃, R₄ and R₅ or are precursors thereof.

17. Intermediates of intermediate formulae (A) and (B) as defined above in which at least one of the radicals R², R'₃, R'₄ or R'₅ is different from a hydrogen atom apart from the compound for which R'₂ = Me when all of the other substituents are hydrogen.

18. Intermediates of formulae (c), (D) as defined in Claim 16.

## Patentansprüche

1. Zusammensetzung zum Färben von Keratinfasern, die in einem zum Färben geeigneten Medium ein 3-Amino-pyrazolo-[1,5-a]-pyridinderivat der Formel (I) sowie seine Salze und Solvate als Oxidationsbase enthält: 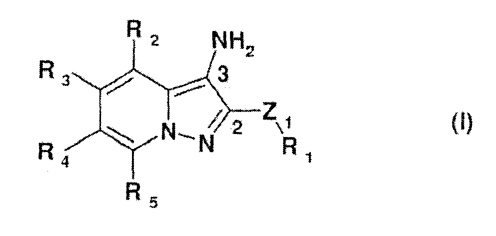 in der Formel:
Z₁ bedeutet ein Sauerstoffatom, eine Gruppe NR₆; wenn Z₁ eine Gruppe NR₆ ist, können R₁ und R₆ gemeinsam mit dem Stickstoffatom, an das sie gebunden sind, einen 5-bis 8-gliedrigen, gesättigten, ungesättigten oder aromatischen He-terocyclus bilden, der gegebenenfalls substituiert ist, Z₁ kann auch eine zweiwertige Gruppe S, SO oder SO₂ bedeu-ten, wenn R₁ CH₃ ist,
R₁ ist unter einer Alkylgruppe, einer mit Hydroxy substituier-ten Alkylgruppe, einer mit einer Amino-oder (Di)alkylamino-gruppe substituierten Alkylgruppe, einer mit einem Stickstoff-heterocyclus substituierten Alkylgruppe ausgewählt,
R₆ kann unter einem Wasserstoffatom, einer Alkylgruppe, ei-ner mit Hydroxy, einer Aminogruppe, einer Alkylaminogrup-pe, einer Dialkylaminogruppe substituierten Alkylgruppe, ei-ner mit einem Stickstoffheterocyclus substituierten Alkyl-gruppe ausgewählt werden,
R₂, R₃, R₄ und R₅ bedeuten unabhängig voneinander: -ein Wasserstoffatom, -eine C₁-₄-Alkylgruppe, die gegebenenfalls substituiert ist,
R₂, R₃, R₄ und R₅ können paarweise mit angrenzenden Grup-pen einen gesättigten oder ungesättigten (Hetero)cyclus bil-den, der gegebenenfalls substituiert ist,
wobei das 2-Methoxy-pyrazolo[1,5-a]pyridin-3-ylamin und das 2-Morpholino-pyrazolo[1,5-a]pyridin-3-ylamin der folgenden For-meln ausgenommen sind:

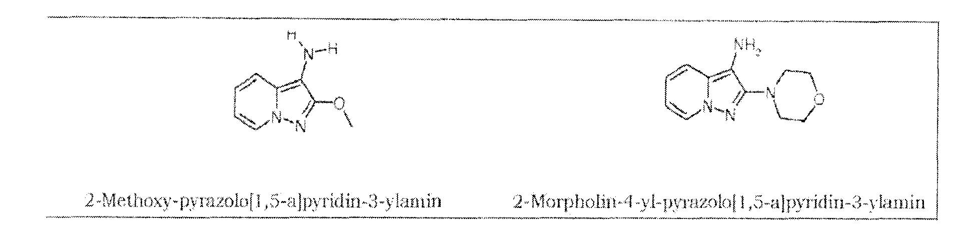

2. Zusammensetzung nach Anspruch 1, wobei Z₁ ein Sauersteff-atom, eine Gruppe -NR₆-oder eine Gruppe -NR₆-bedeutet, die mit R₁ einen Heterocyclus bildet.

3. Zusammensetzung nach Anspruch 1 oder 2, wobei die Alkylgruppe von R1 der R₆ 1 bis 6 Kohlenstoffatome aufweist.

4. Zusammensetzung nach Anspruch 1, wobei R₆ mit R₁ und dem Stickstoffatom, an das sie gebunden sind, einen Heterocyclus bilden, der unter den Imidazolen, Piperazinen, Pyrrolidinen und Diazepanen ausgewählt ist, wobei diese Heterocyclen substituiert oder unsubstituiert vorliegen können.

5. Zusammensetzung nach einem der vorhergehenden Ansprüche, wobei die Gruppen R₄ und R₅ gemeinsam einen 5-bis 8-gliedrigen, gesättigen oder ungesättigten (Hetero)cyclus bilden.

6. Zusammensetzung nach Anspruch 5, wobei R₄ und R₅ gemein-sam eine Cyclopentangruppe bilden.

7. Zusammensetzung nach einem der Ansprüche 1 bis 6, wobei die Verbindung der Formel (I) unter den nachfolgenden Verbindun-gen und den entsprechenden Additionssalzen und Solvaten aus-gewählt sind:

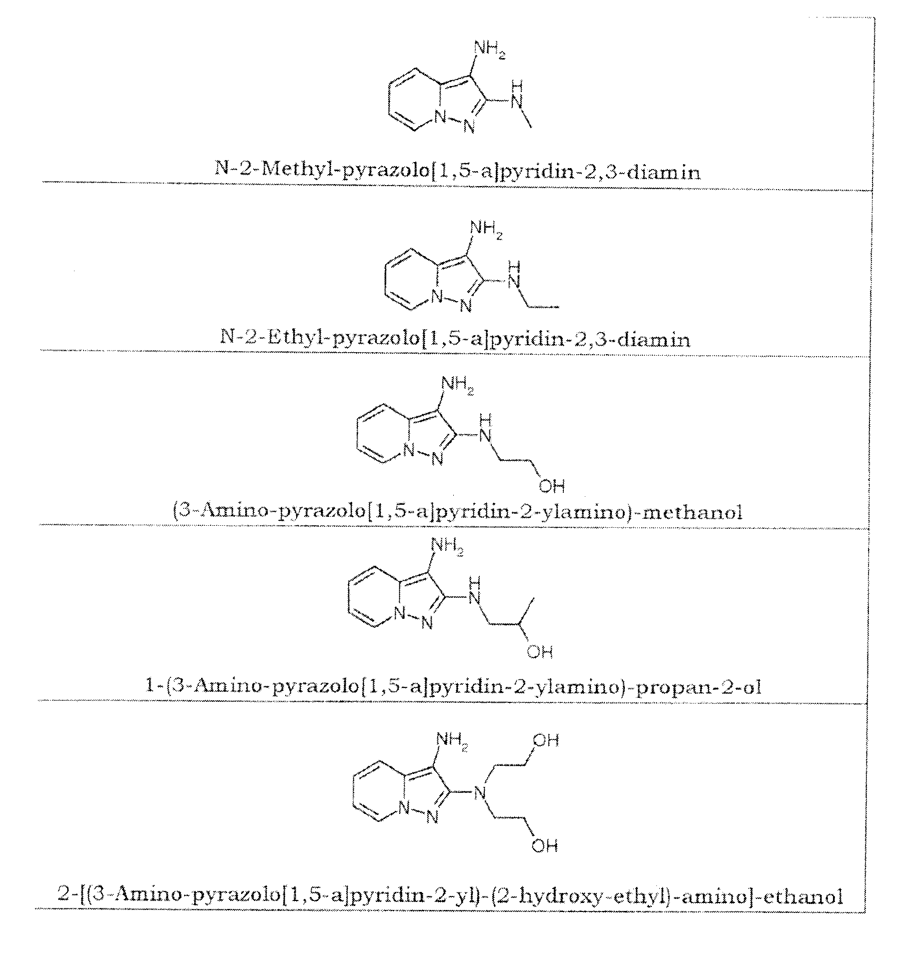

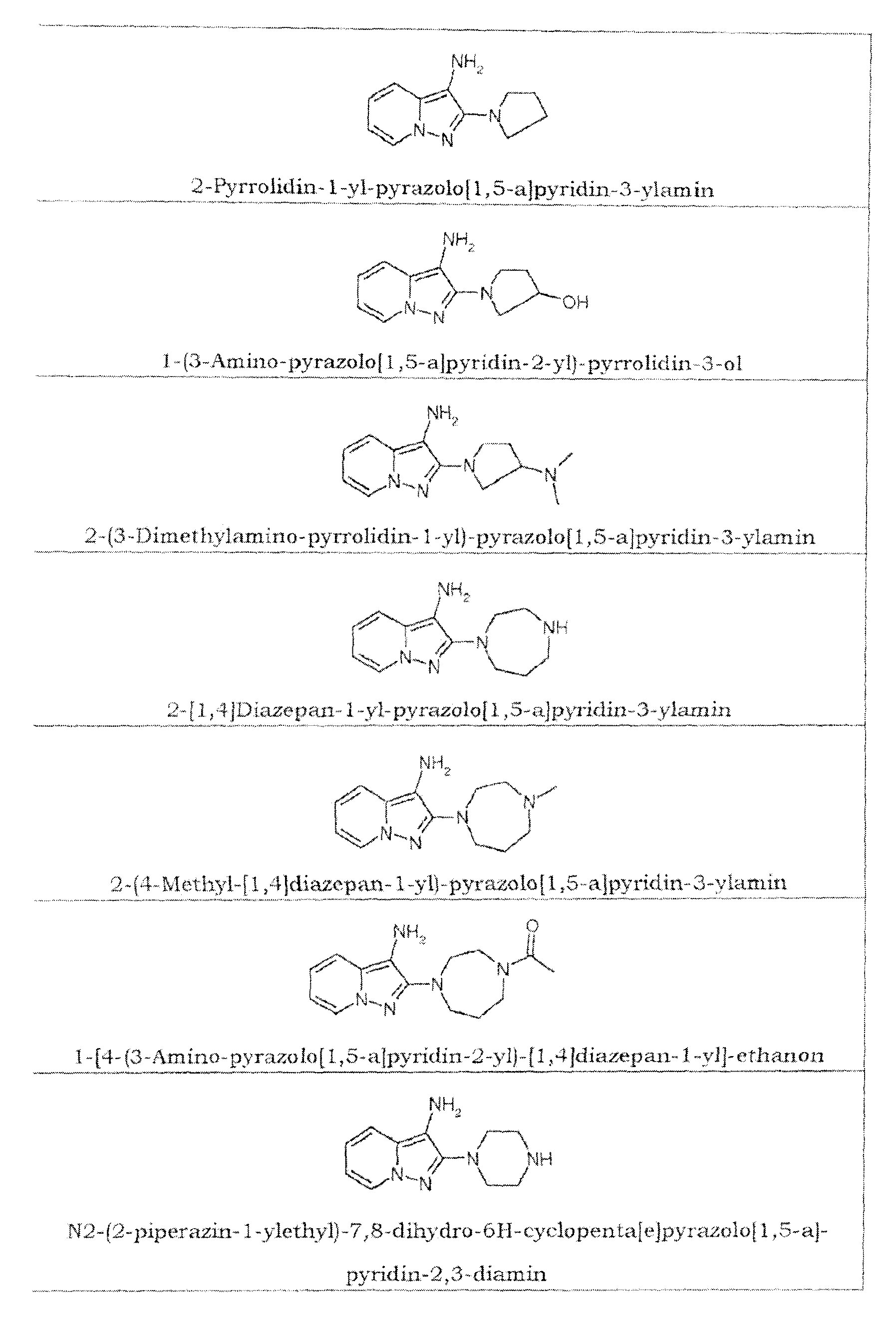

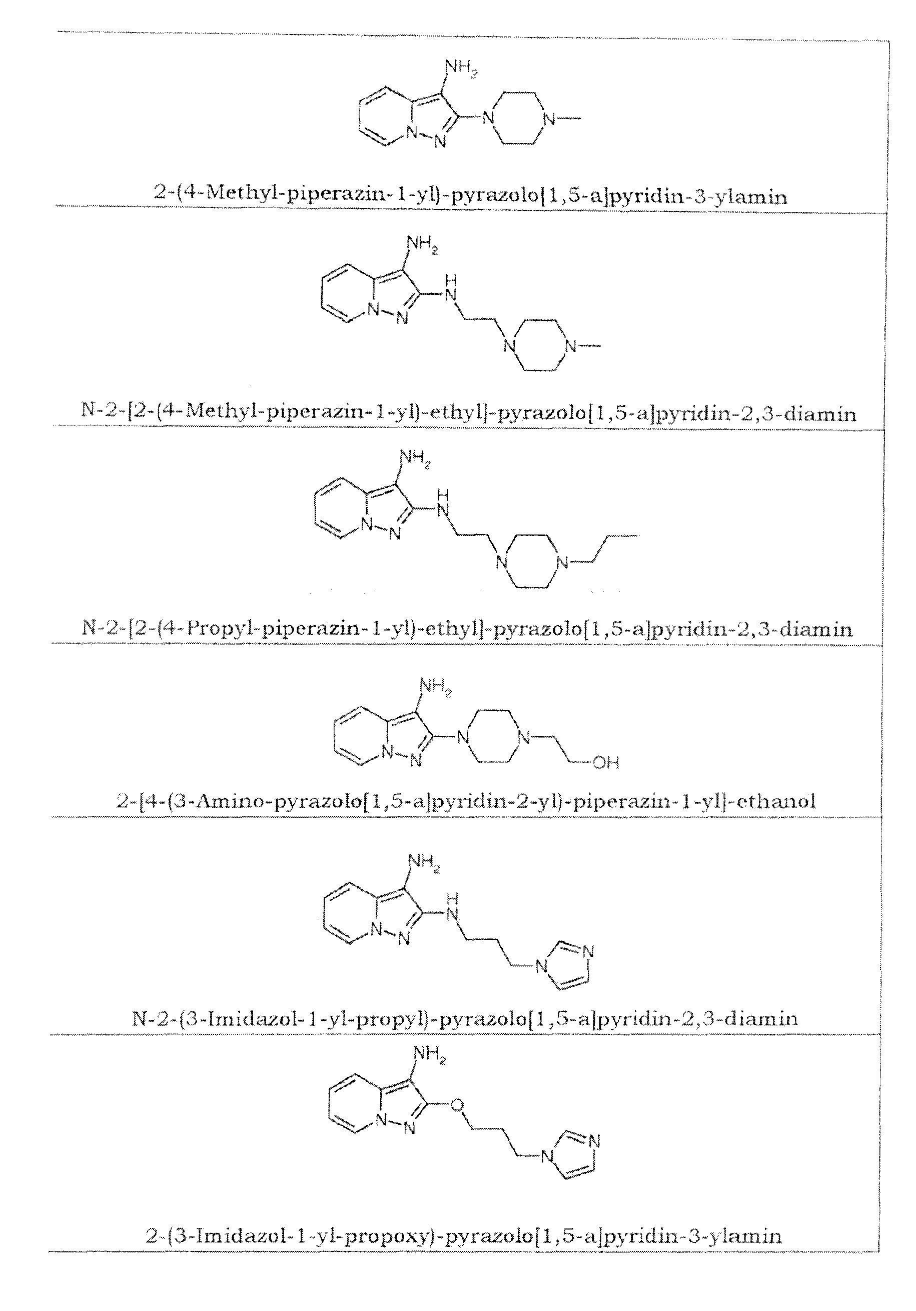

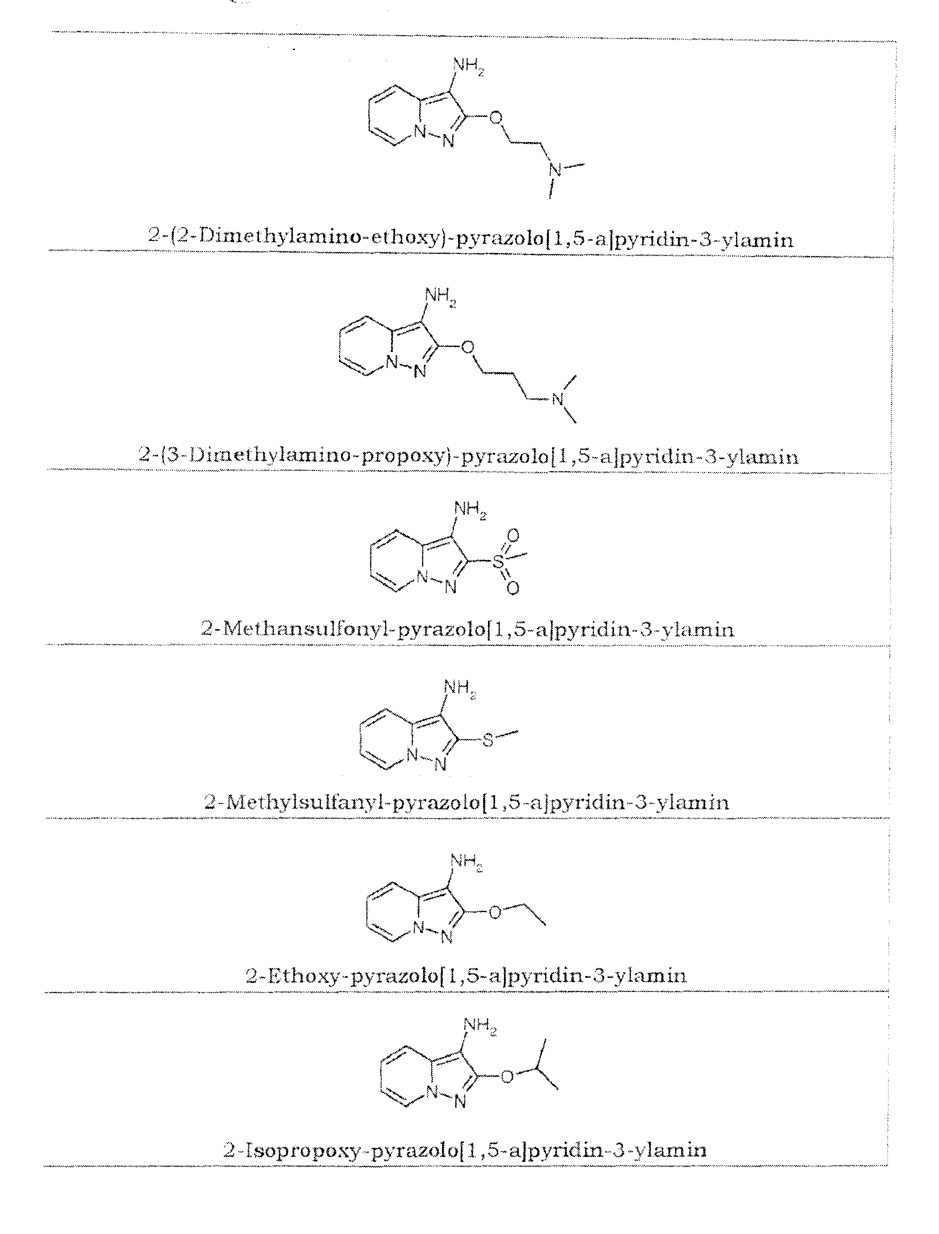

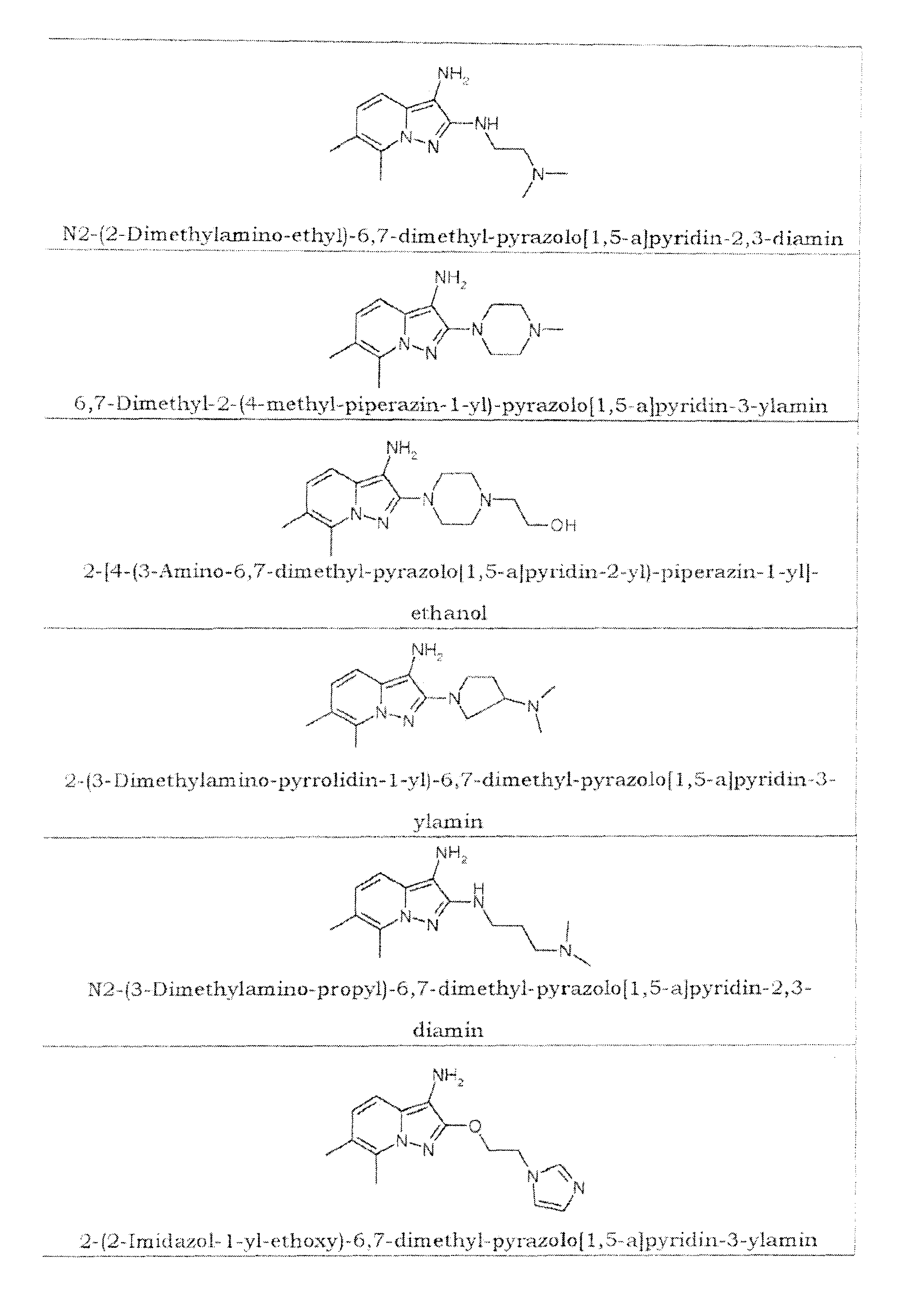

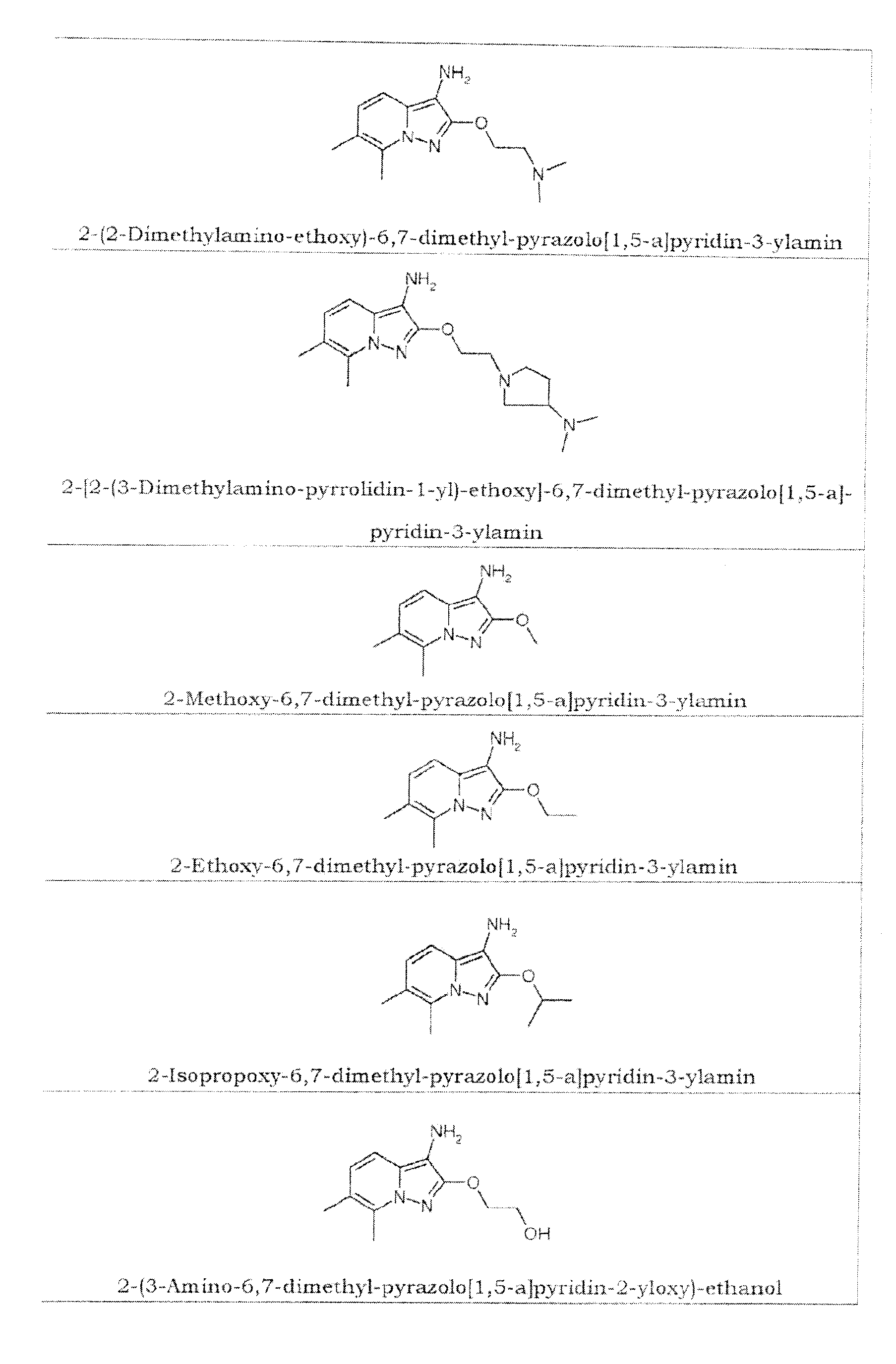

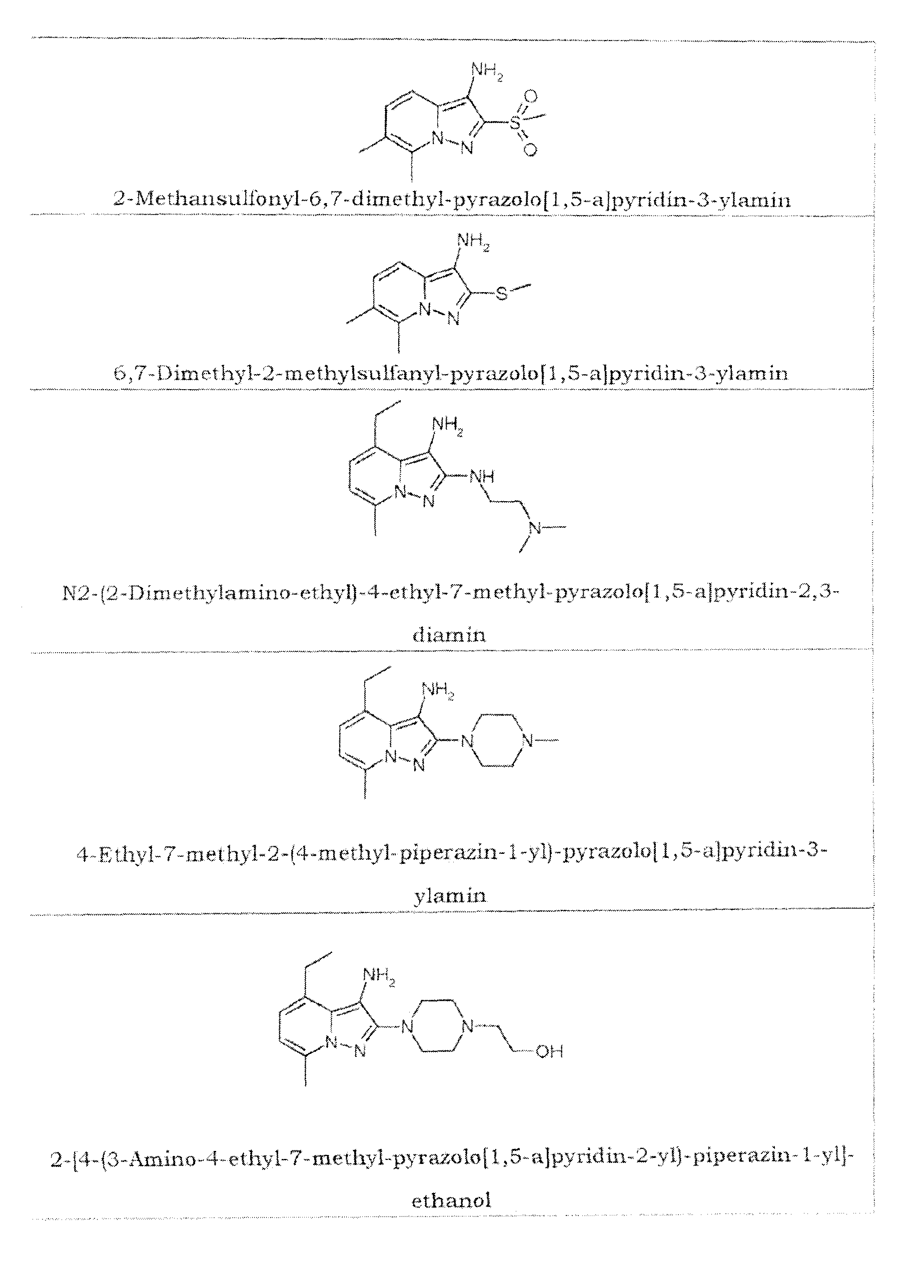

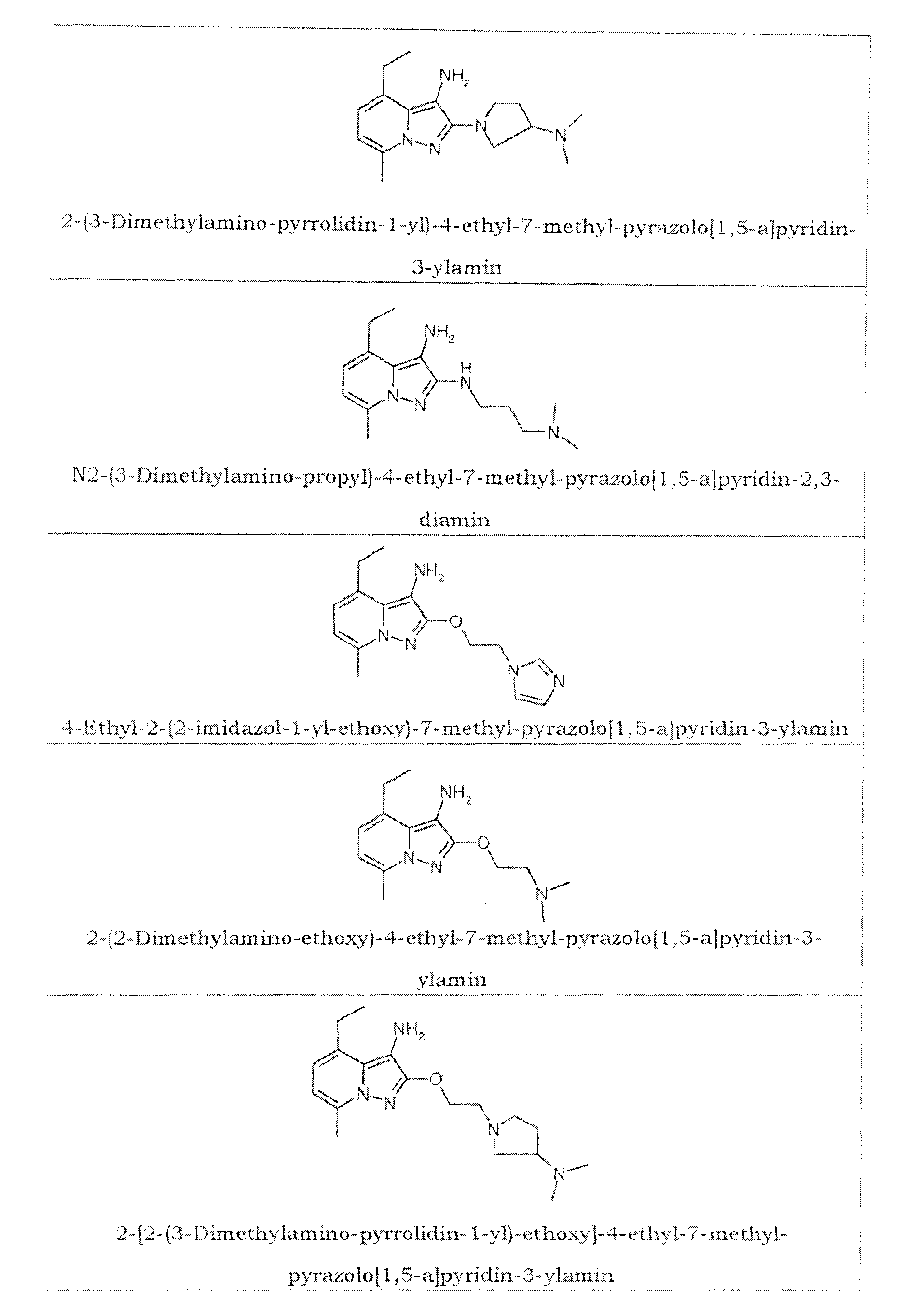

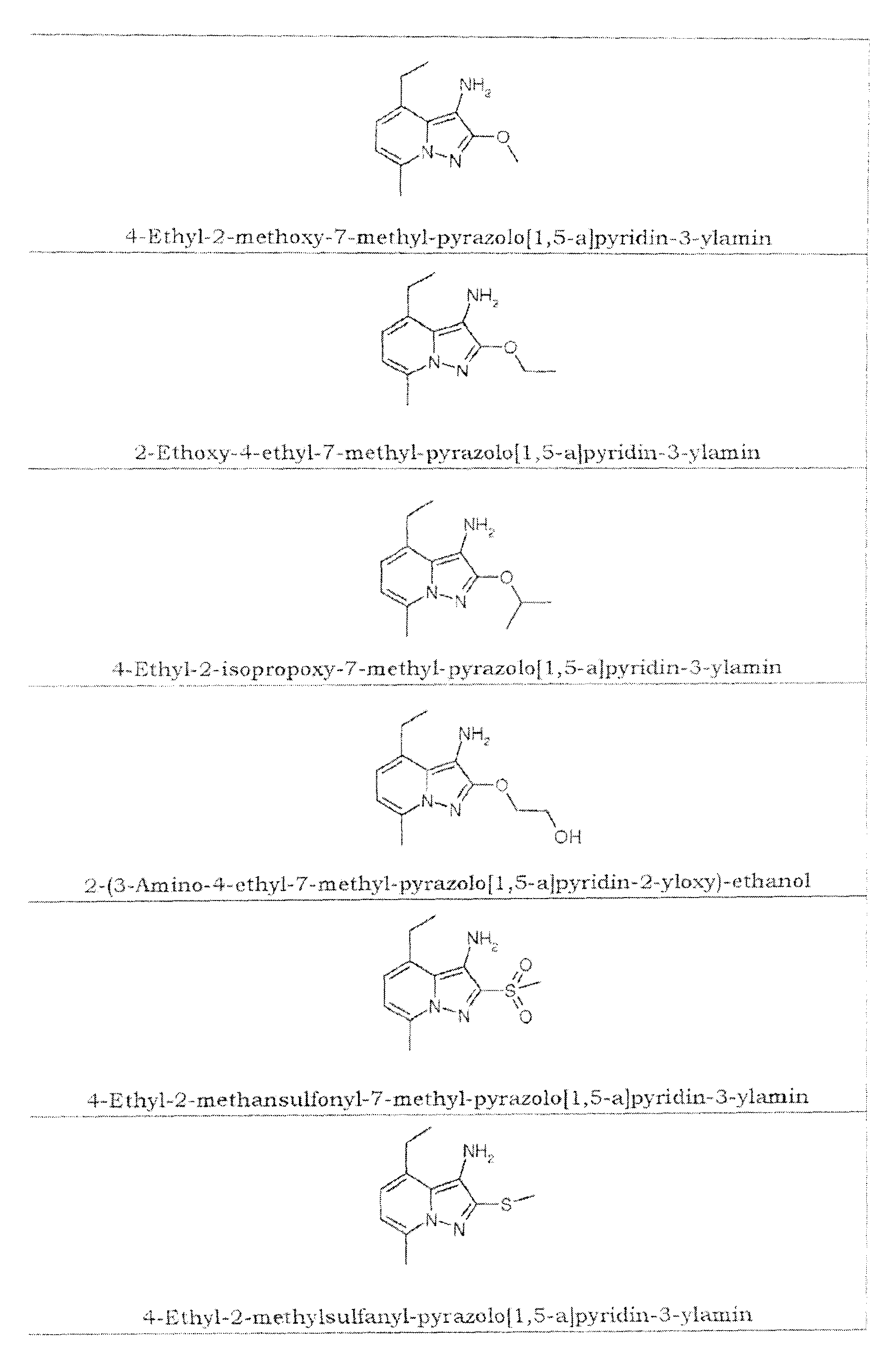

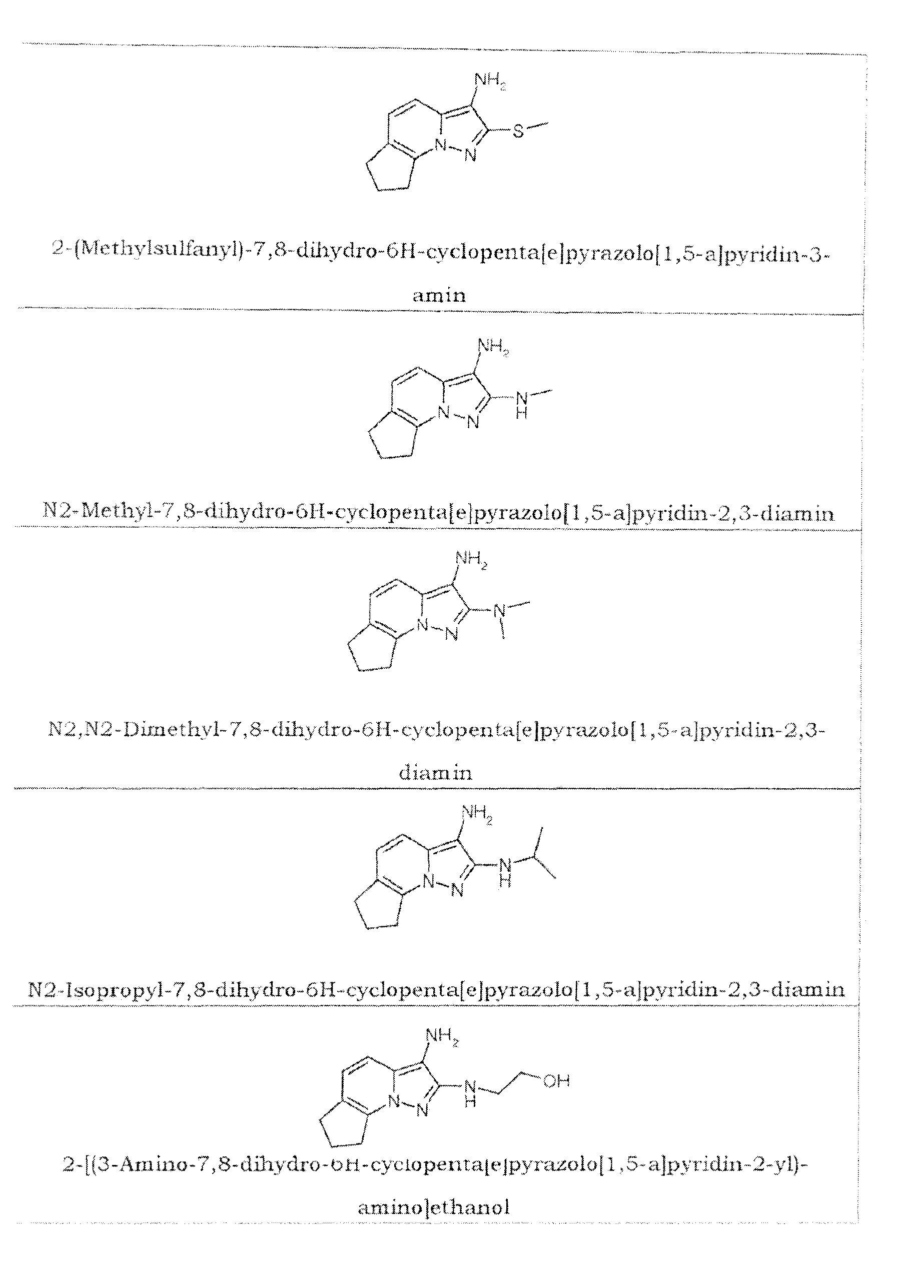

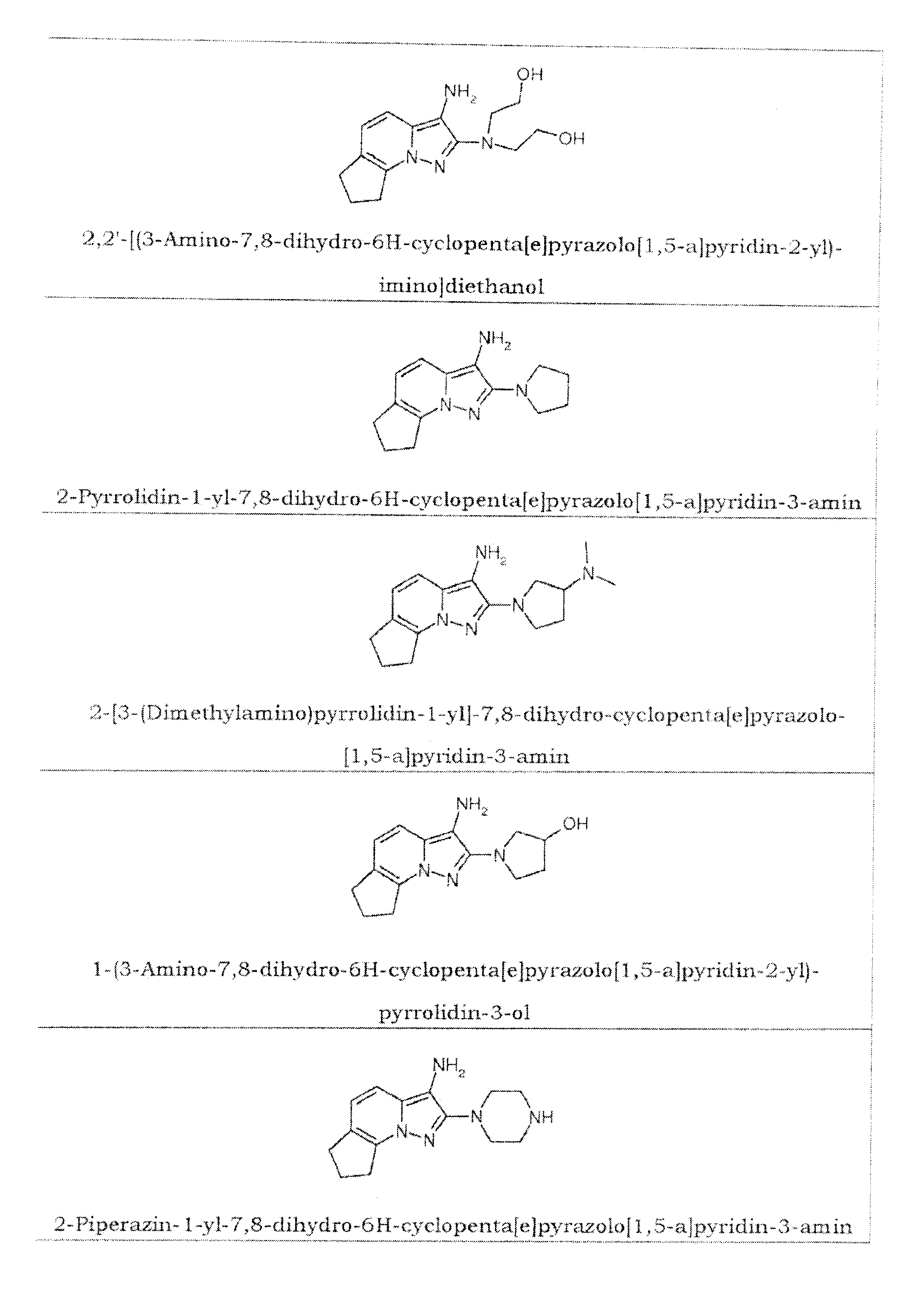

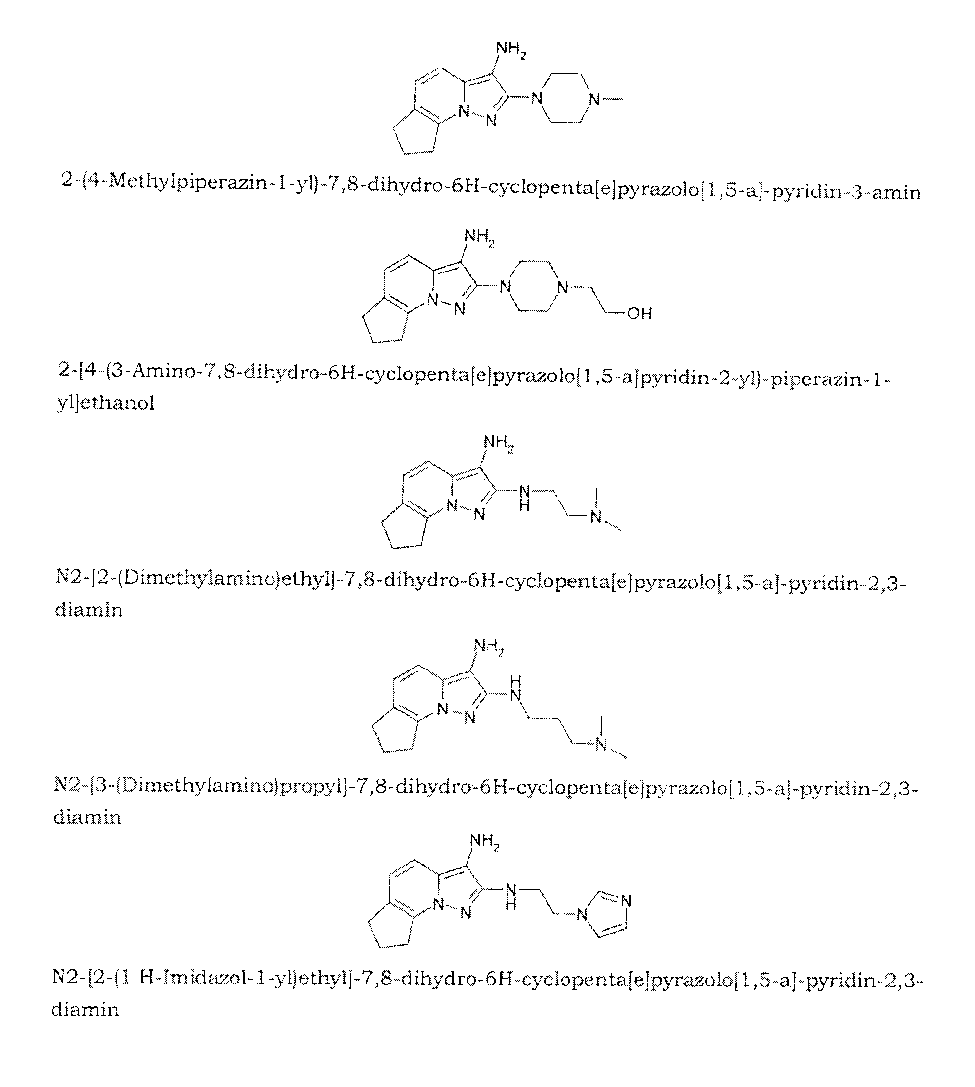

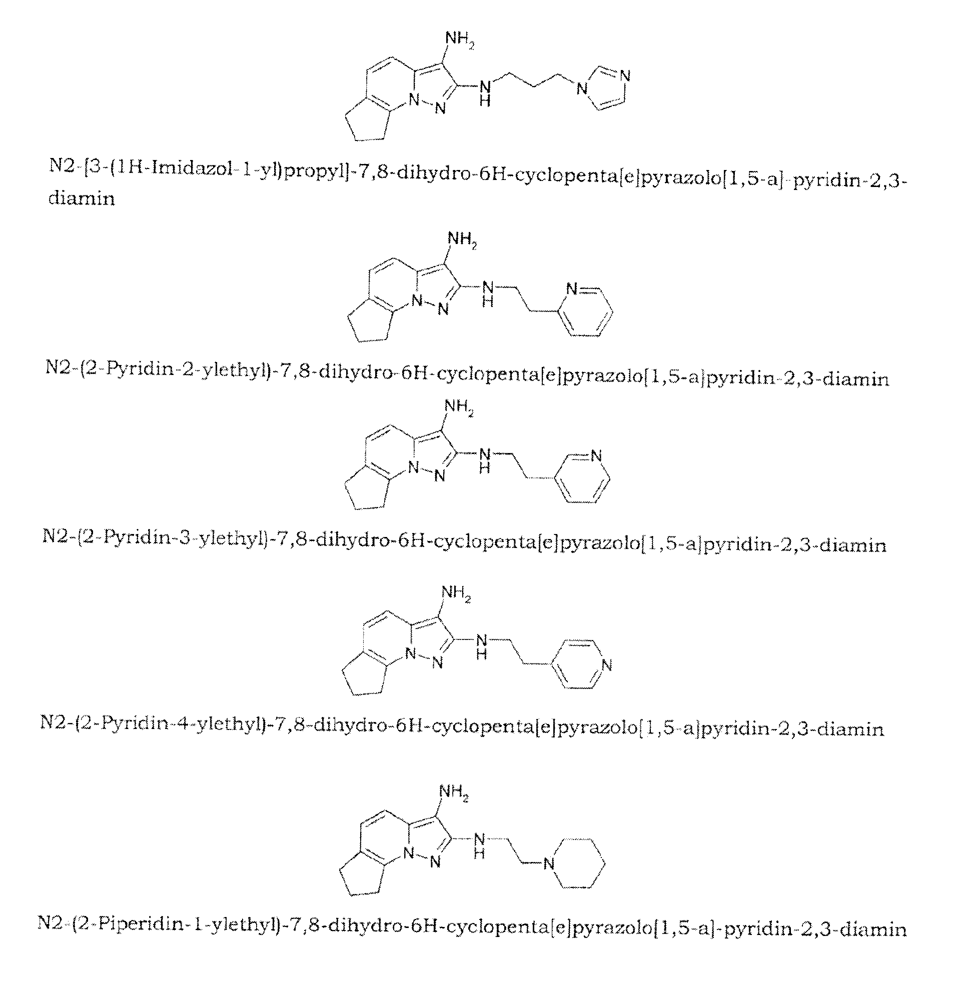

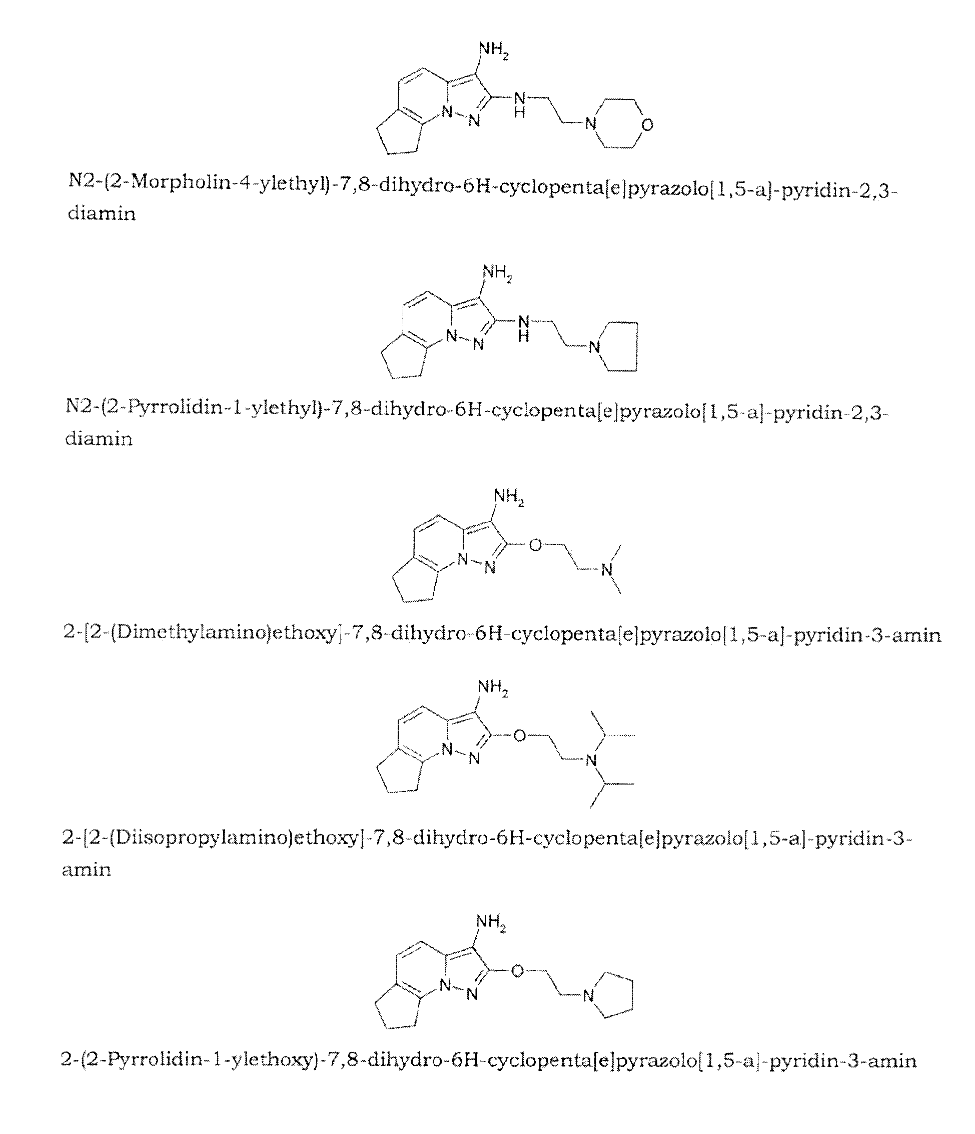

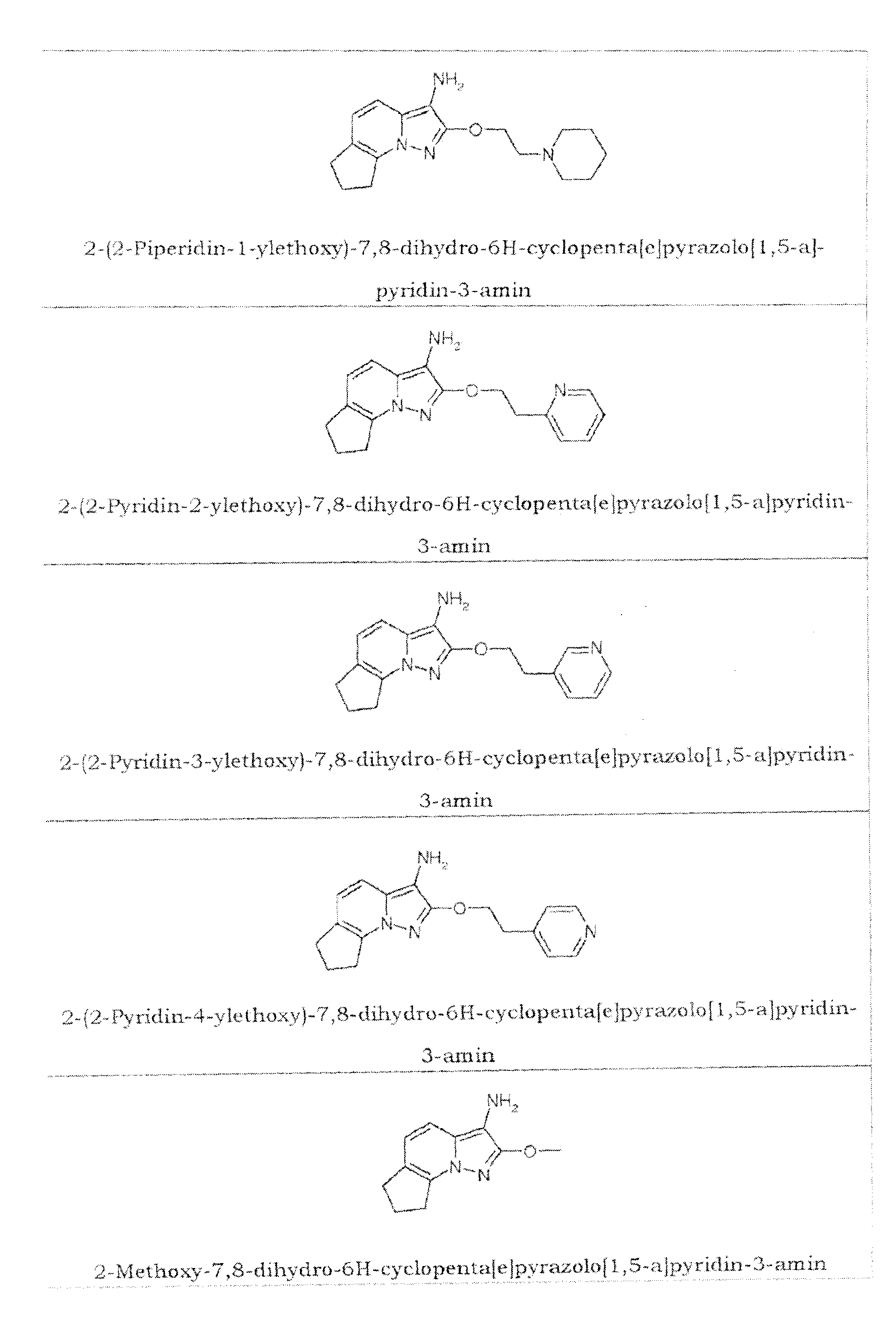

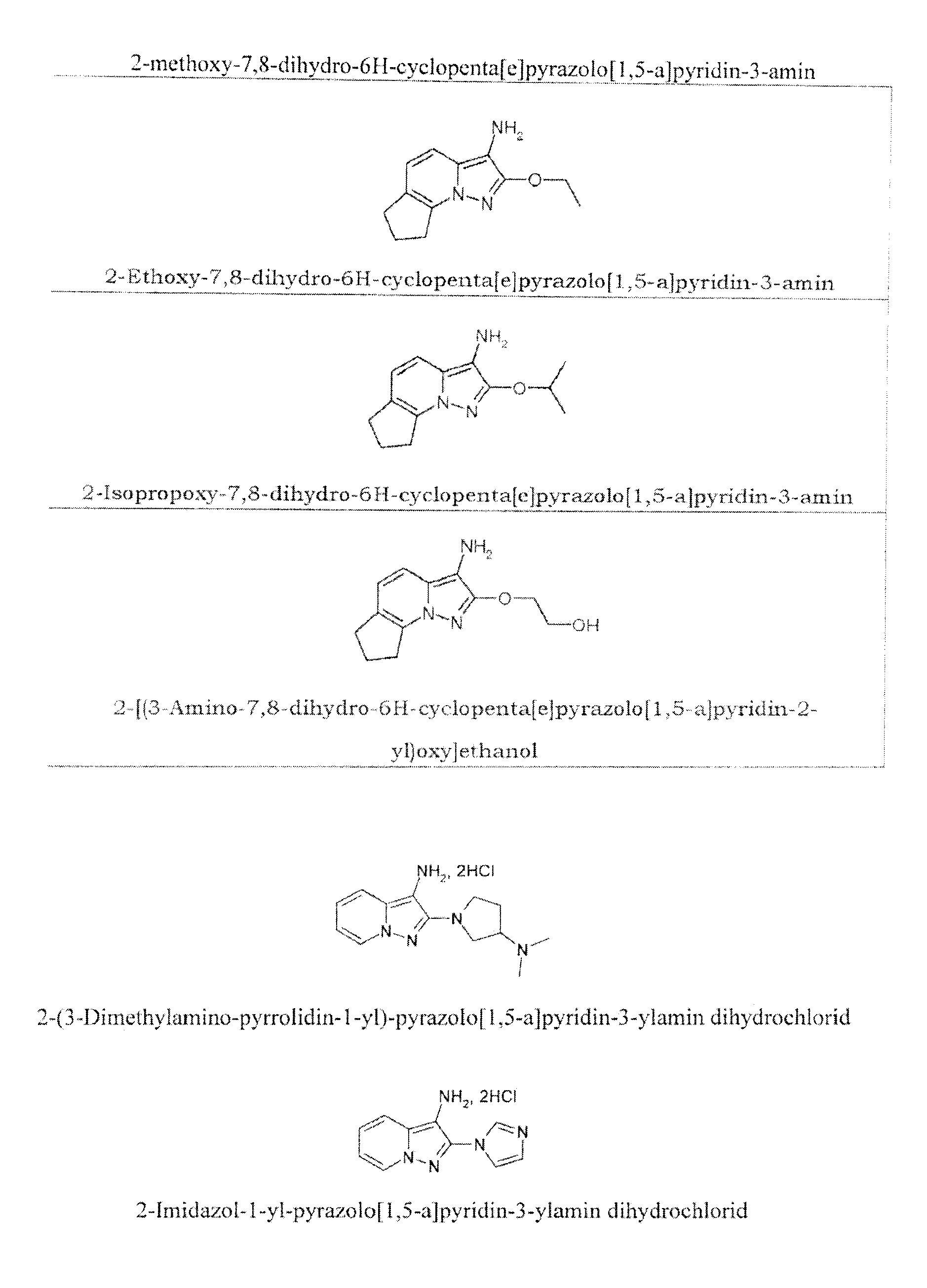

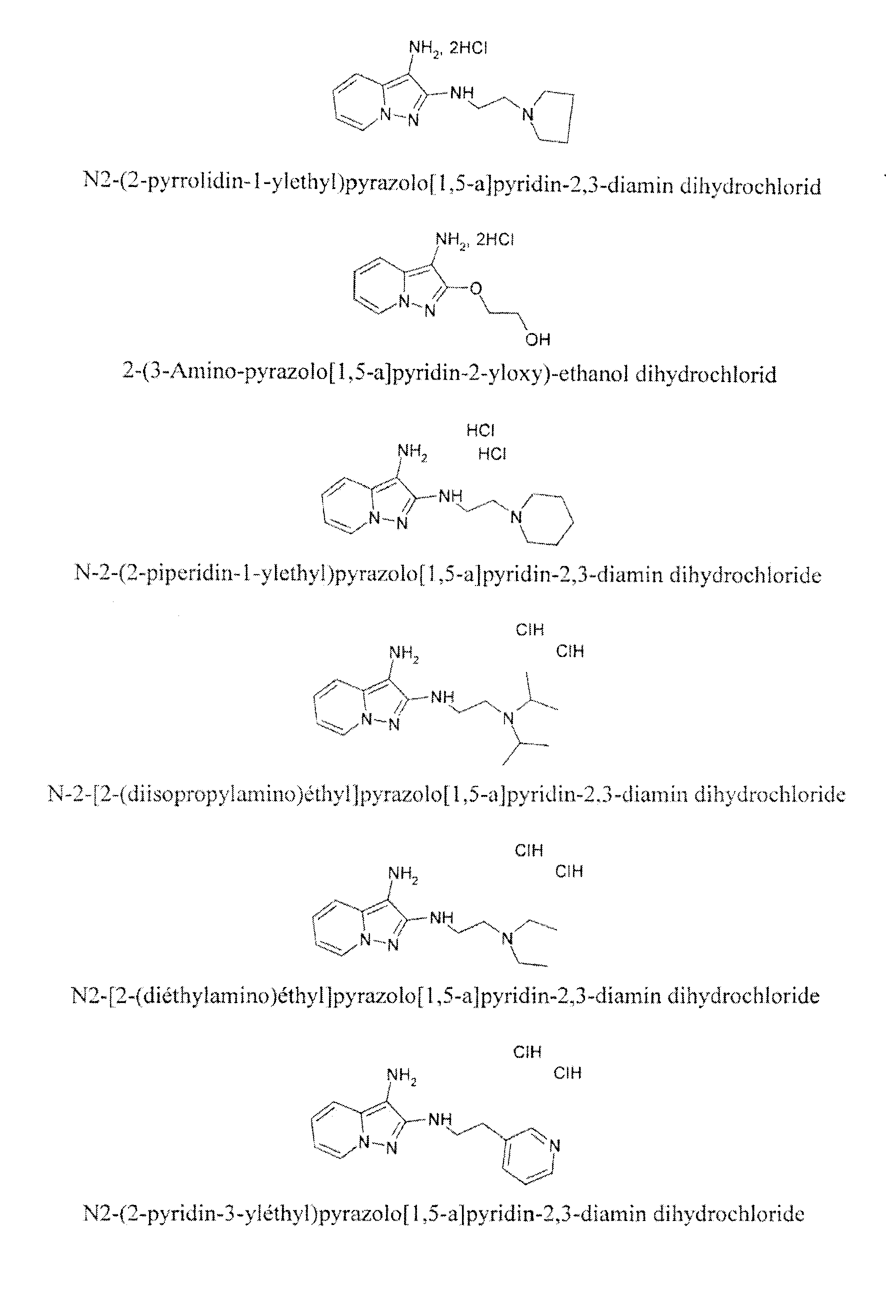

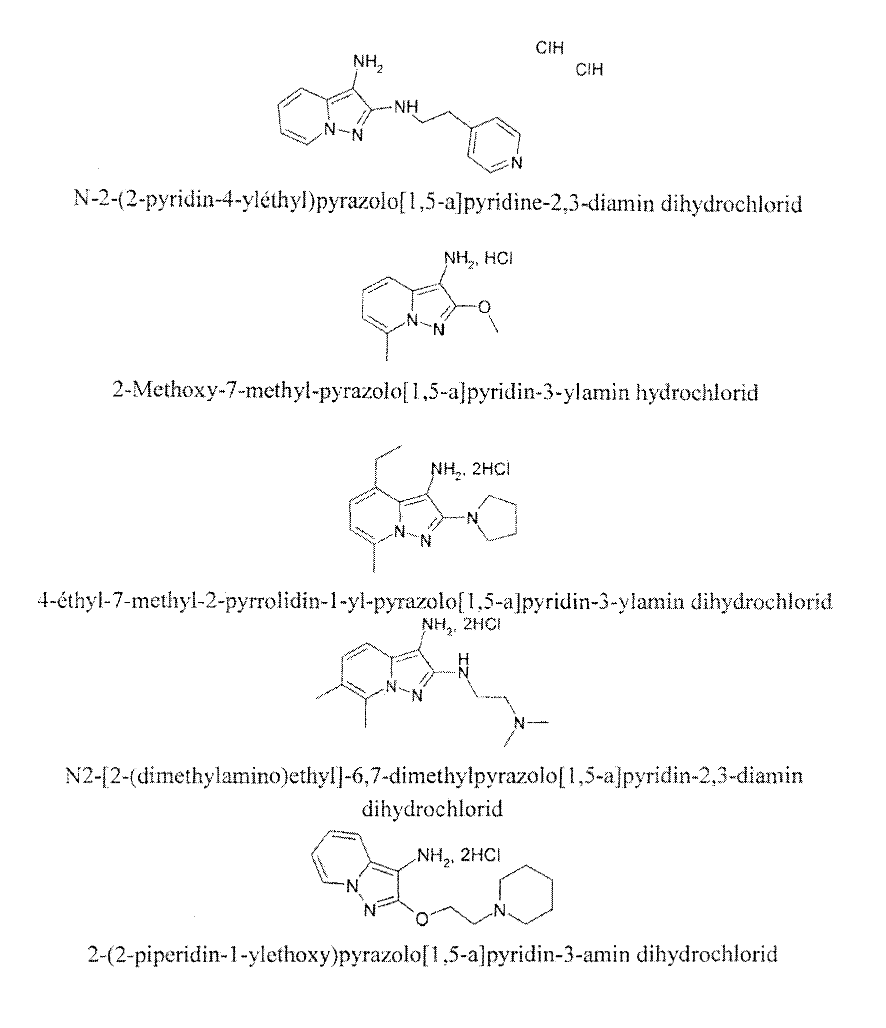

8. Zusammensetzung nach Anspruch 7, wobei die Verbindung der Formel (I) ausgewählt ist unter: N2-Ethylpyrazolo[1,5-a]pyridin-2,3-diamin-Dihydrochlorid, 2-(3-Amino-pyrazolo[1,5-a]pyridin-2-ylamino)-ethanol-Dihydrochlorid, 1-(3-Amino-pyrazolo[1,5-a]pyridin-2-ylamino)-propan-2-ol-Dihydrochlorid, N-2-(3-Imidazol-1-yl-propyl)-pyrazolo[1,5-a]pyridin-2,3-diamin-Dihydrochlorid, 2-Pyrrolidin-1yl-pyrazolo[1,5-a]pyridin-3-ylamin-Dihydrochlorid, 2-(3-Dimethylamino-pyrrolidin-1-yl)-pyrazalo[1,5-a]pyridin3-ylamin-Dihydrochlorid, 2-Imidazol-1-yl-pyrazolo[1,5-a]pyridin-3-ylamin-Dihydrochlorid, N2-(2-Pyrrolidin-1-ylethyl)pyrazolo[1,5-a]pyridin-2,3-diamin-Dihydrochlorid, 2-(3-Amino-pyrazolo[1,5-a]pyridin-2-yloxy)-ethanol-Dihydrochlorid, 2-Ethoxy-pyrazolo[1,5-a]pyridin-3-ylamin-Dihydrochlorid, 2-Methoxy-7-methyl-pyrazolo[1,5-a]pyridin-3-ylamin-Hydrochlorid, 4-Ethyl-2-methoxy-7-methyl-pyrazolo[1,5-a]pyridin-3-ylamin-Dihydrochlorid, 4-Ethyl-7-methyl-2-pyrrolidin-1-yl-pyrazolo[1,5-a]pyridin-3-ylamin-Dihydrochlorid, N2-(2-Piperidin-1-ylethyl)pyrazolo[1,5-a]pyrazolo-2,3diamin-Dihydrochlorid, N2-[2-(Diisopropylamino)ethyl]pyrazolo[1,5-a]pyridin-2,3-diamin-Dihydrochlorid, N2-[2-(Diethylamino)ethyl]pyrazolo[1,5-a]pyridin-2,3-diamin-Dihydrochlorid, N2-(2-Pyridin-3-ylethyl)pyrazolo[1,5-a]pyridin-2,3-diamin-Dihydrochlorid, N2-(2-Pyridin-4-ylethyl)pyrazolo[1,5-a]pyridin-2,3-diamin-Dihydrochlorid, N2-(2-Pyridin-2-ylethyl)pyrazolo[1,5-a]pyridin-2,3-diamin-Dihydrochlorid, N2-(2-piperazin-1-ylethyl)pyrazolo[1,5-a]pyridiin-2,3-diamin, N2-[2-(4-Methylpiperazin-1-yl)ethyl]pyrazolo[1,5-a]pyridin-2,3-diamin-Dihydrochlorid, N2-(2-Morpholin-4-ylethyl)pyrazolo[1,5-a]pyridin-2,3-diamin-Dihydrochlorid, N2-[2-(Dimethylamino)ethyl]-7,8-dihydro-6H-cyclopenta[e]-pyrazolo[1,5-a]pyridin-2,3-diamin-Dihydrochlorid, 2-[(3-Amino-7,8-dihydro-6H-cyclopenta[e]pyrazolo[1,5-a]pyridin-2-yl)amino]ethanol-Hydrochlorid, 2-Methoxy-7,8-dihydro-6H-cyclopenta[e]pyrazolo[1,5-a]pyridin-3-amin-Hydrochlorid, 2-Isopropoxy-7,8-dihydro-6H-cyclopenta[e]pyrazolo[1,5-a]-pyridin-3-amin-Hydrochlorid, 2-[2-(Dimethylamino)ethoxy]pyrazolo[1,5-a]pyridin-3-amin-Dihydrochlorid, 2-(2-Pyrrolidin-1-ylethoxy)pyrazolo[1,5-a]pyridin-3-amin-Dihydrochlorid, 2-(2-Piperidin-1-ylethoxy)pyrazolo[1,5-a]pyridin-3-amin-Dihydrochlorid, 2-(2-Morpholin-4-ylethoxy)pyrazolo[1,5-a]pyridin-3-amin-Dihydrochlorid, 2-[2-(Diisopropylamino)ethoxy]pyrazolo[1,5-a]pyridin-3-amin-Dihydrochlorid, 2-(2-Pyridin-2-ylethoxy)pyrazolo[1,5-a]pyridin-3-amin-Dihydrochlorid, 2-(2-Pyridin-3-ylethoxy)pyrazolo[1,5-a]pyridin-3-amin-Dihydrochlorid, 2-(2-Pyridin-4-ylethoxy)pyrazolo[1,5-a]pyridin-3-amin-Dihydrochlorid.

9. Zusammensetzung nach einem der vorhergehenden Ansprüche, bei der der Mengenanteil jeder Oxidationsbase der Formel (I) im Bereich von etwa 0,001 bis 10 Gew.-% des Gesamtgewichts der Farbmittelzusammensetzung liegt.

10. Zusammensetzung nach einem der vorhergehenden Ansprüche, die ferner einen Kuppler enthält, der unter den meta-Phenylen-diaminen, meta-Aminophenolen, meta-Dihydroxybenzolen, Naphthalinkupplern, heterocyclischen Kupplern und ihren Additionssalzen sowie deren Gemischen ausgewählt ist.

11. Zusammensetzung nach Anspruch 10, wobei der Mengenanteil jedes Kupplers im Bereich von 0,001 bis 10 Gew.-% des Gesamt-gewichts der Farbmittelzusammensetzung liegt.

12. Verfahren zum Färben von Keratinfasern, **dadurch gekennzeichnet, dass** eine Zusammensetzung, wie sie in einem der Ansprü-che 1 bis 11 definiert ist, in Gegenwert eines Oxidationsmittels während einer Zeitspanne, die zur Bildung der gewünschten Färbung ausreichend ist, auf die Keratinfasern aufgebracht wird.

13. Verfahren nach Anspruch 12, wobei das Oxidationsmittel unter Wasserstoffperoxid, Harnstoffperoxid, Alkalimetallbromaten, Salzen von Persäure, Persäure und Oxidase ausgewählt ist.

14. Vorrichtung mit mehreren Abteilungen, wobei eine Abteilung ei-ne Farbmittelzusammensetzung, wie sie in einem der Ansprüche 1 bis 11. definiert ist, und eine zweite Abteilung ein Oxidations-mittel enthält.

15. Verbindungen der Formel (I), wie sie in einem der Ansprüche 1 bis 11 definiert sind, ausgenommen 2-Methylsulfanyl-pyrazolo-[1,5-a]pyridin-3-ylamin und 2,3-Diamino-pyrazolo[1,5-a]pyridin.

16. Verfahren zur Herstellung einer Verbindung der Formel (I), wie sie in einem der Ansprüche 1 bis 8 definiert ist, das die folgen-dien Schritte umfasst: 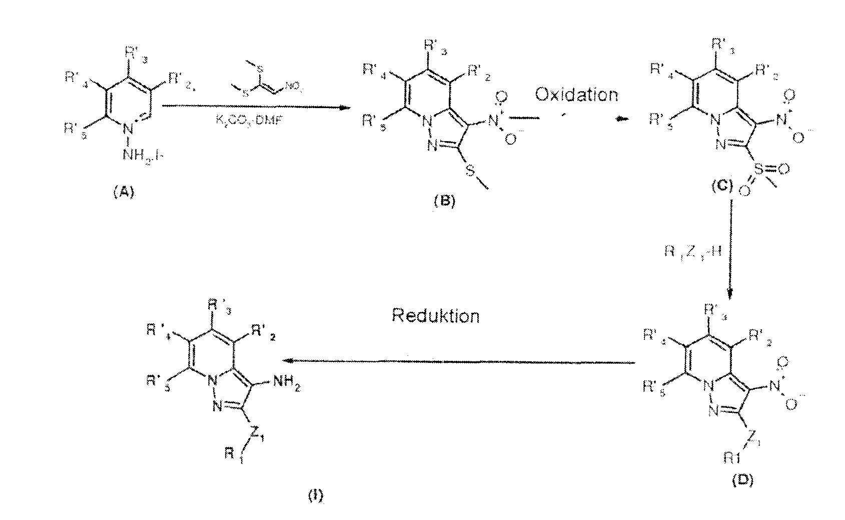 wobei die Gruppen R'₂, R'₃, R'₄ und R'₅ die für R₂, R₃, R₄ und R₅ angegebenen Bedeutungen aufweisen oder deren Precursoren sind.

17. Zwischenprodukte der Formeln (A) und (B), wie sie oben definiert sind, wobei mindestens eine der Gruppen R'₂, R'₃, R'₄ oder R'₅ von Wasserstoff verschieden ist, mit Ausnahme der Verbindung, bei der R'₂ = Me, wenn alle anderen Substituenten Wasserstoff bedeuten.

18. Zwischenprodukte der Formeln (C) und (D), wie sie in Anspruch 16 definiert sind.
